# EUROPEAN PATENT APPLICATION

(11) **EP 4 632 382 A2**
(43) Date of publication of application: **15.10.2025**
(21) Application number: 25180151.0
(22) Date of filing: 21.11.2018
(51) Int. Cl.: G01N 33/566

(54) **METHOD OF IDENTIFYING LAUNDRY MALODOR, MOLDY MALODOR AND/OR SWEAT MALODOR MODULATING COMPOUNDS**

(30) Priority: 22.11.2017 US 201762589947 P; 21.02.2018 EP 18157790; 02.11.2018 US 201862754899 P
(62) Divisional of application: 18811752.7
(71) Applicant: Firmenich SA, 1242 Satigny (CH)
(72) Inventor: KAO, Huey-Ling, Plainsboro, New Jersey 08536 (US); SHEIKH, Mushhood, Plainsboro, New Jersey 08536 (US); PFISTER, Patrick, Plainsboro, New Jersey 08536 (US); SANDU, Cristinel, Plainsboro, New Jersey 08536 (US); EVANS, Barry, Plainsboro, New Jersey 08536 (US); O'LEARY, Nicholas, Plainsboro, New Jersey 08536 (US); SMITH, Ben, Plainsboro, New Jersey 08536 (US)
(74) Representative: Strych, Sebastian

(57) **Abstract**

The present invention relates to a method for identifying a compound that binds, suppresses, blocks, inhibits, and/or modulates the activity of an olfactory receptor that is activated by a laundry malodor causing substance, a moldy malodour causing substance and/or a sweat malodor-causing substance.

## Description

### CROSS REFERENCE TO RELATED APPLICATIONS

The present application claims priority to U.S. Provisional Patent Application Serial No. 62/589,947, filed on November 22, 2017, European Patent Application Serial No. 18157790.9, filed on February 21, 2018, and U.S. Provisional Patent Application Serial No. 62/754,899, filed on November 2, 2018, the entire contents of which are hereby incorporated by reference in their entirety.

### FIELD

The technical field is directed to odorant and aroma receptors and assays that can be used to identify odorant and/or aroma compounds, and more specifically inhibitors, modulators, or counteractants of malodor compounds of laundry malodour, moldy malodour, and/or sweat malodour, such as dimethyl trisulfide (DMTS), geonol, 1-octen-3-ol, butyric acid, 3-methyl-2-hexenoic acid, 3-hydroxy-3-methyl-hexanoic acid and 3-methyl-3-sulphanylhexanol (transpirol).

### BACKGROUND

Olfaction is one of the most complex and poorly understood of human sensory systems. From olfactory receptor (OR) activation to perception, there are many steps that still require further investigation. If we can understand how the OR code for individual odorants and mixtures translates into perception then we can exploit this knowledge to bring significant benefit in several areas. These areas include odor modulators like malodor counteractants that block the perception of unpleasant odors, new flavor and fragrance ingredients that replace non-biodegradable or toxic compounds, and odor enhancers that would limit our reliance on difficult to source compounds from natural sources. The 'olfactory code' combinatorial paradigm is centered on the observation that any single OR may be activated by multiple odorants, and conversely most odorants are capable of activating several ORs. In the mouse genome there are approximately 1,200 distinct intact ORs. Humans, by contrast, have approximately 400. In both cases, the repertoire of ORs is activated by many thousands of odorants in the world, and it is this combinatorial complexity that allows for the breadth of olfactory sensations we can perceive. However, odorants or ligands for only 82 mouse (approximately 8%) and 17 human ORs (approximately 10%) have been identified as of 2014 using traditional deorphanization methods. In addition, the physiological relevance of most ligands for the human ORs, essentially identified *in vitro,* has not been tested.

A method that can rapidly and reliably identify a relatively small subset of ORs, within the entire repertoire of ORs that exist in an organism that are specifically activated or inhibited by one or more odorants is described in WO2014/210585. However, using this method, there remains a need to identify odorant receptors and more particularly malodor receptors that are activated by particular malodor-causing substances.

Malodor-causing compounds such as dimethyl trisulfide (DMTS), geonol, 1-octen-3-ol, butyric acid, 3-methyl-2-hexenoic acid, 3-hydroxy-3-methyl-hexanoic acid and transpirol can generate unpleasant odors that arise, for example, from laundry or body odor caused by sweat or sebum, or moldy malodor

Hence, the problem to be solved by the present invention is the provision of methods of identifying of moldy malodor, laundry malodor and/or sweat malodor modulators or counteractants that bind, suppress, block, inhibit, and/or modulate the activity of one or more olfactory receptor that is activated by a particular malodor-causing substance (MCS) characteristic for such type of malodor. Assays that rely on new malodor receptors or on malodor receptors newly identified as associated with particular malodors-causing substances to identify new compounds or compounds mixtures that bind to these receptors are further desired.

### SUMMARY

The above-mentioned problem was surprisingly solved by identifying panels of MCSs which are characteristic for laundry malodor, moldy malodor or sweat malodor and panels of olfactory receptors that are activated by such MCS, and which may be used as targets to identify modulator substances to counteract activation of these olfactory receptors by such MCS.

As laundry MCS geonol, DMTS and 1-octen-3-ol have been identified. As olfactory receptor polypeptides activated by at least one of said compounds the human receptors OR2W1, OR1A1, OR2J3, OR4Q3, OR5K1, OR11A1, OR2M3, OR4S2, the mouse orthologues Olfr263, Olfr403, Olfr137, Olfr173, Olfr164, Olfr735, Olfr1193, Olfr96, and the mouse receptors Olfr1487, Olfr339, Olfr93, Olfr398, Olfr120, Olfr1364 and Olfr937 could be identified.

As sweat MCS butyric acid, 3-methyl-2-hexenoic acid, 3-hydroxy-3-methyl-hexanoic acid and transpirol have been identified. As olfactory receptor polypeptides activated by at least one of said compounds the human receptors OR2W1, OR1A1, OR2J3, OR5K1, OR51E1, OR51I2, the mouse orthologues Olfr263, Olfr403, Olfr558 Olfr641, Olfr137, Olfr164, and the mouse receptors Olfr1126 and Olfr46 could be identified.

As moldy MCS geonol, DMTS and 1-octen-3-ol have been identified. As olfactory receptor polypeptides activated by at least one of said compounds the human receptors OR2W1, OR1A1, OR2J3, OR4Q3, OR5K1, OR11A1, OR2M3, OR4S2, the mouse orthologues Olfr263, Olfr403, Olfr735, Olfr1193, Olfr96, Olfr137, Olfr173, Olfr164, and the mouse receptors Olfr1487, Olfr339, Olfr93, Olfr398, Olfr120, Olfr1364 and Olfr937 could be identified.

Further provided by the present invention are methods to assay whether a compound binds, suppresses, blocks, inhibits, and/or modulates the activity of an olfactory receptor that is activated by a laundry malodor, moldy malodor, and/or sweat malodor-causing substance, and in particular methods of identifying such compounds.

Further provided are isolated olfactory receptor polypeptides, corresponding encoding nucleic acid sequences, recombinant nucleic acids comprising such encoding sequences and corresponding expression vectors.

Further provided are non-human host organism or a host cell that has been modified to express such olfactory receptor polypeptides which are activated by MCSs which a characteristic for laundry malodor, moldy malodor or sweat malodor.

Further provided is the use of such olfactory receptor polypeptides which are activated by MCSs and which are characteristic for laundry malodor, moldy malodor or sweat malodor for identifying a malodor modulating compound.

### DESCRIPTION OF THE DRAWINGS

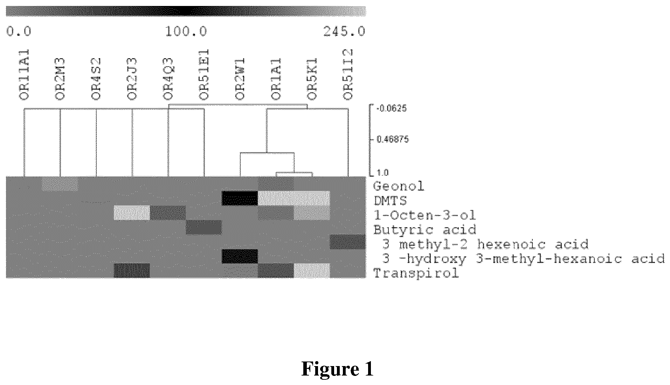

**Figure 1** shows certain MCS and olfactory receptors according to some aspects presented herein.

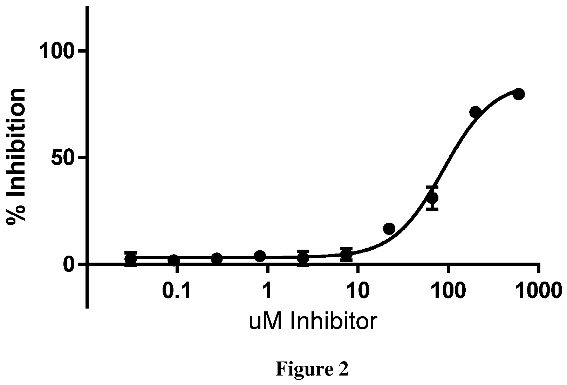

**Figure 2** shows the potency of the antagonist 1-[(1S,7S)-2,3,4-trimethyltricyclo[5.2.1.0-1.5-]dec-4-yl) ethanone oxime as an inhibitor of the butyric acid olfactory receptor OR51E1 under highly stringent screening conditions. The black line denotes the % inhibition of the butyric acid olfactory receptor activity observed from cells treated with 81 µM butyric acid (EC₈₇) and the antagonist at the concentrations [µM] indicated.

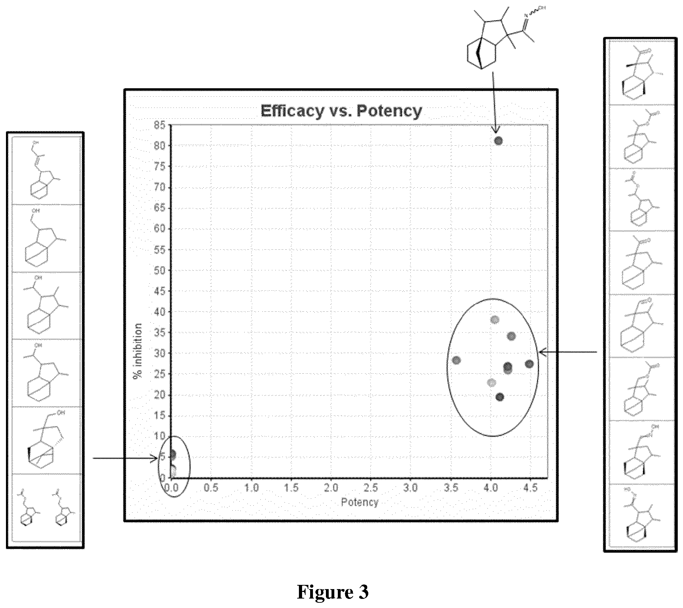

**Figure 3** shows a summary of the % inhibition of the butyric acid olfactory receptor OR51E1 under highly stringent screening conditions observed from cells treated with compounds according to some aspects presented herein. The structures of the compounds tested are shown to the sides of the figure.

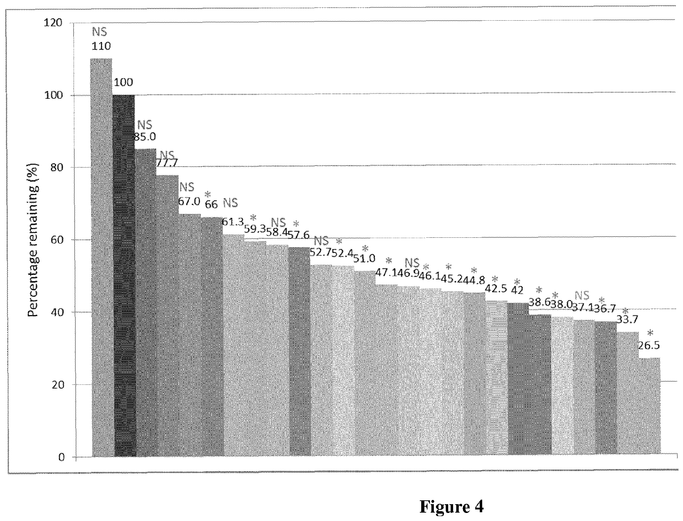

**Figure 4** shows the ability of compounds according to some aspects of the present disclosure to reduce the perceived intensity of geonol in human sensory panel tests. Many of these compounds are shown to inhibit the activity of a geonol olfactory receptor. NS, the % geonol perception remaining is not statistically different compared to geonol alone at isointense concentrations; Asterisk (*), the % geonol perception remaining is statistically different compared to geonol alone at isointense concentrations.

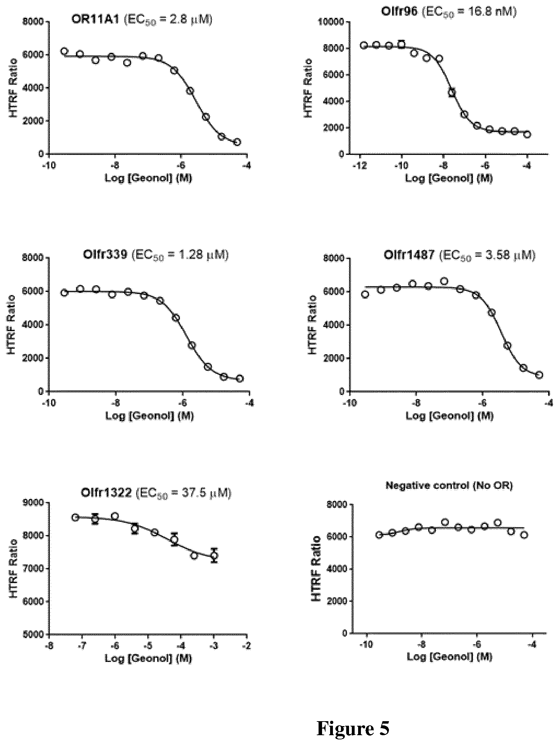

**Figure 5** shows geonol concentration-dependent and specific activation of OR11A1, Olfr96, Olfr339, Olfr1487 and Olfr1322 in a cell-based assay, according to assays and methods disclosed in WO2014/210585 and further in the Methods of Monitoring OR Activity section. The detection method pertains to OR-dependent accumulation of cAMP in said cells, as a result of OR activation by geonol.

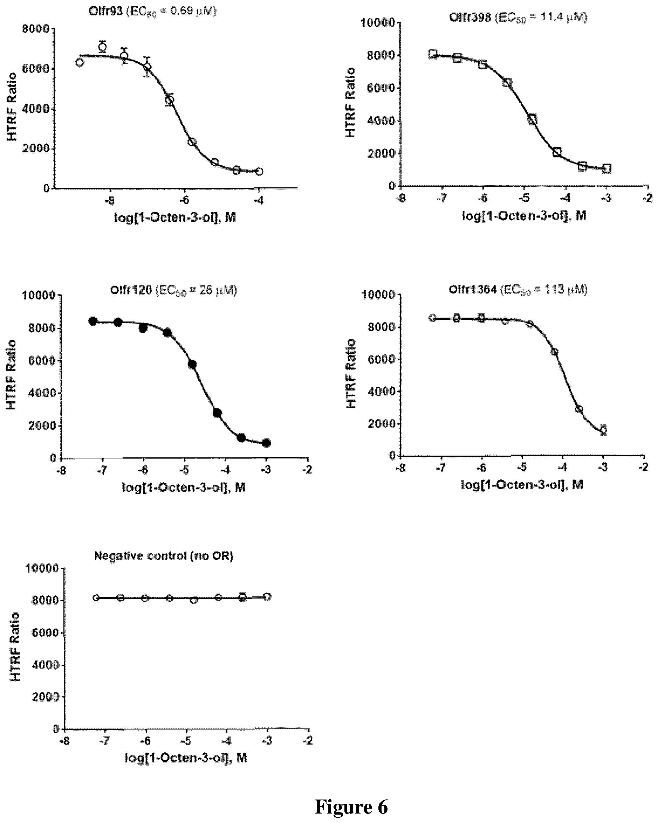

**Figure 6** shows 1-octen-3-ol concentration-dependent and specific activation of Olfr93, Olfr398, Olfr120 and Olfr1364 in a cell-based assay, according to assays and methods disclosed in WO2014/210585 and further in the Methods of Monitoring OR Activity section. The detection method pertains to OR-dependent accumulation of cAMP in said cells, as a result of OR activation by 1-octen-3-ol.

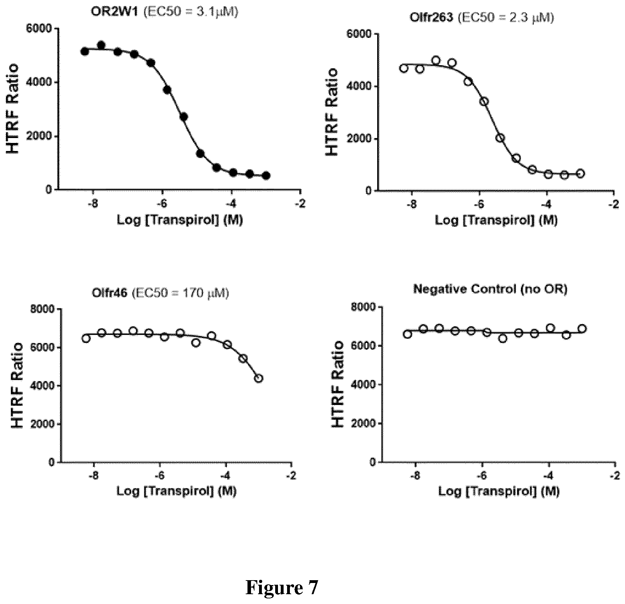

**Figure 7** shows transpirol concentration-dependent and specific activation of OR2W1, Olfr263 and Olfr46 in a cell-based assay, according to assays and methods disclosed in WO2014/210585 and further in the Methods of Monitoring OR Activity section. The detection method pertains to OR-dependent accumulation of cAMP in said cells, as a result of OR activation by transpirol.

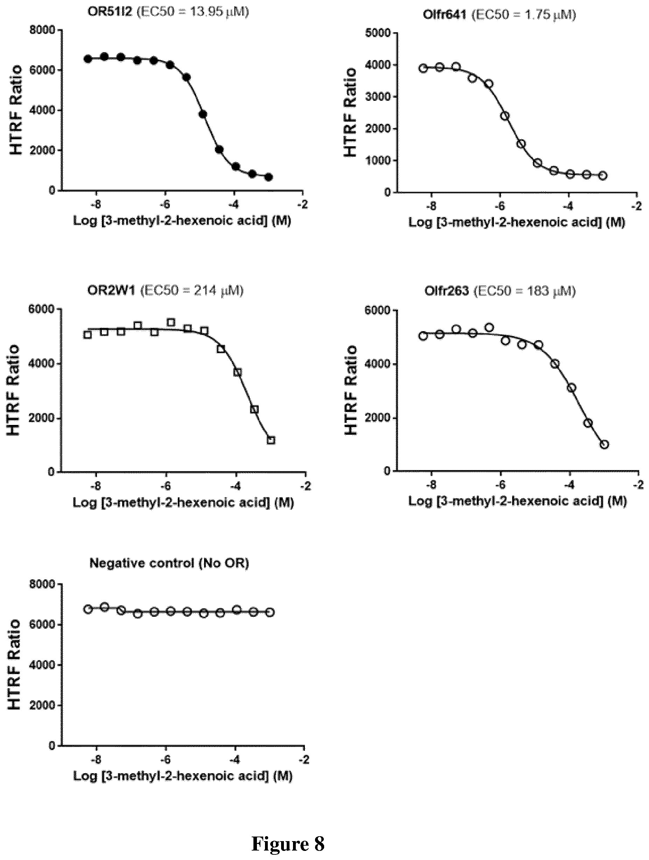

**Figure 8** shows 3-methyl-2-hexenoic acid concentration-dependent and specific activation of OR51I2, Olfr641, OR2W1 and Olfr263 in a cell-based assay according to assays and methods disclosed in WO2014/210585 and further in the Methods of Monitoring OR Activity section. The detection method pertains to OR-dependent accumulation of cAMP in said cells, as a result of OR activation by 3-methyl-2-hexenoic acid.

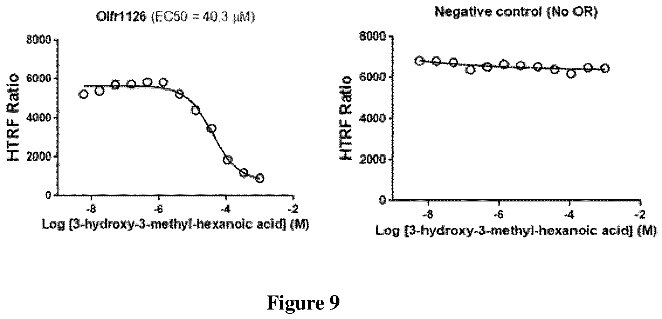

**Figure 9** shows 3-hydroxy-3-methyl-hexanoic acid concentration-dependent and specific activation of Olfr1126 in a cell-based assay, according to assays and methods disclosed in WO2014/210585 and further in the Methods of Monitoring OR Activity section. The detection method pertains to OR-dependent accumulation of cAMP in said cells, as a result of OR activation by 3-hydroxy-3-methyl-hexanoic acid.

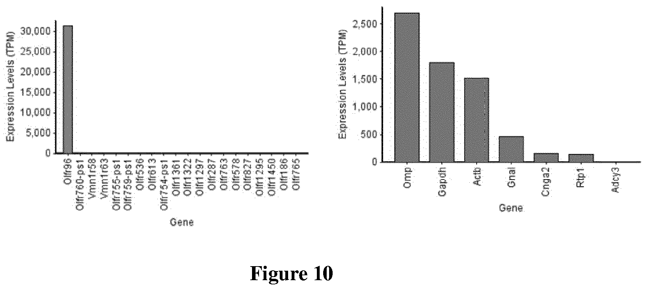

**Figure 10** shows the NGS data from a single OSN responding to geonol, according to the method described in WO2014/210585, and further in the Methods of OR activity part. Left panel shows the relative transcriptional abundance of ORs, with Olfr96 being significantly higher than the rest. Right panel shows the presence and relative abundance of key marker housekeeping genes for OSN (omp, actb, cnga2, rtp1, gnal, adcy3), in the same NGS sample.

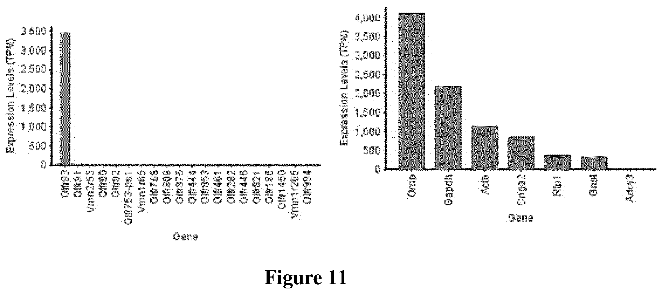

**Figure 11** shows the NGS data from a single OSN responding to 1-octen-3-ol, according to the method described in WO2014/210585, and further in the Methods of OR activity part. Left panel shows the relative transcriptional abundance of ORs, with Olfr93 being significantly higher than the rest. Right panel shows the presence and relative abundance of key marker housekeeping genes for OSN (omp, actb, cnga2, rtp1, gnal, adcy3), in the same NGS sample.

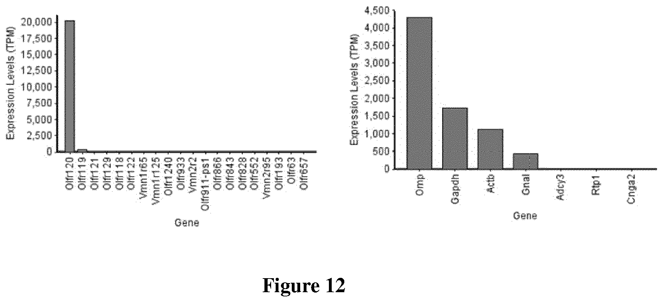

**Figure 12** shows the NGS data from a single OSN responding to 1-octen-3-ol, according to the method described in WO2014/210585, and further in the Methods of OR activity part. Left panel shows the relative transcriptional abundance of ORs, with Olfr120 being significantly higher than the rest. Right panel shows the presence and relative abundance of key marker housekeeping genes for OSN (omp, actb, cnga2, rtp1, gnal, adcy3), in the same NGS sample.

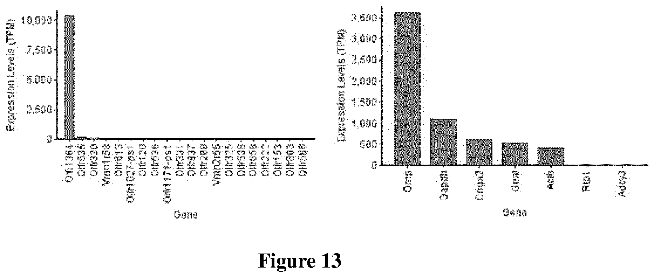

**Figure 13** shows the NGS data from a single OSN responding to 1-octen-3-ol, according to the method described in WO2014/210585, and further in the Methods of OR activity part. Left panel shows the relative transcriptional abundance of ORs, with Olfr1364 being significantly higher than the rest. Right panel shows the presence and relative abundance of key marker housekeeping genes for OSN (omp, actb, cnga2, rtp1, gnal, adcy3), in the same NGS sample.

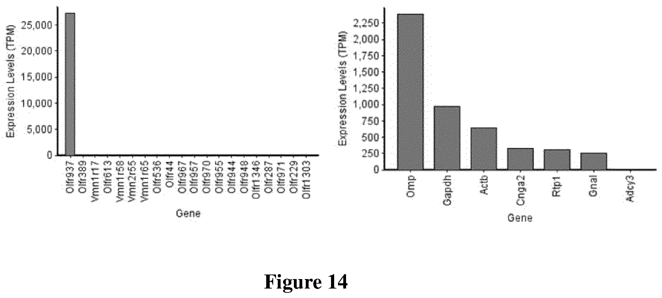

**Figure 14** shows the NGS data from a single OSN responding to 1-octen-3-ol, according to the method described in WO2014/210585, and further in the Methods of OR activity part. Left panel shows the relative transcriptional abundance of ORs, with Olfr937 being significantly higher than the rest. Right panel shows the presence and relative abundance of key marker housekeeping genes for OSN (omp, actb, cnga2, rtp1, gnal, adcy3) in the same NGS sample.

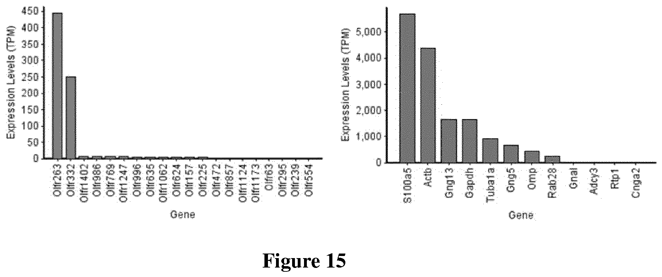

**Figure 15** shows the NGS data from a single OSN responding to transpirol, according to the method described in WO2014/210585, and further in the Methods of OR activity part. Left panel shows the relative transcriptional abundance of ORs, with Olfr263 being significantly higher than the rest. Right panel shows the presence and relative abundance of key marker housekeeping genes for OSNs in the same NGS sample.

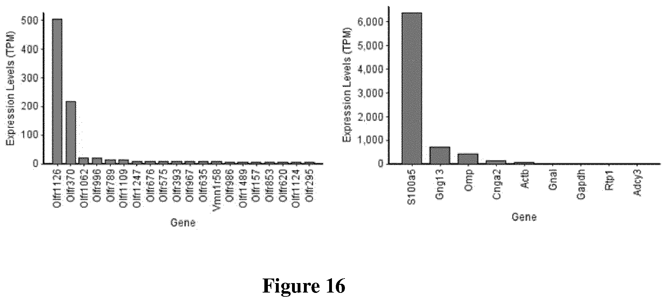

**Figure 16** shows the NGS data from a single OSN responding to 3-hydroxy-3-methyl-hexanoic acid, according to the method described in WO2014/210585, and further in the Methods of OR activity part. Left panel shows the relative transcriptional abundance of ORs, with Olfr1126 being significantly higher than the rest. Right panel shows the presence and relative abundance of key marker housekeeping genes for OSNs in the same NGS sample.

### DETAILED DESCRIPTION

### Definitions

For the descriptions herein and the appended claims, the use of "or" means "and/or" unless stated otherwise.

Similarly, "comprise," "comprises," "comprising," "include," "includes," and "including" are interchangeable and not intended to be limiting.

It is to be further understood that where descriptions of various embodiments use the term "comprising," those skilled in the art would understand that in some specific instances, an embodiment can be alternatively described using language "consisting essentially of" or "consisting of."

The following terms have the meanings ascribed to them unless specified otherwise.

"OR" refers to one or more members of a family of G protein-coupled receptors (GPCRs) that are expressed in olfactory cells. Olfactory receptor cells can also be identified on the basis of morphology or by the expression of proteins specifically expressed in olfactory cells. OR family members may have the ability to act as receptors for olfactory signal transduction.

"DMTS OR" refers to a member of the family of G protein-coupled receptors that is expressed in an olfactory cell, which receptors bind and/or are activated by DMTS in a binding or activity assay for identifying ligands that bind and/or activate GPCRs. Such assays are described below. DMTS receptors herein will include fragments, variants, including synthetic and naturally occurring, and chimeras or recombinant nucleic acids or proteins that respond to or bind DMTS. The same definition applies in analogy to the following receptors: "Geonol OR", "1-Octen-3-ol OR", "Butyric acid OR", "3-Methyl-2-hexenoic acid OR", "3-Hydroxy-3-methyl-hexanoic acid OR" and "Transpirol OR",

"OR" polypeptides are considered as such if they pertain to the 7-transmembrane-domain G protein-coupled receptor superfamily encoded by a single ~1kb long exon and exhibit characteristic olfactory receptor-specific amino acid motifs. The seven domains are called "transmembrane" or "TM" domains TM I to TM VII connected by three "internal cellular loop" or "IC" domains IC I to IC III, and three "external cellular loop" or "EC" domains EC I to EC III. The motifs and the variants thereof are defined as, but not restricted to, the MAYDRYVAIC motif (SEQ ID NO: 53) overlapping TM III and IC II, the FSTCSSH motif (SEQ ID NO: 54) overlapping IC III and TM VI, the PMLNPFIY motif (SEQ ID NO: 55) in TM VII as well as three conserved C residues in EC II, and the presence of highly conserved GN residues in TM I [Zhang, X. & Firestein, S. Nat. Neurosci. 5, 124-133 (2002); Malnic, B., et al. Proc. Natl. Acad. Sci. U. S. A. 101, 2584-2589 (2004)].

"OR" nucleic acids encode a family of GPCRs with seven transmembrane regions that have "G protein-coupled receptor activity," e.g., they may bind to G proteins in response to extracellular stimuli and promote production of second messengers such as IP₃, cAMP, cGMP, and Ca²⁺ via stimulation of enzymes such as phospholipase C and adenylate cyclase.

"Paralogous" OR genes or "paralogs" are the result of gene duplications and refer to closely related homologous genes within the same species.

"Orthologous" OR genes or "orthologs" are defined as phylogenetically linked by a gene present in a common ancestor and refer to closely related homologous genes in other species.

The "N terminal domain" region starts at the N-terminus and extends to a region close to the start of the first transmembrane region.

"Transmembrane regions" comprise the seven "transmembrane domains," which refers to the domain of OR polypeptides that lies within the plasma membrane, and may also include the corresponding cytoplasmic (intracellular) and extracellular loops. The seven transmembrane regions and extracellular and cytoplasmic loops can be identified using standard methods such as hydrophobicity profiles, or as described in Kyte & Doolittle, J. Mol. Biol., 157:105-32 (1982), or in Stryer. The general secondary and tertiary structure of transmembrane domains, in particular the seven transmembrane domains of G protein-coupled receptors such as olfactory receptors, are known in the art. Thus, primary structure sequence can be predicted based on known transmembrane domain sequences, as described in detail below. These transmembrane domains are useful for *in vitro* ligand-binding assays.

The phrase "functional effects" in the context of assays for testing compounds that modulate OR family member mediated olfactory transduction includes the determination of any parameter that is indirectly or directly under the influence of the receptor, e.g., functional, physical and chemical effects. It includes ligand binding, changes in ion flux, membrane potential, current flow, transcription, G protein binding, GPCR phosphorylation or dephosphorylation, signal transduction receptor-ligand interactions, second messenger concentrations (e.g., cAMP, cGMP IP₃, or intracellular Ca. ²⁺), *in vitro*, *in vivo*, and *ex vivo* and also includes other physiologic effects such as increases or decreases of neurotransmitter or hormone release.

By "determining the functional effect" or "confirming the activity" in the context of assays is meant assays for a compound that increases or decreases a parameter that is indirectly or directly under the influence of an OR family member, e.g., functional, physical and chemical effects. Such functional effects can be measured by any means known to those skilled in the art, e.g., changes in spectroscopic characteristics (e.g., fluorescence, absorbance, refractive index), hydrodynamic (e.g., shape), chromatographic, or solubility properties, patch clamping, voltage-sensitive dyes, whole cell currents, radioisotope efflux, inducible markers, oocyte OR gene expression; tissue culture cell OR expression; transcriptional activation of OR genes or activity induced genes such as egr-1 or c-fos; ligand-binding assays; voltage, membrane potential and conductance changes; ion flux assays; changes in intracellular second messengers such as cAMP, cGMP, and inositol triphosphate (IP3); changes in intracellular calcium levels; neurotransmitter release, and the like.

"Binder, " "suppressors, " "blockers, " "inhibitors," and/or "modulators" of OR genes or proteins are used interchangeably to refer to binding, suppressing, blocking, inhibitory, or modulating molecules identified using *in vivo*, *in vitro* and *ex vivo* assays for olfactory transduction, e.g., ligands, agonists, antagonists, enhancers, and their homologs and mimetics.

Inhibitors are compounds that, e.g., bind to, partially or totally block stimulation, decrease, suppress, prevent, delay activation, inactivate, desensitize, or down regulate olfactory transduction, e.g., antagonists. On the other hand activators are compounds that, e.g., bind to, stimulate, increase, open activate, facilitate, enhance activation, sensitize, or up regulate olfactory transduction, e.g., agonists.

Modulators include compounds that, e.g., alter the interaction of a receptor with: extracellular proteins that bind activators or inhibitor (e.g., odorant-binding proteins, ebnerin and other members of the hydrophobic carrier family, or a member of the lipocalin family); G proteins; kinases (e.g., homologs of rhodopsin kinase and beta adrenergic receptor kinases that are involved in deactivation and desensitization of a receptor); and arrestins, which also deactivate and desensitize receptors.

Modulators also include compounds that alter the affinity or the transduction efficacy of an OR altering the effect of an activator on the OR. Modulators can include genetically modified versions of OR family members, e.g., with altered activity, as well as naturally occurring and synthetic ligands, antagonists, agonists, small chemical molecules and the like. Such assays for inhibitors and activators include, e.g., expressing OR family members in cells or cell membranes, applying putative modulator compounds, in the presence or absence of flavour, fragrance or malodour molecules, e.g. a malodour-causing substance such as herein defined, and then determining the functional effects on olfactory transduction, as described above. Samples or assays comprising OR family members that are treated with a potential activator, inhibitor, or modulator are compared to control samples without the inhibitor, activator, or modulator to examine the extent of modulation. Control samples (untreated with modulators) are assigned a relative maximal OR activity value of 100%. Inhibition of an OR is achieved when the OR activity value relative to the control is about 80% or less, 70% or less, 60% or less, 50% or less, 40% or less, 30% or less, or 25-0%, like 1, 2, 3, 4 ,5 ,6 7, 8, 9 or 10%.

The terms "purified," "substantially purified," and "isolated" as used herein refer to the state of being free of other, dissimilar compounds with which the compound of the invention is normally associated in its natural state, so that the "purified," "substantially purified," and "isolated" subject comprises at least 0.5%, 1%, 5%, 10%, or 20%, or at least 50% or 75% of the mass, by weight, of a given sample. In one particular embodiment, these terms refer to the compound of the invention comprising at least 95, 96, 97, 98, 99 or 100% of the mass, by weight, of a given sample. As used herein, the terms "purified," "substantially purified," and " "isolated," when referring to a nucleic acid or protein, of nucleic acids or proteins, also refers to a state of purification or concentration different than that which occurs naturally in the mammalian, especially human body. Any degree of purification or concentration greater than that which occurs naturally in the mammalian, especially human body, including (1) the purification from other associated structures or compounds or (2) the association with structures or compounds to which it is not normally associated in the mammalian, especially human, body, are within the meaning of "isolated." The nucleic acid or protein or classes of nucleic acids or proteins, described herein, may be isolated, or otherwise associated with structures or compounds to which they are not normally associated in nature, according to a variety of methods and processes known to those of skill in the art.

As used herein, the terms "amplifying" and "amplification" refer to the use of any suitable amplification methodology for generating or detecting recombinant of naturally expressed nucleic acid, as described in detail, below. For example, the invention provides methods and reagents (e.g., specific degenerate oligonucleotide primer pairs, oligo dT primer) for amplifying (e.g., by polymerase chain reaction, PCR) naturally expressed (e.g., genomic DNA or mRNA) or recombinant (e.g., cDNA) nucleic acids of the invention *in vivo*, *ex vivo* or *in vitro.*

The term "7-transmembrane receptor" means a polypeptide belonging to a superfamily of transmembrane proteins that have seven domains that span the plasma membrane seven times (thus, the seven domains are called "transmembrane" or "TM" domains TM I to TM VII). The families of olfactory and certain taste receptors each belong to this super-family. 7-transmembrane receptor polypeptides have similar and characteristic primary, secondary and tertiary structures, as discussed in further detail below.

The term "nucleic acid" or "nucleic acid sequence" refers to a deoxy-ribonucleotide or ribonucleotide oligonucleotide in either single- or double-stranded form. The term encompasses nucleic acids, i.e., oligonucleotides, containing known analogs of natural nucleotides. The term also encompasses nucleic-acid-like structures with synthetic backbones. Unless otherwise indicated, a particular nucleic acid sequence also implicitly encompasses conservatively modified variants thereof (e.g., degenerate codon substitutions) and complementary sequences, as well as the sequence explicitly indicated. Specifically, degenerate codon substitutions may be achieved by generating, e.g., sequences in which the third position of one or more selected codons is substituted with mixed-base and/or deoxyinosine residues.

In addition to the gene sequences shown in the sequences disclosed herein, it will be apparent for the person skilled in the art that variants also include DNA sequence polymorphisms that may exist within a given population, which may lead to changes in the amino acid sequence of the polypeptides disclosed herein. Such genetic polymorphisms may exist in cells from different populations or within a population due to natural allelic variation. Allelic variants may also include functional equivalents.

Further embodiments also relate to the molecules derived by such sequence polymorphisms from the concretely disclosed nucleic acids. These natural variations usually bring about a variance of about 1 to 5% in the nucleotide sequence of a gene or in the amino acid sequence of the polypeptides disclosed herein. As mentioned above, the nucleic acid encoding the polypeptide of an embodiment herein is a useful tool to modify non-human host organisms or host cells intended to be used in the methods described herein.

The terms "polypeptide," "peptide" and "protein" are used interchangeably herein to refer to a polymer of amino acid residues, whether comprising naturally occurring amino acids or polymers and non-naturally occurring amino acids or polymers. The term "heterologous" when used with reference to portions of a nucleic acid indicates that the nucleic acid comprises two or more subsequences that are not found in the same relationship to each other in nature. For instance, the nucleic acid is typically recombinantly produced, having two or more sequences from unrelated genes arranged to make a new functional nucleic acid, e.g., a promoter from one source and a coding region from another source. Similarly, a heterologous protein indicates that the protein comprises two or more subsequences that are not found in the same relationship to each other in nature (e.g., a fusion protein).

A "promoter" is defined as an array of nucleic acid sequences that direct transcription of a nucleic acid. As used herein, a promoter includes necessary nucleic acid sequences near the start site of transcription, such as, in the case of a polymerase II type promoter, a TATA element. A promoter also optionally includes distal enhancer or repressor elements, which can be located as much as several thousand base pairs from the start site of transcription. A "constitutive" promoter is a promoter that is active under most environmental and developmental conditions. An "inducible" promoter is a promoter that is active under environmental or developmental regulation. The term "operably linked" refers to a functional linkage between a nucleic acid expression control sequence (such as a promoter, or array of transcription factor binding sites) and a second nucleic acid sequence, wherein the expression control sequence directs transcription of the nucleic acid corresponding to the second sequence.

As used herein, "recombinant" refers to a polynucleotide synthesized or otherwise manipulated *in vitro* (e.g., "recombinant polynucleotide"), to methods of using recombinant polynucleotides to produce gene products in cells or other biological systems, or to a polypeptide ("recombinant protein") encoded by a recombinant polynucleotide. "Recombinant" means also the ligation of nucleic acids having various coding regions or domains or promoter sequences from different sources into an expression cassette or vector for expression of, e.g., inducible or constitutive expression of a fusion protein comprising a translocation domain of the invention and a nucleic acid sequence potentially amplified using a primer. "Recombinant" means also modifications obtained by genome editing techniques, such as CRISPR/Cas9, of a cell that leads to stable or transient expression of endogenous genes such as the receptor gene referred to herein.

The term "expression vector" refers to any recombinant expression system for the purpose of expressing a nucleic acid sequence of the invention *in vitro*, *ex vivo*, or *in vivo*, constitutively or inducibly, in any cell, including prokaryotic, yeast, fungal, plant, insect or mammalian cell. The term includes any linear or circular expression systems including but not limited to viral vectors, bacteriophages and plasmids. The skilled person is capable of selecting a suitable vector according to the expression system. The term includes expression systems that remain episomal or integrate into the host cell genome. The expression systems can have the ability to self-replicate or not, i.e., drive transient expression in a cell. The term includes recombinant "expression cassettes" which can contain the minimum elements needed for transcription of the recombinant nucleic acid. The term also covers cassettes or vectors for expression of endogenous genes through, for example, genome editing methods such as CRISPR/Cas9.

By "a non-human organism or a host cell" is meant a non-human organism or a cell that contains a nucleic acid as described herein or an expression vector and supports the replication or expression of the expression vector. Host cells may be prokaryotic cells such as *E. coli*, or eukaryotic cells such as yeast, insect, amphibian, or mammalian cells such as CHO, HeLa, HEK-293, and the like, e.g., cultured cells, explants, and cells *in vivo.*

By "tag" or "tag combination" is meant a short polypeptide sequence that can be added to the odorant receptor protein. Typically, the DNA encoding a "tag" or a "tag combination" is added to the DNA encoding the receptor, eventually resulting in a fusion protein where the "tag" or a "tag combination" is fused to the N-terminus or C-terminus of the receptor. Lucy, FLAG^{®} and/or Rho tags can enhance the receptor trafficking to the cell membrane, hence they can assist in expression of a functional odorant receptor for *in vitro* cell based assay [Shepard, B. et al. PLoS One 8, e68758-e68758 (2013), and Zhuang, H. & Matsunami, H. J. Biol. Chem. 282, 15284-15293 (2007)].

"Geonol" and/ or "Geosmin" refers to (4S,4aS,8aR)-4,8a-Dimethyl-1,2,3,4,5,6,7,-octahydronaphthalen-4a-ol.

### Particular Embodiments

In particular the present invention makes use of the human olfactory receptors (ORs) OR2W1, OR1A1, OR2J3, OR4Q3, OR5K1, OR11A1, OR2M3, OR51E1, OR4S2, OR51I2, OR2H1, OR2W3, OR8G1, and corresponding mouse orthologs Olfr263, Olfr403, Olfr735, Olfr96, Olfr1193, Olfr641, Olfr137, Olfr173, Olfr164, and Olfr558, as well as mouse ORs Olfr1487, Olfr339, Olfr1126, Olfr93, Olfr398, Olfr120, Olfr1364, Olfr937, Olfr1322, and Olfr46 as receptors for the malodour-causing substances geonol, dimethyl trisulfide (DMTS), 1-octen-3-ol, butyric acid, 3-methyl-2-hexenoic acid, 3-hydroxy-3-methyl-hexanoic acid, and transpirol (3-methyl-3-sulfanylhexanol), as shown in Table 1a.

**Table 1a: Human odorant receptors and pairs with orthologous mouse receptors and their corresponding identity at the amino acid level as well as further mouse ORs; together with their respective agonists.**

| **Mouse Ortholog** | **Agonists** | **Human Ortholog** | **Agonists** | **Mouse-Human Amino Acid Identity** |
|---|---|---|---|---|
| Olfr263 | 1-octen-3-ol, DMTS, 3-hydroxy-3-methylhexanoic acid, transpirol, 3-methyl-2-hexenoic acid | OR2W1 | 1-octen-3-ol, DMTS, 3-hydroxy-3-methylhexanoic acid, transpirol, 3-methyl-2-hexenoic acid | 86% |
| Olfr403 | 1-octen-3-ol, geonol, DMTS, transpirol | OR1A1 | 1-octen-3-ol, geonol, DMTS, transpirol | 84% |
| Olfr137 | 1-octen-3-ol, transpirol | OR2J3 | 1-octen-3-ol, transpirol | 82% |
| Olfr735 | 1-octen-3-ol | OR4Q3 | 1-octen-3-ol | 82% |
| Olfr173 | 1-octen-3-ol, DMTS, transpirol | OR5K1 | 1-octen-3-ol, DMTS, transpirol | 85% |
| Olfr96 | geonol | OR11A1 | geonol | 75% |
| Olfr164 | geonol | OR2M3 | geonol | 75% |
| Olfr558 | butyric acid | OR51E1 | butyric acid | 94% |
| Olfr1193 | DMTS | OR4S2 | DMTS | 88% |
| Olfr641 | 3-methyl-2-hexenoic acid | OR5112 | 3-methyl-2-hexenoic acid | 89.10% |
| Olfr1487 | geonol | None | NA | NA |
| Olfr339 | geonol | None | NA | NA |
| Olfr1126 | 3-hydroxy-3-methylhexanoic acid | None | NA | NA |
| Olfr93 | 1-octen-3-ol | OR2H1 | NA | 81% |
| Olfr398 | 1-octen-3-ol | None | NA | NA |
| Olfr120 | 1-octen-3-ol | None | NA | NA |
| Olfr1364 | 1-octen-3-ol | OR2W3 | NA | 74% |
| Olfr937 | 1-octen-3-ol | OR8G1 | NA | 79% |
| Olfr1322 | geonol | None | NA | NA |
| Olfr46 | transpirol | None | NA | NA |

Table 1b states the corresponding SEQ ID NOs of ORs as applied according to the present invention:

**Table 1b: SEQ ID NOs of human and mouse odorant receptors**

| **Mouse Ortholog** | **SEQ ID NO:** | **Human Ortholog** | **SEQ ID NO:** |
|---|---|---|---|
| Olfr263 | 21 (NA) | OR2W1 | 1 (NA) |
| | 22 (AA) | | 2 (AA) |
| Olfr403 | 23 (NA) | OR1A1 | 3 (NA) |
| | 24 (AA) | | 4 (AA) |
| Olfr137 | 35 (NA) | OR2J3 | 5 (NA) |
| | 36 (AA) | | 6 (AA) |
| Olfr735 | 25 (NA) | OR4Q3 | 7 (NA) |
| | 26 (AA) | | 8 (AA) |
| Olfr173 | 37 (NA) | OR5K1 | 9 (NA) |
| | 38 (AA) | | 10 (AA) |
| Olfr96 | 27 (NA) | OR11A1 | 11 (NA) |
| | 28 (AA) | | 12 (AA) |
| Olfr164 | 39 (NA) | OR2M3 | 13 (NA) |
| | 40 (AA) | | 14 (AA) |
| Olfr558 | 29 (NA) | OR51E1 | 15 (NA) |
| | 30 (AA) | | 16 (AA) |
| Olfr1193 | 31 (NA) | OR4S2 | 17 (NA) |
| | 32 (AA) | | 18 (AA) |
| Olfr641 | 33 (NA) | OR5112 | 19 (NA) |
| | 34 (AA) | | 20 (AA) |
| Olfr1487 | 41 (NA) | None | |
| | 42 (AA) | | |
| Olfr339 | 43 (NA) | None | |
| | 44 (AA) | | |
| Olfr1126 | 45 (NA) | None | |
| | 46 (AA) | | |
| Olfr93 | 62 (NA) | OR2H1 | 56 (NA) |
| | 63 (AA) | | 57 (AA) |
| Olfr398 | 64 (NA) | None | |
| | 65 (AA) | | |
| Olfr120 | 66 (NA) | None | |
| | 67 (AA) | | |
| Olfr1364 | 68 (NA) | OR2W3 | 58 (NA) |
| | 69 (AA) | | 59 (AA) |
| Olfr937 | 70 (NA) | OR8G1 | 60 (NA) |
| | 71 (AA) | | 61 (AA) |
| Olfr1322 | 72 (NA) | None | |
| | 73 (AA) | | |
| Olfr46 | 74 (NA) | None | |
| | 75 (AA) | | |

| | | | |
|---|---|---|---|
| NA = nucleic acid sequence; AA = amino acid sequence | | | |

These receptors had not been previously associated with the malodor-causing substances selected from geonol, dimethyl trisulfide (DMTS), 1-octen-3-ol, butyric acid, 3-methyl-2-hexenoic acid, 3-hydroxy-3-methyl-hexanoic acid, and transpirol.

In one embodiment provided herein is an isolated nucleic acid molecule comprising a nucleic acid sequence having at least 70%, 75%, 80%, 85%, 90%, 95%, 96%, 97%, 98%, 99%, or 100% sequence identity to SEQ ID NO: 1, 3, 5, 7, 9, 11, 13, 15, 17, 19, 21, 23, 25, 27, 29, 31, 33, 35, 37, 39, 41, 43, 45, 56, 58, 60, 62, 64, 66, 68, 70, 72, 74, or the reverse complement thereof.

In one embodiment provided herein is an isolated nucleic acid sequence as described above which encodes a polypeptide comprising an amino acid sequence having at least 70%, 75%, 80%, 85%, 90%, 95%, 96%, 97%, 98%, 99%, or 100% sequence identity to SEQ ID NO: 2, 4, 6, 8, 10, 12, 14, 16, 18, 20, 22, 24, 26, 28, 30, 32, 34, 36, 38, 40, 42, 44, 46, 57, 59, 61, 63, 65, 67, 69, 71, 73, or 75.

In a further embodiment provided herein is an isolated polypeptide comprising an amino acid sequence having at least 70%, 75%, 80%, 85%, 90%, 95%, 96%, 97%, 98%, 99%, or 100% sequence identity to SEQ ID NO: 2, 4, 6, 8, 10, 12, 14, 16, 18, 20, 22, 24, 26, 28, 30, 32, 34, 36, 38, 40, 42, 44, 46, 57, 59, 61, 63, 65, 67, 69, 71, 73, or 75.

In one embodiment, a non-human organism or a host cell is transformed to express a polypeptide comprising an amino acid sequence having at least 70%, 75%, 80%, 85%, 90%, 95%, 96%, 97%, 98%, 99%, or 100% sequence identity to SEQ ID NO: 2, 4, 6, 8, 10, 12, 14, 16, 18, 20, 22, 24, 26, 28, 30, 32, 34, 36, 38, 40, 42, 44, 46, 57, 59, 61, 63, 65, 67, 69, 71, 73, or 75.

In one embodiment, a non-human organism or a host cell is transformed to express a polypeptide comprising an amino acid sequence that is identical to SEQ ID NO: 2, 4, 6, 8, 10, 12, 14, 16, 18, 20, 22, 24, 26, 28, 30, 32, 34, 36, 38, 40, 42, 44, 46, 57, 59, 61, 63, 65, 67, 69, 71, 73, or 75.

Further provided herein is an expression vector comprising a nucleic acid that encodes a polypeptide comprising an amino acid sequence having at least 70%, 75%, 80%, 85%, 90%, 95%, 96%, 97%, 98%, 99%, or 100% sequence identity to SEQ ID NO: 2, 4, 6, 8, 10, 12, 14, 16, 18, 20, 22, 24, 26, 28, 30, 32, 34, 36, 38, 40, 42, 44, 46, 57, 59, 61, 63, 65, 67, 69, 71, 73, or 75.

Also provided herein is an expression vector comprising a nucleic acid that comprises a nucleic acid sequence having at least 70%, 75%, 80%, 85%, 90%, 95%, 96%, 97%, 98%, 99%, or 100% sequence identity SEQ ID NO: 1, 3, 5, 7, 9, 11, 13, 15, 17, 19, 21, 23, 25, 27, 29, 31, 33, 35, 37, 39, 41, 43, 45, 56, 58, 60, 62, 64, 66, 68, 70, 72, 74, or the reverse complement thereof.

Also provided herein is an expression vector having a nucleic acid wherein the nucleic acid comprises a nucleotide sequence that is identical to SEQ ID NO: 1, 3, 5, 7, 9, 11, 13, 15, 17, 19, 21, 23, 25, 27, 29, 31, 33, 35, 37, 39, 41, 43, 45, 56, 58, 60, 62, 64, 66, 68, 70, 72, 74, or the reverse complement thereof.
More particularly, the present invention relates to the following specific embodiments:
1. A method for identifying a compound that binds, suppresses, blocks, inhibits, and/or modulates the activity of an olfactory receptor (OR) that is activated by a laundry malodour, a moldy malodour, and/or a sweat malodor-causing substance comprising:
   a. contacting a test substance and said laundry malodour, moldy malodour and/or sweat malodor-causing substance to at least one, in particular one single, olfactory receptor polypeptide selected from the group consisting of OR2W1, OR1A1, OR2J3, OR4Q3, OR5K1, OR11A1, OR2M3, OR51E1, OR4S2, OR51I2, OR2H1, OR2W3, OR8G1, Olfr263, Olfr403, Olfr735, Olfr96, Olfr1193, Olfr641, Olfr137, Olfr173, Olfr164, Olfr1487, Olfr339, Olfr1126, Olfr558, Olfr93, Olfr398, Olfr120, Olfr1364, Olfr937, Olfr1322 and Olfr46 and chimera or functional fragments thereof; or to at least one olfactory receptor polypeptide having at least 70%, as for example 75%, 80%, 85%, 90%, and particularly 95%, 96%, 97%, 98%, 99%, or 100% amino acid sequence identity to at least one, in particular one single of the aforementioned olfactory receptor polypeptides; and
   b. measuring the response of said at least one, in particular one single, olfactory receptor to the malodor-causing substance by measuring the response of said olfactory receptor in the presence and absence of the test substance.

   A particular subgroup of ORs comprises human ORs OR2W1, OR1A1, OR2J3, OR4Q3, OR5K1, OR11A1, OR2M3, OR51E1, OR4S2, OR51I2, OR2H1, OR2W3, and OR8G1.
   Another particular subgroup of ORs comprises the mouse ORs Olfr263, Olfr403, Olfr735, Olfr96, Olfr1193, Olfr641, Olfr137, Olfr173, Olfr164, Olfr1487, Olfr339, Olfr1126, Olfr558, Olfr93, Olfr398, Olfr120, Olfr1364, Olfr937, Olfr1322, and Olfr46.
2. The method of embodiment 1, further comprising
   c. identifying a test substance that modulates the response of said at least one, in particular one single, olfactory receptor on the basis of the response that was measured in the presence and absence of the test substance; and
   d. selecting the identified test substance as a compound that modulates the response of said at least one, in particular one single, olfactory receptor to the malodor-causing substance.
3. The method of embodiment 1 or 2, wherein said malodor-causing substance is selected from the group consisting of geonol, dimethyl trisulfide (DMTS), 1-octen-3-ol, butyric acid, 3-methyl-2-hexenoic acid, 3-hydroxy-3-methyl-hexanoic acid, transpirol, and mixtures of at least two, like two or three, of said substances.
4. The method of anyone of the preceding embodiments, wherein said at least one olfactory receptor polypeptide
   a. comprises an amino acid sequence having at least 70%, as for example 75%, 80%, 85%, 90%, and particularly 95%, 96%, 97%, 98%, 99%, or 100%, sequence identity to SEQ ID NO: 2, 4, 6, 8, 10, 12, 14, 16, 18, 20, 22, 24, 26, 28, 30, 32, 34, 36, 38, 40, 42, 44, 46, 57, 59, 61, 63, 65, 67, 69, 71, 73, or 75; and/or
   b. is encoded by a nucleic acid molecule comprising a nucleotide sequence having at least 70%, as for example 75%, 80%, 85%, 90%, and particularly 95%, 96%, 97%, 98%, 99%, or 100%, sequence identity to SEQ ID NO: 1, 3, 5, 7, 9, 11, 13, 15, 17, 19, 21, 23, 25, 27, 29, 31, 33, 35, 37, 39, 41, 43, 45, 56, 58, 60, 62, 64, 66, 68, 70, 72, 74, or the reverse complement thereof.

   A particular subgroup of ORs of this embodiment is or is derived from SEQ ID NO: 2, 4, 6, 8, 10, 12, 14, 16, 18, 20, 57, 59, or 61.
   Another particular subgroup of ORs of this embodiment is encoded by a nucleic acid molecule comprising a nucleotide sequence of SEQ ID NO: 1, 3, 5, 7, 9, 11, 13, 15, 17, 19, 56, 58, 60 or a nucleotide sequence derived therefrom.
5. The method of embodiment 4, wherein the polypeptide
   a. comprises an amino acid sequence having at least 90%, 95%, 98%, 99% or 100% sequence identity to SEQ ID NO: 2, 4, 6, 8, 10, 12, 14, 16, 18, 20, 22, 24, 26, 28, 30, 32, 34, 36, 38, 40, 42, 44, 46, 57, 59, 61, 63, 65, 67, 69, 71, 73, or 75; and/or
   b. is encoded by a nucleic acid molecule comprising a nucleotide sequence having at least 90%, 95%, 98%, 99% or 100% sequence identity to SEQ ID NO: 1, 3, 5, 7, 9, 11, 13, 15, 17, 19, 21, 23, 25, 27, 29, 31, 33, 35, 37, 39, 41, 43, 45, 56, 58, 60, 62, 64, 66, 68, 70, 72, 74, or the reverse complement thereof.

   A particular subgroup of ORs of this embodiment is or is derived from SEQ ID NO: 2, 4, 6, 8, 10, 12, 14, 16, 18, 20, 57, 59, or 61.
   Another particular subgroup of ORs of this embodiment is encoded by a nucleic acid molecule comprising a nucleotide sequence of SEQ ID NO: 1, 3, 5, 7, 9, 11, 13, 15, 17, 19, 56, 58, 60 or a nucleotide sequence derived therefrom.
6. The method of any one of the embodiments 1 to 5, wherein said malodor-causing substance is a laundry malodor causing substance selected from the group consisting of geonol, DMTS, 1-octen-3-ol, and mixtures of at least two of said substances.
7. The method of any one of the embodiments 1 to 5, wherein said malodor-causing substance is a moldy malodor causing substance selected from the group consisting of geonol, DMTS, 1-octen-3-ol, and mixtures of at least two of said substances.
8. The method of anyone of the embodiments 1 to 5, wherein said malodor-causing substance is a sweat malodor causing substance selected from the group consisting of butyric acid, 3-methyl-2-hexenoic acid, 3-hydroxy-3-methyl-hexanoic acid, transpirol, and mixtures of at least two of said substances.
9. The method of embodiment 6 or 7, wherein the olfactory receptor polypeptide is selected from the group consisting of OR2W1, OR1A1, OR2J3, OR4Q3, OR5K1, OR11A1, OR2M3, OR4S2, Olfr263, Olfr403, Olfr735, Olfr1193, Olfr137, Olfr173, Olfr164, Olfr1487, Olfr339, Olfr96, Olfr1322, Olfr93, Olfr398, Olfr120, Olfr1364 and Olfr937; chimera and functional fragments thereof; and olfactory receptor polypeptides having at least 70%, as for example 75%, 80%, 85%, 90%, and particularly 95%, 96%, 97%, 98%, 99%, or 100%, amino acid sequence identity to at least one of the aforementioned olfactory receptor polypeptides. A particular subgroup of ORs of this embodiment is selected from OR2W1, OR1A1, OR2J3, OR4Q3, OR5K1, OR11A1, OR2M3 and OR4S2.
10. The method of embodiment 9, wherein the malodour causing substance comprises 1-octen-3-ol and the olfactory receptor is selected from the group consisting of OR2W1, OR1A1, OR2J3, OR5K1, OR4Q3, Olfr263, Olfr403, Olfr137, Olfr173, Olfr735, Olfr93, Olfr398, Olfr120, Olfr1364 and Olfr937; chimera and functional fragments thereof; and olfactory receptor polypeptides having at least 70%, as for example 75%, 80%, 85%, 90%, and particularly 95%, 96%, 97%, 98%, 99%, or 100% , amino acid sequence identity to at least one of the aforementioned olfactory receptor polypeptides. A particular subgroup of ORs of this embodiment is selected from OR2W1, OR1A1, OR2J3, OR5K1, and OR4Q3.
11. The method of embodiment 9, wherein the malodour causing substance comprises geonol and the olfactory receptor is selected from the group consisting of OR1A1, OR11A1, OR2M3, Olfr403, Olfr164, Olfr1487, Olfr339, Olfr96 and Olfr1322; chimera and functional fragments thereof; and olfactory receptor polypeptides having at least 70%, as for example 75%, 80%, 85%, 90%, and particularly 95%, 96%, 97%, 98%, 99%, or 100%, amino acid sequence identity to at least one of the aforementioned olfactory receptor polypeptides. A particular subgroup of ORs of this embodiment is selected from OR11A1, OR2M3 and OR1A1.
12. The method of embodiment 9, wherein the malodour causing substance comprises DMTS and the olfactory receptor is selected from the group consisting of OR2W1, OR1A1, OR5K1, OR4S2, Olfr263, Olfr1193, Olfr173 and Olfr403; chimera and functional fragments thereof; and olfactory receptor polypeptides having at least 70%, as for example 75%, 80%, 85%, 90%, and particularly 95%, 96%, 97%, 98%, 99%, or 100%, amino acid sequence identity to at least one of the aforementioned olfactory receptor polypeptides. A particular subgroup of ORs of this embodiment is selected from OR2W1, OR1A1, OR5K1 and OR4S2.
13. The method of embodiment 8, wherein the olfactory receptor polypeptide is selected from the group consisting of OR2W1, OR1A1, OR2J3, OR5K1, OR51E1, OR51I2, Olfr263, Olfr403, Olfr641, Olfr137, Olfr173, Olfr1126, Olfr558 and Olfr46; chimera and functional fragments thereof; and olfactory receptor polypeptides having at least 70%, as for example 75%, 80%, 85%, 90%, and particularly 95%, 96%, 97%, 98%, 99%, or 100%, amino acid sequence identity to at least one of the aforementioned olfactory receptor polypeptides. A particular subgroup of ORs of this embodiment is selected from OR2W1, OR1A1, OR2J3, OR5K1, OR51E1 and OR51I2.
14. The method of embodiment 13, wherein the malodour causing substance comprises butyric acid and the olfactory receptor is selected from the group consisting of OR51E1, and Olfr558; chimera and functional fragments thereof; and olfactory receptor polypeptides having at least 70%, as for example 75%, 80%, 85%, 90%, and particularly 95%, 96%, 97%, 98%, 99%, or 100%, amino acid sequence identity to at least one of the aforementioned olfactory receptor polypeptides. A particular subgroup of ORs of this embodiment is OR51E1.
15. The method of embodiment 13, wherein the malodour causing substance comprises 3-methyl-2-hexenoic acid, and the olfactory receptor is selected from the group consisting of OR51I2, OR2W1, Olfr641 and Olfr263; chimera and functional fragments thereof; and olfactory receptor polypeptides having at least 70%, as for example 75%, 80%, 85%, 90%, and particularly 95%, 96%, 97%, 98%, 99%, or 100%, amino acid sequence identity to at least one of the aforementioned olfactory receptor polypeptides.
16. The method of embodiment 13, wherein the malodour causing substance comprises 3-hydroxy-3-methyl-hexanoic acid, and the olfactory receptor is selected from the group consisting of OR2W1, Olfr1126 and Olfr263; chimera and functional fragments thereof; and olfactory receptor polypeptides having at least 70, as for example 75%, 80%, 85%, 90%, and particularly 95%, 96%, 97%, 98%, 99%, or 100%, amino acid sequence identity to at least one of the aforementioned olfactory receptor polypeptides.
17. The method of embodiment 13, wherein the malodour causing substance comprises transpirol and the olfactory receptor is selected from the group consisting of OR2W1, OR1A1, OR2J3, OR5K1, Olfr263, Olfr137, Olfr173, Olfr403 and Olfr46; chimera and functional fragments thereof; and olfactory receptor polypeptides having at least 70%, as for example 75%, 80%, 85%, 90%, and particularly 95%, 96%, 97%, 98%, 99%, or 100%, amino acid sequence identity to at least one of the aforementioned olfactory receptor polypeptides. A particular subgroup of ORs of this embodiment is selected from OR2W1, OR1A1, OR2J3 and OR5K1.
18. A method for identifying a compound that binds, suppresses, blocks, inhibits, and/or modulates the activity of at least one olfactory receptor that is activated by a malodor-causing substance, wherein the receptor is a polypeptide
   a. that comprises an amino acid sequence having at least 70%, as for example 75%, 80%, 85%, 90%, and particularly 95%, 96%, 97%, 98%, 99%, or 100%, sequence identity to SEQ ID NO: 2, 4, 6, 8, 10, 12, 14, 16, 18, 20, 22, 24, 26, 28, 30, 32, 34, 36, 38, 40, 42, 44, 46, 57, 59, 61, 63, 65, 67, 69, 71, 73, or 75; and/or
   b. is encoded by a nucleic acid molecule comprising a nucleotide sequence having at least 70%, as for example 75%, 80%, 85%, 90%, and particularly 95%, 96%, 97%, 98%, 99%, or 100%, sequence identity to SEQ ID NO: 1, 3, 5, 7, 9, 11, 13, 15, 17, 19, 21, 23, 25, 27, 29, 31, 33, 35, 37, 39, 41, 43, 45, 56, 58, 60, 62, 64, 66, 68, 70, 72, 74, or the reverse complement thereof,

   wherein the method comprises:
      1. contacting the receptor, or a chimera or fragment thereof with a compound;
      2. determining whether the compound has an effect on the activity of the receptor; and
   wherein said malodor-causing substance is selected from the group consisting of geonol, DMTS, 1-octen-3-ol, butyric acid, 3-methyl-2-hexenoic acid, 3-hydroxy-3-methyl-hexanoic acid, transpirol, and mixtures of at least two of said substances.
      A particular subgroup of ORs of this embodiment is or is derived from SEQ ID NO: 2, 4, 6, 8, 10, 12, 14, 16, 18, 20, 57, 59, or 61.
      Another particular subgroup of ORs of this embodiment is encoded by a nucleic acid molecule comprising a nucleotide sequence of SEQ ID NO: 1, 3, 5, 7, 9, 11, 13, 15, 17, 19, 56, 58, 60 or a nucleotide sequence derived therefrom.
19. A method for identifying a compound that binds, suppresses, blocks, inhibits, and/or modulates the activity of an olfactory receptor that is activated by a laundry malodour, a moldy malodour and/or sweat malodor-causing substance, selected from the group consisting of geonol, DMTS, 1-octen-3-ol, butyric acid, 3-methyl-2-hexenoic acid, 3-hydroxy-3-methyl-hexanoic acid, transpirol, and mixtures of at least two of said substances comprising:
   (i) contacting a cell line that expresses at least one olfactory receptor polypeptide selected from the group consisting of OR2W1, OR1A1, OR2J3, OR4Q3, OR5K1, OR11A1, OR2M3, OR51E1, OR4S2, OR51I2, OR2H1, OR2W3, OR8G1, Olfr263, Olfr403, Olfr735, Olfr96, Olfr1193, Olfr641, Olfr137, Olfr173, Olfr164, Olfr1487, Olfr339, Olfr1126, Olfr558, Olfr93, Olfr398, Olfr120, Olfr1364, Olfr937, Olfr1322 and Olfr46 and chimera or functional fragments thereof; or to at least one olfactory receptor polypeptide having at least 70% amino acid sequence identity to at least one of the aforementioned olfactory receptor polypeptides; with at least one compound;
   (ii) screening for compounds that bind, suppress, block, inhibit, and/or modulate the activity of said olfactory receptor polypeptide; and
   (iii) identifying a compound that putatively modulates laundry malodor, moldy malodor and/or sweat malodor if it binds, suppresses, blocks, inhibits, and/or modulates the activity of said receptor polypeptide.

   A particular subgroup of ORs comprises OR2W1, OR1A1, OR2J3, OR4Q3, OR5K1, OR11A1, OR2M3, OR51E1, OR4S2, OR51I2, OR2H1, OR2W3, and OR8G1.
   Another particular subgroup of ORs are Olfr263, Olfr403, Olfr735, Olfr96, Olfr1193, Olfr641, Olfr137, Olfr173, Olfr164, Olfr1487, Olfr339, Olfr1126, Olfr558, Olfr93, Olfr398, Olfr120, Olfr1364, Olfr937, Olfr1322, and Olfr46.
20. An isolated polypeptide comprising an amino acid sequence having at least 70%, 75%, 80%, 85%, 90%, 95%, 96%, 97%, 98%, 99%, or 100% sequence identity to SEQ ID NO: 2, 4, 6, 8, 10, 12, 14, 16, 18, 20, 22, 24, 26, 28, 30, 32, 34, 36, 38, 40, 42, 44, 46, 57, 59, 61, 63, 65, 67, 69, 71, 73, or 75.
21. An isolated nucleic acid molecule comprising a nucleic acid sequence encoding the polypeptide of embodiment 20.
22. The isolated nucleic acid molecule of embodiment 20, comprising a nucleic acid sequence having at least 70%, 75%, 80%, 85%, 90%, 95%, 96%, 97%, 98%, 99%, or 100% sequence identity to SEQ ID NO: 1, 3, 5, 7, 9, 11, 13, 15, 17, 19, 21, 23, 25, 27, 29, 31, 33, 35, 37, 39, 41, 43, 45, 56, 58, 60, 62, 64, 66, 68, 70, 72, 74, or the reverse complement thereof.
23. A recombinant nucleic acid molecule comprising
   a. a nucleic acid comprising a tag combination that comprises at least one of a Lucy tag, a FLAG^{®} tag, and/or a Rho tag; and
   b. a nucleic acid encoding a receptor selected from the group consisting of OR2W1, OR1A1, OR2J3, OR4Q3, OR5K1, OR11A1, OR2M3, OR51E1, OR4S2, OR51I2, OR2H1, OR2W3, OR8G1, Olfr263, Olfr403, Olfr735, Olfr96, Olfr1193, Olfr641, Olfr137, Olfr173, Olfr164, Olfr1487, Olfr339, Olfr1126, Olfr558, Olfr93, Olfr398, Olfr120, Olfr1364, Olfr937, Olfr1322 and Olfr46, or the complement thereof.

   A particular subgroup of ORs are OR2W1, OR1A1, OR2J3, OR4Q3, OR5K1, OR11A1, OR2M3, OR51E1, OR4S2, OR51I2, OR2H1, OR2W3, and OR8G1.
   Another particular subgroup of ORs are Olfr263, Olfr403, Olfr735, Olfr96, Olfr1193, Olfr641, Olfr137, Olfr173, Olfr164, Olfr1487, Olfr339, Olfr1126, Olfr558, Olfr93, Olfr398, Olfr120, Olfr1364, Olfr937, Olfr1322, and Olfr46.
24. The recombinant nucleic acid molecule of embodiment 12, wherein the Lucy tag comprises SEQ ID NO: 51, the FLAG^{®} tag comprises SEQ ID NO: 47, and the Rho tag comprises SEQ ID NO: 49.
25. An expression vector comprising the nucleic acid of any one of embodiments 21 to 24.
26. A non-human host organism or a host cell that has been modified to express a receptor that is selected from the group consisting of OR2W1, OR1A1, OR2J3, OR4Q3, OR5K1, OR11A1, OR2M3, OR51E1, OR4S2, OR51I2, OR2H1, OR2W3, OR8G1, Olfr263, Olfr403, Olfr735, Olfr96, Olfr1193, Olfr641, Olfr137, Olfr173, Olfr164, Olfr1487, Olfr339, Olfr1126, Olfr558, Olfr93, Olfr398, Olfr120, Olfr1364, Olfr937, Olfr1322 and Olfr46.
   A particular subgroup of ORs are OR2W1, OR1A1, OR2J3, OR4Q3, OR5K1, OR11A1, OR2M3, OR51E1, OR4S2, OR51I2, OR2H1, OR2W3, and OR8G1.
   Another particular subgroup of ORs Olfr263, Olfr403, Olfr735, Olfr96, Olfr1193, Olfr641, Olfr137, Olfr173, Olfr164, Olfr1487, Olfr339, Olfr1126, Olfr558, Olfr93, Olfr398, Olfr120, Olfr1364, Olfr937, Olfr1322, and Olfr46.
27. The non-human host organism or host cell of embodiment 26, wherein the receptor comprises the polypeptide of embodiment 19 or a polypeptide encoded by the nucleic acid of any one of embodiments 21 to 24.
28. A non-human host organism or a host cell comprising
   a. the nucleic acid of any one of embodiments 21 to 24; or
   b. the expression vector of embodiment 25.
29. The non-human host organism or host cell of any one of embodiments 26 to 28, wherein the cell is a eukaryotic cell.
30. The non-human host organism or host cell of any one of embodiments 26 to 28, wherein the cell is a prokaryotic cell.
31. The non-human host organism or host cell of any one of embodiments 26 to 29, wherein the non-human host organism or host cell is selected from the group consisting of HEK293, CHO, *Xenopus oocytes*, COS, yeast and cells derived from the olfactory placode.
32. Use of a polypeptide that can be activated by a substance, selected from the group consisting of geonol, DMTS, 1-octen-3-ol, butyric acid, 3-methyl-2-hexenoic acid, 3-hydroxy-3-methyl-hexanoic acid, transpirol, and mixtures of at least two of said substances, comprising an amino acid sequence having at least 70%, 75%, 80%, 85%, 90%, 95%, 96%, 97%, 98%,99%, or 100% sequence identity to SEQ ID NO: 2, 4, 6, 8, 10, 12, 14, 16, 18, 20, 22, 24, 26, 28, 30, 32, 34, 36, 38, 40, 42, 44, 46, 57, 59, 61, 63, 65, 67, 69, 71, 73, or 75 for identifying a malodor modulating compound.

A particular subgroup of ORs of this embodiment is or is derived from SEQ ID NO: 2, 4, 6, 8, 10, 12, 14, 16, 18 or 20.

As illustrated herein, the ORs as identified according to the present invention are either specific for one single MCS or they may be activated by a panel of structurally different MCS. Therefore, the present invention allows different strategies for identifying a suitable modulating substance.

For example, if an inhibitor just for Geonol is of interest making use the OR11A1 or OR2M3 in the assay method of the invention may be advisable.

For example, if an inhibitor for DTMS, and 1-octen-3ol and optionally for Geonol is of interest making use the OR2W3, OR5K1 or optionally OR1A1 in the assay method of the invention may be advisable. Other assay strategies for identifying modulators antagonizing laundry malodor, moldy malodor or sweat malodor causing substances or even for identifying modulators which antagonize laundry malodor, moldy malodor and sweat malodor causing substances become evident from the experimental results disclosed herein.

Further provided is any one of a number of malodor modulating compounds that binds, suppresses, blocks, inhibits, and/or modulates the activity of an olfactory receptor that is activated by a malodor-causing substance selected from the group consisting of geonol, DMTS, 1-octen-3-ol, butyric acid, 3-methyl-2-hexenoic acid, 3-hydroxy-3-methyl-hexanoic acid, transpirol, and mixtures of at least two of said substances, and that is identified by the methods disclosed herein.

According to one embodiment a malodor modulating compound is provided that binds, suppresses, blocks, inhibits, and/or modulates the activity of at least one olfactory receptor selected from the group consisting of OR2W1, OR1A1, OR2J3, OR4Q3, OR5K1, OR11A1, OR2M3, OR51E1, OR4S2, OR51I2, OR2H1, OR2W3, OR8G1, Olfr263, Olfr403, Olfr735, Olfr96, Olfr1193, Olfr641, Olfr137, Olfr173, Olfr164, Olfr1487, Olfr339, Olfr1126, Olfr558, Olfr93, Olfr398, Olfr120, Olfr1364, Olfr937, Olfr1322 and Olfr46, and that is identified by the methods disclosed herein. A particular subgroup of ORs of this embodiment is or is derived from OR2W1, OR1A1, OR2J3, OR4Q3, OR5K1, OR11A1, OR2M3, OR51E1, OR4S2, OR51I2, OR2H1, OR2W3, and OR8G1. Another particular subgroup of ORs are Olfr263, Olfr403, Olfr735, Olfr96, Olfr1193, Olfr641, Olfr137, Olfr173, Olfr164, Olfr1487, Olfr339, Olfr1126, Olfr558, Olfr93, Olfr398, Olfr120, Olfr1364, Olfr937, Olfr1322 and Olfr46.

The test substances to be tested in the method of the present invention are not particularly limited. The test substance may be a naturally occurring substance or a chemically or biologically synthesized artificial substance. The test substance may be a compound or a mixture of compounds.

In order to identify unknown receptors specific for the MCS geonol, DMTS, 1-octen-3-ol, butyric acid, 3-methyl-2-hexenoic acid, 3-hydroxy-3-methyl-hexanoic acid, or transpirol, said MCS compounds can be used to screen dissociated olfactory sensory neurons (OSNs). Said MCS compounds can be further used for cell-based dose-response experiments performed on specific receptors for said MCS to assess both specificity and sensitivity of the receptors.

In one aspect, provided herein are methods to identify mammalian odorant receptors for malodour modulating compounds and the use of the receptors for screening, particularly for high throughput screening (HTS) of malodor modulators (e.g. that bind, suppress, block, inhibit and/or modulate the activity of an OR).

### Methods of Monitoring OR Activity

As long as the function of the OR is not impaired, it may be used in any form in a method of the present invention. For example, the OR may be use as follows: tissues or cells which intrinsically express an OR such as olfactory sensory neurons isolated from living bodies and cultured products thereof; olfactory cell membrane bearing the OR; recombinant cells genetically modified so as to express the OR and cultured products thereof; membrane of the recombinant cells; and artificial lipid bilayer membrane carrying the OR.

In a further embodiment, indicators for monitoring the activity of olfactory receptors are selected from a fluorescent calcium indicator dye, a calcium indicator protein (e.g. GcaMP, a genetically encoded calcium indicator), a fluorescent cAMP indicator, a cell mobilization assay, a cellular dynamic mass redistribution assay, a label-free cell based assay, a cAMP response element (CRE) mediated reporter protein, a biochemical cAMP HTRF assay, a beta-arrestin assay, or an electrophysiological recording. Particularly, a calcium indicator dye is selected that can be used to monitor the activity of olfactory receptors expressed on the membrane of the olfactory neurons (e.g., Fura-2 AM).

In a particular embodiment, compounds are screened sequentially and the odorant-dependent changes in calcium dye fluorescence are measured using a fluorescent microscope or fluorescent-activated cell sorter (FACS).

In a further embodiment, molecular 3D receptor modeling of olfactory receptors is used to assess the binding potential *in silico* and to identify compounds that may activate, mimic, block, inhibit, modulate, and/or enhance the activity of an olfactory receptor.

As an example, olfactory neurons activated by geonol, DMTS, 1-octen-3-ol, butyric acid, 3-methyl-2-hexenoic acid, 3-hydroxy-3-methyl-hexanoic acid or transpirol are isolated using either a glass capillary attached to a micromanipulator or a FACS machine. Mouse olfactory sensory neurons are screened by Ca²⁺ imaging similar to procedures previously described [Malnic, B., et al. Cell 96, 713-723 (1999); Araneda, R. C. et al. J. Physiol. 555, 743-756 (2004); and WO2014/210585 hereby incorporated by reference in its entirety]. Particularly, a motorized movable microscope stage is used to increase the number of cells that can be screened to at least 1,500 per experiment. Since there are approximately 1,200 different olfactory receptors in the mouse and each olfactory sensory neurons expresses only 1 of 1,200 olfactory receptor genes, this screening capacity will cover virtually the entire mouse odorant receptor repertoire. In other words, the combination of calcium imaging for high-throughput olfactory sensory neuron screening leads to the identification of nearly all of the odorant receptors that respond to a particular profile of odorants. In a particular aspect, odorant receptors that respond to geonol, DMTS, 1-octen-3-ol, butyric acid, 3-methyl-2-hexenoic acid, 3-hydroxy-3-methyl-hexanoic acid or transpirol can be isolated. For example, at least one neuron is isolated.

For calcium imaging of olfactory neurons, the main olfactory epithelium may be dissected from a mouse before neuronal dissociation. Dissected olfactory epithelium may then be transferred to a dissociation buffer for mechanical and enzymatic dissociation. Dissociated neurons may then be seeded onto a coverslip allowing the screening of thousands of cells by fluorescence microscopy and the cells may be loaded with a calcium sensitive dye (Fura-2 AM) for example for about 30 minutes at 31 °C and transferred onto the microscope ready for screening. Cells are stimulated by perfusing diluted solutions of odorants (in physiological saline) over the dissociated olfactory neurons. The rare cells that respond to the malodor compound are identified by for example stimulating the receptors with 50 µM of the malodor compounds and then by monitoring the intracellular Ca²⁺ flux indicated by changes in Fura-2 fluorescence. After analysis, responding cells may be retrieved from a glass coverslip with a suction micropipette. Isolated cells are then pooled into one sample or treated individually for subsequent identification of the odorant receptor genes expressed as mRNA in the responding cells.

In a particular embodiment, the mRNAs of olfactory neurons are purified and amplified according to the method generally described in Marko, N. F., et al., (2005) A robust method for the amplification of RNA in the sense orientation. BMC genomics, 6, 27; doi:10.1186/1471-2164-6-27 (Eberwine method). At least a portion of the transcriptome (up to including the entire transcriptome) is sequenced using Next-Generation Sequencing (NGS) technologies or hybridized to known genes using Microarray technologies. NGS is generally discussed and described in Metzker, M. L. Nat. Rev. Genet. 11, 31-46 (2010). In a particular embodiment, a minimum of 5 neurons presenting the same response profile are pooled. The mRNAs are released by cell lysis immediately after picking; no DNAse and no purification steps are carried out. The mRNA are amplified by two consecutive rounds of *in vitro* transcription (IVT). The amplification may be done according to MesageAmpII aRNA kit (Ambion, AMA1751) with the following parameters: two rounds of consecutive 14 hour long IVT.

In a further embodiment, the mRNA of a single olfactory neuron is purified and amplified with LD-PCR (Long Distance Polymerase Chain Reaction) based methods such as the one described in NGS-ready kits (e.g., Clontech/Takara, SMARTer^{®} Ultra^{®} Low Input RNA Kit for Sequencing - v3, cat. 634848). Single cell mRNA is first reverse transcribed into the corresponding cDNA, which subsequently is amplified with 18 PCR cycles and serves as NGS sample for transcriptome sequencing.

In yet another embodiment, the identity of a group or gene family of olfactory receptors for geonol, DMTS, 1-octen-3-ol, butyric acid, 3-methyl-2-hexenoic acid, 3-hydroxy-3-methyl-hexanoic acid or transpirol is determined (e.g., up to as many as the number of neurons picked) by comparing the results of the NGS reads obtained from the isolated activated olfactory sensory neurons to a reference genome sequence of the same species. Particularly, the putative receptors for geonol, DMTS, 1-octen-3-ol, butyric acid, 3-methyl-2-hexenoic acid, 3-hydroxy-3-methyl-hexanoic acid or transpirol will be the most highly abundant olfactory receptor mRNA in the olfactory neuron-derived NGS sample or present in more than one independent biological replicate. Because of the combinatorial nature of the olfactory code (one compound activates many ORs and one OR can be activated by many compounds), pooling several neurons activated by given compounds allows the retrieval of virtually all of the receptors responsible for the perception of these molecules in a single NGS experiment. Pooling functionally similar neurons thus greatly improves the deorphanization throughput and speed.

Standard bioinformatics tools are then used to identify the most closely related human odorant receptor(s) to other putative mammalian (non-human) receptor(s) for geonol, DMTS, 1-octen-3-ol, butyric acid, 3-methyl-2-hexenoic acid, 3-hydroxy-3-methyl-hexanoic acid or transpirol under the assumption that homologous sequence receptors retain similar function. Several methods successfully identify human OR-ligand pairs based on this assumption [Armelin-Correa and Malnic (2017)] and up to 80% of mouse-human orthologs appear to maintain similar functional response profiles [Adipietro, K. A, et al. PLoS Genet. 8, e1002821-e1002821 (2012)]. Default parameters of BLASTP and/or BLASTN algorithm, or other ortholog pair identification algorithms such as InParanoid may be used.

The human or non-human mammalian receptors for geonol, DMTS, 1-octen-3-ol, butyric acid, 3-methyl-2-hexenoic acid, 3-hydroxy-3-methyl-hexanoic acid or transpirol may be adapted to a functional assay that can be used to identify compounds that bind, suppress, block, inhibit, and/or modulate the activity of the olfactory receptors. In particular, the assay may be a cell-based assay or a binding assay and the method for identifying compounds may be a high-throughput screening assay. More particularly, provided herein are receptor-based assays adaptable for high-throughput screening of receptors with compound libraries for the discovery of modulating compounds (e.g., binding, blocking, inhibiting, suppressing and masking).

In a particular embodiment, geonol, DMTS, 1-octen-3-ol, butyric acid, 3-methyl-2-hexenoic acid, 3-hydroxy-3-methyl-hexanoic acid or transpirol receptor gene sequences are identified from geonol, DMTS, 1-octen-3-ol, butyric acid, 3-methyl-2-hexenoic acid, 3-hydroxy-3-methyl-hexanoic acid or transpirol -sensitive cells as follows: Pooled neurons are heated to 75°C for 10 minutes to break the cell membrane and render their mRNA available for amplification. This amplification step is important when applying NGS technologies with limited amount of starting material, typically between 1 to 15 cells. A linear amplification according to the Eberwine method (IVT) ensures the maintenance of the relative transcription levels of expressed genes. Two consecutive overnight (14h) rounds of *in vitro* transcription are used to yield sufficient amounts of cRNA; Amplified cRNA is then used to generate an Illumina HiSeq cDNA library. The resulting short sequences of typically 75 to 150 base pairs (commonly referred to as "reads") are aligned against the reference genome of the mouse (such as UCSC version mm9 or mm10) in order to build the full transcriptome of these cells. Quantitative analysis of the transcriptome data yields a list of transcribed odorant receptor genes and their respective expression levels. Odorant receptor genes that show the most abundant levels of mRNA (most abundant "reads") or are present in more than one replicate experiment are considered putative geonol, DMTS, 1-octen-3-ol, butyric acid, 3-methyl-2-hexenoic acid, 3-hydroxy-3-methyl-hexanoic acid or transpirol receptors.

The predicted mouse OR genes are then used to mine the latest versions of both the mouse and human genome databases in order to identify the most closely related receptors (i.e. highest sequence similarity) in mouse (paralogous genes) and in human (orthologous genes). This process may be performed using the BLAST search algorithm (publically available at the NCBI website), a sequence similarity search tool, where every putative gene sequence previously obtained from the initial transcriptome analysis is used as a query sequence. The newly identified genes identified from this data mining process are considered to be potential geonol, DMTS, 1-octen-3-ol, butyric acid, 3-methyl-2-hexenoic acid, 3-hydroxy-3-methyl-hexanoic acid or transpirol receptors under the assumption that paralogous and orthologous genes are highly likely to possess similar activities. In a particular embodiment, pairwise comparison of sequence homology is carried out to identify closely related receptors in mouse and humans and the receptors are identified as described in WO2014/210585. Other approaches may also be used such as RT-PCR and microarray approaches.

In a further embodiment, to complete the deorphanization process, the candidate OR genes are further expressed *in vitro* for confirmation of activity against the compounds used to isolate the olfactory sensory neurons and other structurally-related compounds of interest. The mouse receptors identified from isolated olfactory neurons that respond to geonol, DMTS, 1-octen-3-ol, butyric acid, 3-methyl-2-hexenoic acid, 3-hydroxy-3-methyl-hexanoic acid or transpirol are modified at their N-terminus with short polypeptide sequences (e.g., FLAG^{®} (SEQ ID NO: 48), Rho (SEQ ID NO: 50; 20 first amino acids of the bovine rhodopsin receptor), and/or Lucy (SEQ ID NO: 52; cleavable leucine-rich signal peptide sequence) tags), transiently expressed in HEK 293T cells, and stimulated separately with geonol, DMTS, 1-octen-3-ol, butyric acid, 3-methyl-2-hexenoic acid, 3-hydroxy-3-methyl-hexanoic acid or transpirol to confirm their identity as bona fide geonol, dimethyl trisulfide (DMTS), 1-octen-3-ol, butyric acid, 3-methyl-2-hexenoic acid, 3-hydroxy-3-methyl-hexanoic acid or transpirol receptors. In a further embodiment, an RTP1 gene can also be expressed in the cell lines whether through activation of the endogenous RTP1 gene or through transformation. Co-expression of the human G alpha subunit Gα_{olf} in this cell-based assay activates the Gs transduction pathway that leads to an internal cAMP increase upon binding to the appropriate ligand. Alternatively, co-expression of the human G alpha subunit Gα₁₅ in the cell based assay activates the Gq transduction pathway that leads to an internal Ca²⁺ increase upon binding to the appropriate ligand. The above process and the results obtained so far serve to validate the process for rapid and reliable identification of mammalian odorant receptors for geonol, DMTS, 1-octen-3-ol, butyric acid, 3-methyl-2-hexenoic acid, 3-hydroxy-3-methyl-hexanoic acid or transpirol.

Further provided are assays for identifying compounds that bind to geonol, DMTS, 1-octen-3-ol, butyric acid, 3-methyl-2-hexenoic acid, 3-hydroxy-3-methyl-hexanoic acid or transpirol odorant receptors. In a further embodiment provided herein is a malodor modulating compound that binds, suppresses, blocks, inhibits, and/or modulates the activity of at least one olfactory receptor selected from the group consisting of OR2W1, OR1A1, OR2J3, OR4Q3, OR5K1, OR11A1, OR2M3, OR51E1, OR4S2, OR51I2, OR2H1, OR2W3, OR8G1, Olfr263, Olfr403, Olfr735, Olfr96, Olfr1193, Olfr641, Olfr137, Olfr173, Olfr164, Olfr1487, Olfr339, Olfr1126, Olfr558, Olfr93, Olfr398, Olfr120, Olfr1364, Olfr937, Olfr1322 and Olfr46 and that is identified by the methods described herein, for example, the compounds described herein below.

In one embodiment the activity of the compound is determined by comparing its binding to that of geonol, DMTS, 1-octen-3-ol, butyric acid, 3-methyl-2-hexenoic acid, 3-hydroxy-3-methyl-hexanoic acid or transpirol. In another embodiment, the receptor or a chimera or fragment thereof is contacted with a compound in the presence of geonol, DMTS, 1-octen-3-ol, butyric acid, 3-methyl-2-hexenoic acid, 3-hydroxy-3-methyl-hexanoic acid or transpirol under conditions that allow for the binding of the compound along with geonol, DMTS, 1-octen-3-ol, butyric acid, 3-methyl-2-hexenoic acid, 3-hydroxy-3-methyl-hexanoic acid or transpirol to the receptor.

In a further embodiment, a compound is contacted to a receptor, or a chimera or fragment thereof that is activated by geonol, DMTS, 1-octen-3-ol, butyric acid, 3-methyl-2-hexenoic acid, 3-hydroxy-3-methyl-hexanoic acid or transpirol, wherein the receptor, or a chimera or fragment thereof is expressed in a cell that is recombinantly modified to express the receptor polypeptide.

The activity of the compound can be determined using *in vivo*, *ex vivo*, *in vitro* and synthetic screening systems.

In another embodiment, the contacting is performed with liposomes or virus-induced budding membranes containing the polypeptides described herein.

In another embodiment, the methods for identifying compounds that bind, suppress, block, inhibit, and/or modulate the activity of an olfactory receptor that can be activated by geonol, DMTS, 1-octen-3-ol, butyric acid, 3-methyl-2-hexenoic acid, 3-hydroxy-3-methyl-hexanoic acid or transpirol, may be performed on intact cells or a membrane fraction from cells expressing the polypeptides described here.

The ORs described herein are therefore involved in the perception of malodor elicited by geonol, DMTS, 1-octen-3-ol, butyric acid, 3-methyl-2-hexenoic acid, 3-hydroxy-3-methyl-hexanoic acid or transpirol and constitute valuable candidate receptors for the identification of modulators, antagonists and/or blockers that would modulate, reduce, suppress, inhibit and/or block the perception of laundry malodor, moldy malodor and/or sweat malodor.

### Polypeptides applicable according to the invention

The present invention relates to "functional equivalents" or "analogs" or "functional mutations" of the polypeptides specifically described herein.

For example, "functional equivalents" refer to polypeptides which, in a test used for OR activity, display at least a 1 to 10 %, or at least 20 %, or at least 50 %, or at least 75 %, or at least 90 % higher or lower OR activity, as defined herein.

"Functional equivalents", according to the invention, also cover particular mutants, which, in at least one sequence position of the amino acid sequences stated herein, have an amino acid that is different from that concretely stated, but nevertheless possess one of the aforementioned biological activities. "Functional equivalents" thus comprise the mutants obtainable by one or more, like 1 to 20, 1 to 15 or 5 to 10 amino acid additions, substitutions, in particular conservative substitutions, deletions and/or inversions, where the stated changes can occur in any sequence position, provided they lead to a mutant with the profile of properties according to the invention. Functional equivalence is in particular also provided if the activity patterns coincide qualitatively between the mutant and the unchanged polypeptide, i.e. if, for example, interaction with the same agonist or antagonist, however at a different rate, (i.e. expressed by an EC₅₀ or IC₅₀ value or any other parameter suitable in the present technical field) is observed. Examples of suitable (conservative) amino acid substitutions are shown in the following table:

| **Original residue** | **Examples of substitution** |
|---|---|
| Ala | Ser |
| Arg | Lys |
| Asn | Gln; His |
| Asp | Glu |
| Cys | Ser |
| Gln | Asn |
| Glu | Asp |
| Gly | Pro |
| His | Asn; Gln |
| Ile | Leu; Val |
| Leu | Ile; Val |
| Lys | Arg; Gln; Glu |
| Met | Leu; Ile |
| Phe | Met; Leu; Tyr |
| Ser | Thr |
| Thr | Ser |
| Trp | Tyr |
| Tyr | Trp; Phe |
| Val | Ile; Leu |

"Functional equivalents" in the above sense are also "precursors" of the polypeptides described, as well as "functional derivatives" and "salts" of the polypeptides.

"Precursors" are in that case natural or synthetic precursors of the polypeptides with or without the desired biological activity.

The expression "salts" means salts of carboxyl groups as well as salts of acid addition of amino groups of the protein molecules according to the invention. Salts of carboxyl groups can be produced in a known way and comprise inorganic salts, for example sodium, calcium, ammonium, iron and zinc salts, and salts with organic bases, for example amines, such as triethanolamine, arginine, lysine, piperidine and the like. Salts of acid addition, for example salts with inorganic acids, such as hydrochloric acid or sulfuric acid and salts with organic acids, such as acetic acid and oxalic acid, are also covered by the invention.

"Functional derivatives" of polypeptides according to the invention can also be produced on functional amino acid side groups or at their N-terminal or C-terminal end using known techniques. Such derivatives comprise for example aliphatic esters of carboxylic acid groups, amides of carboxylic acid groups, obtainable by reaction with ammonia or with a primary or secondary amine; N-acyl derivatives of free amino groups, produced by reaction with acyl groups; or O-acyl derivatives of free hydroxyl groups, produced by reaction with acyl groups.

"Functional equivalents" naturally also comprise polypeptides that can be obtained from other organisms, as well as naturally occurring variants. For example, areas of homologous sequence regions can be established by sequence comparison, and equivalent polypeptides can be determined on the basis of the concrete parameters of the invention.

"Functional equivalents" also comprise fragments, preferably individual domains or sequence motifs, of the polypeptides according to the invention, which for example display the desired biological function.

"Functional equivalents" are, moreover, fusion proteins, which have one of the polypeptide sequences stated herein or functional equivalents derived there from and at least one further, functionally different, heterologous sequence in functional N-terminal or C-terminal association (i.e. without substantial mutual functional impairment of the fusion protein parts). Non-limiting examples of these heterologous sequences are e.g. signal peptides, histidine anchors or enzymes.

"Functional equivalents" which are also comprised in accordance with the invention are homologs to the specifically disclosed polypeptides. These have at least 60%, preferably at least 75%, in particular at least 80 or 85%, such as, for example, 90, 91, 92, 93, 94, 95, 96, 97, 98 or 99%, homology (or identity) to one of the specifically disclosed amino acid sequences, calculated by the algorithm of Pearson and Lipman, Proc. Natl. Acad, Sci. (USA) 85(8), 1988, 2444-2448. A homology or identity, expressed as a percentage, of a homologous polypeptide according to the invention means in particular an identity, expressed as a percentage, of the amino acid residues based on the total length of one of the amino acid sequences described specifically herein.

The identity data, expressed as a percentage, may also be determined with the aid of BLAST alignments, algorithm blastp (protein-protein BLAST), or by applying the Clustal settings specified herein below.

In the case of a possible protein glycosylation, "functional equivalents" according to the invention comprise polypeptides as described herein in deglycosylated or glycosylated form as well as modified forms that can be obtained by altering the glycosylation pattern.

Such functional equivalents or homologues of the polypeptides according to the invention can be produced by mutagenesis, e.g. by point mutation, lengthening or shortening of the protein or as described in more detail below.

Such functional equivalents or homologues of the polypeptides according to the invention can be identified by screening combinatorial databases of mutants, for example shortening mutants. For example, a variegated database of protein variants can be produced by combinatorial mutagenesis at the nucleic acid level, e.g. by enzymatic ligation of a mixture of synthetic oligonucleotides. There are a great many methods that can be used for the production of databases of potential homologues from a degenerated oligonucleotide sequence. Chemical synthesis of a degenerated gene sequence can be carried out in an automatic DNA synthesizer, and the synthetic gene can then be ligated in a suitable expression vector. The use of a degenerated genome makes it possible to supply all sequences in a mixture, which code for the desired set of potential protein sequences. Methods of synthesis of degenerated oligonucleotides are known to a person skilled in the art (e.g. Narang, S.A. (1983); Itakura et al. (1984) (a); Itakura et al., (1984) (b); Ike et al. (1983)).

In the prior art, several techniques are known for the screening of gene products of combinatorial databases, which were produced by point mutations or shortening, and for the screening of cDNA libraries for gene products with a selected property. These techniques can be adapted for the rapid screening of the gene banks that were produced by combinatorial mutagenesis of homologues according to the invention. The techniques most frequently used for the screening of large gene banks, which are based on a high-throughput analysis, comprise cloning of the gene bank in expression vectors that can be replicated, transformation of the suitable cells with the resultant vector database and expression of the combinatorial genes in conditions in which detection of the desired activity facilitates isolation of the vector that codes for the gene whose product was detected. Recursive Ensemble Mutagenesis (REM), a technique that increases the frequency of functional mutants in the databases, can be used in combination with the screening tests, in order to identify homologues (Arkin and Yourvan (1992); Delgrave et al. (1993)).

### Coding nucleic acid sequences applicable according to the invention

The invention also relates to nucleic acid sequences that code for polypeptides as defined herein.

The present invention also relates to nucleic acids with a certain degree of "identity" to the sequences specifically disclosed herein. "Identity" between two nucleic acids means identity of the nucleotides, in each case over the entire length of the nucleic acid.

For example the identity may be calculated by means of the Vector NTI Suite 7.1 program of the company Informax (USA) employing the Clustal Method (Higgins DG, Sharp PM. ((1989))) with the following settings:

| Multiple alignment parameter: | |
|---|---|
| Gap opening penalty | 10 |
| Gap extension penalty | 10 |
| Gap separation penalty range | 8 |
| Gap separation penalty | off |
| % identity for alignment delay | 40 |
| Residue specific gaps | off |
| Hydrophilic residue gap | off |
| Transition weighing | 0 |

| Pairwise alignment parameter: | |
|---|---|
| FAST algorithm | on |
| K-tuple size | 1 |
| Gap penalty | 3 |
| Window size | 5 |
| Number of best diagonals | 5 |

Alternatively the identity may be determined according to Chenna, et al. (2003), the web page: http://www.ebi.ac.uk/Tools/clustalw/index.html# and the following settings:

| | |
|---|---|
| DNA Gap Open Penalty | 15.0 |
| DNA Gap Extension Penalty | 6.66 |
| DNA Matrix | Identity |
| Protein Gap Open Penalty | 10.0 |
| Protein Gap Extension Penalty | 0.2 |
| Protein matrix | Gonnet |
| Protein/DNA ENDGAP | -1 |
| Protein/DNA GAPDIST | 4 |

All the nucleic acid sequences mentioned herein (single-stranded and double-stranded DNA and RNA sequences, for example cDNA and mRNA) can be produced in a known way by chemical synthesis from the nucleotide building blocks, e.g. by fragment condensation of individual overlapping, complementary nucleic acid building blocks of the double helix. Chemical synthesis of oligonucleotides can, for example, be performed in a known way, by the phosphoamidite method (Voet, Voet, 2nd edition, Wiley Press, New York, pages 896-897). The accumulation of synthetic oligonucleotides and filling of gaps by means of the Klenow fragment of DNA polymerase and ligation reactions as well as general cloning techniques are described in Sambrook et al. (1989), see below.

The invention also relates to nucleic acid sequences (single-stranded and double-stranded DNA and RNA sequences, e.g. cDNA and mRNA), coding for one of the above polypeptides and their functional equivalents, which can be obtained for example using artificial nucleotide analogs.

The invention relates both to isolated nucleic acid molecules, which code for polypeptides according to the invention or biologically active segments thereof, and to nucleic acid fragments, which can be used for example as hybridization probes or primers for identifying or amplifying coding nucleic acids according to the invention.

The nucleic acid molecules according to the invention can in addition contain non-translated sequences from the 3' and/or 5' end of the coding genetic region.

The invention further relates to the nucleic acid molecules that are complementary to the concretely described nucleotide sequences or a segment thereof.

The nucleotide sequences according to the invention make possible the production of probes and primers that can be used for the identification and/or cloning of homologous sequences in other cellular types and organisms. Such probes or primers generally comprise a nucleotide sequence region which hybridizes under "stringent" conditions (see below) on at least about 12, preferably at least about 25, for example about 40, 50 or 75 successive nucleotides of a sense strand of a nucleic acid sequence according to the invention or of a corresponding antisense strand.

An "isolated" nucleic acid molecule is separated from other nucleic acid molecules that are present in the natural source of the nucleic acid and can moreover be substantially free from other cellular material or culture medium, if it is being produced by recombinant techniques, or can be free from chemical precursors or other chemicals, if it is being synthesized chemically.

A nucleic acid molecule according to the invention can be isolated by means of standard techniques of molecular biology and the sequence information supplied according to the invention. For example, cDNA can be isolated from a suitable cDNA library, using one of the concretely disclosed complete sequences or a segment thereof as hybridization probe and standard hybridization techniques (as described for example in Sambrook, (1989)). In addition, a nucleic acid molecule comprising one of the disclosed sequences or a segment thereof, can be isolated by the polymerase chain reaction, using the oligonucleotide primers that were constructed on the basis of this sequence. The nucleic acid amplified in this way can be cloned in a suitable vector and can be characterized by DNA sequencing. The oligonucleotides according to the invention can also be produced by standard methods of synthesis, e.g. using an automatic DNA synthesizer.

Nucleic acid sequences according to the invention or derivatives thereof, homologues or parts of these sequences, can for example be isolated by usual hybridization techniques or the PCR technique from other bacteria, e.g. via genomic or cDNA libraries. These DNA sequences hybridize in standard conditions with the sequences according to the invention.

"Hybridize" means the ability of a polynucleotide or oligonucleotide to bind to an almost complementary sequence in standard conditions, whereas nonspecific binding does not occur between non-complementary partners in these conditions. For this, the sequences can be 90-100 % complementary. The property of complementary sequences of being able to bind specifically to one another is utilized for example in Northern Blotting or Southern Blotting or in primer binding in PCR or RT-PCR.

Short oligonucleotides of the conserved regions are used advantageously for hybridization. However, it is also possible to use longer fragments of the nucleic acids according to the invention or the complete sequences for the hybridization. These standard conditions vary depending on the nucleic acid used (oligonucleotide, longer fragment or complete sequence) or depending on which type of nucleic acid - DNA or RNA - is used for hybridization. For example, the melting temperatures for DNA:DNA hybrids are approx. 10 °C lower than those of DNA:RNA hybrids of the same length.

For example, depending on the particular nucleic acid, standard conditions mean temperatures between 42 and 58 °C in an aqueous buffer solution with a concentration between 0.1 to 5 x SSC (1 X SSC = 0.15 M NaCl, 15 mM sodium citrate, pH 7.2) or additionally in the presence of 50 % formamide, for example 42 °C in 5 x SSC, 50 % formamide. Advantageously, the hybridization conditions for DNA:DNA hybrids are 0.1 x SSC and temperatures between about 20 °C to 45 °C, preferably between about 30 °C to 45 °C. For DNA:RNA hybrids the hybridization conditions are advantageously 0.1 x SSC and temperatures between about 30 °C to 55 °C, preferably between about 45 °C to 55 °C. These stated temperatures for hybridization are examples of calculated melting temperature values for a nucleic acid with a length of approx. 100 nucleotides and a G + C content of 50 % in the absence of formamide. The experimental conditions for DNA hybridization are described in relevant genetics textbooks, for example Sambrook et al., 1989, and can be calculated using formulae that are known by a person skilled in the art, for example depending on the length of the nucleic acids, the type of hybrids or the G + C content. A person skilled in the art can obtain further information on hybridization from the following textbooks: Ausubel et al. (eds), (1985), Brown (ed) (1991).

"Hybridization" can in particular be carried out under stringent conditions. Such hybridization conditions are for example described in Sambrook (1989), or in Current Protocols in Molecular Biology, John Wiley & Sons, N.Y. (1989), 6.3.1-6.3.6.

As used herein, the term hybridization or hybridizes under certain conditions is intended to describe conditions for hybridization and washes under which nucleotide sequences that are significantly identical or homologous to each other remain bound to each other. The conditions may be such that sequences, which are at least about 70%, such as at least about 80%, and such as at least about 85%, 90%, or 95% identical, remain bound to each other. Definitions of low stringency, moderate, and high stringency hybridization conditions are provided herein.

Appropriate hybridization conditions can be selected by those skilled in the art with minimal experimentation as exemplified in Ausubel et al. (1995, Current Protocols in Molecular Biology, John Wiley & Sons, sections 2, 4, and 6). Additionally, stringency conditions are described in Sambrook et al. (1989, Molecular Cloning: A Laboratory Manual, 2nd ed., Cold Spring Harbor Press, chapters 7, 9, and 11).

As used herein, defined conditions of low stringency are as follows. Filters containing DNA are pretreated for 6 h at 40°C in a solution containing 35% formamide, 5x SSC, 50 mM Tris-HCl (pH 7.5), 5 mM EDTA, 0.1% PVP, 0.1% Ficoll, 1% BSA, and 500 µg/ml denatured salmon sperm DNA. Hybridizations are carried out in the same solution with the following modifications: 0.02% PVP, 0.02% Ficoll, 0.2% BSA, 100 µg/ml salmon sperm DNA, 10% (wt/vol) dextran sulfate, and 5-20x106 32P-labeled probe is used. Filters are incubated in hybridization mixture for 18-20 h at 40°C, and then washed for 1.5 h at 55°C. In a solution containing 2x SSC, 25 mM Tris-HCl (pH 7.4), 5 mM EDTA, and 0.1% SDS. The wash solution is replaced with fresh solution and incubated an additional 1.5 h at 60°C. Filters are blotted dry and exposed for autoradiography.

As used herein, defined conditions of moderate stringency are as follows. Filters containing DNA are pretreated for 7 h at 50°C. in a solution containing 35% formamide, 5x SSC, 50 mM Tris-HCl (pH 7.5), 5 mM EDTA, 0.1% PVP, 0.1% Ficoll, 1% BSA, and 500 µg/ml denatured salmon sperm DNA. Hybridizations are carried out in the same solution with the following modifications: 0.02% PVP, 0.02% Ficoll, 0.2% BSA, 100 µg/ml salmon sperm DNA, 10% (wt/vol) dextran sulfate, and 5-20x106 32P-labeled probe is used. Filters are incubated in hybridization mixture for 30 h at 50°C, and then washed for 1.5 h at 55°C. In a solution containing 2x SSC, 25 mM Tris-HCl (pH 7.4), 5 mM EDTA, and 0.1% SDS. The wash solution is replaced with fresh solution and incubated an additional 1.5 h at 60°C. Filters are blotted dry and exposed for autoradiography.

As used herein, defined conditions of high stringency are as follows. Prehybridization of filters containing DNA is carried out for 8 h to overnight at 65°C in buffer composed of 6x SSC, 50 mM Tris-HCl (pH 7.5), 1 mM EDTA, 0.02% PVP, 0.02% Ficoll, 0.02% BSA, and 500 µg/ml denatured salmon sperm DNA. Filters are hybridized for 48 h at 65°C in the prehybridization mixture containing 100 µg /ml denatured salmon sperm DNA and 5-20x106 cpm of 32P-labeled probe. Washing of filters is done at 37°C for 1 h in a solution containing 2x SSC, 0.01% PVP, 0.01% Ficoll, and 0.01% BSA. This is followed by a wash in 0.1x SSC at 50°C for 45 minutes.
Other conditions of low, moderate, and high stringency well known in the art (e.g., as employed for cross-species hybridizations) may be used if the above conditions are inappropriate (e.g., as employed for cross-species hybridizations).

The invention also relates to derivatives of the concretely disclosed or derivable nucleic acid sequences.

Thus, further nucleic acid sequences according to the invention can be derived from the sequences specifically disclosed herein and can differ from it by addition, substitution, insertion or deletion of individual or several nucleotides, and furthermore code for polypeptides with the desired profile of properties.

The invention also encompasses nucleic acid sequences that comprise so-called silent mutations or have been altered, in comparison with a concretely stated sequence, according to the codon usage of a special original or host organism, as well as naturally occurring variants, e.g. splicing variants or allelic variants, thereof.

It also relates to sequences that can be obtained by conservative nucleotide substitutions (i.e. the amino acid in question is replaced by an amino acid of the same charge, size, polarity and/or solubility).

The invention also relates to the molecules derived from the concretely disclosed nucleic acids by sequence polymorphisms. These genetic polymorphisms can exist between individuals within a population owing to natural variation. These natural variations usually produce a variance of 1 to 5 % in the nucleotide sequence of a gene.

Derivatives of nucleic acid sequences according to the invention mean for example allelic variants, having at least 60 % homology at the level of the derived amino acid, preferably at least 80 % homology, quite especially preferably at least 90 % homology over the entire sequence range (regarding homology at the amino acid level, reference should be made to the details given above for the polypeptides). Advantageously, the homologies can be higher over partial regions of the sequences.

Furthermore, derivatives are also to be understood to be homologues of the nucleic acid sequences according to the invention, for example animal, plant, fungal or bacterial homologues, shortened sequences, single-stranded DNA or RNA of the coding and noncoding DNA sequence. For example, homologues have, at the DNA level, a homology of at least 40 %, preferably of at least 60 %, especially preferably of at least 70 %, quite especially preferably of at least 80 % over the entire DNA region given in a sequence specifically disclosed herein.

Moreover, derivatives are to be understood to be, for example, fusions with promoters. The promoters that are added to the stated nucleotide sequences can be modified by at least one nucleotide exchange, at least one insertion, inversion and/or deletion, though without impairing the functionality or efficacy of the promoters. Moreover, the efficacy of the promoters can be increased by altering their sequence or can be exchanged completely with more effective promoters even of organisms of a different genus.

### Generation of functional polypeptide mutants

Moreover, a person skilled in the art is familiar with methods for generating functional mutants, that is to say nucleotide sequences which code for a polypeptide with at least 80%, 81%, 82%, 83%, 84%, 85%, 86%, 87%, 88%, 89%, 90%, 91%, 92%, 93%, 94%, 95%, 96%, 97%, 98% or 99% sequence identity to anyone of SEQ ID NO. 2, 4, 6, 8, 10, 12, 14, 16, 18, 20, 22, 24, 26, 28, 30, 32, 34, 36, 38, 40, 42, 44, 46, 57, 59, 61, 63, 65, 67, 69, 71, 73, or 75; and/or encoded by a nucleic acid molecule comprising a nucleotide sequence having at least 70% sequence identity to SEQ ID NO: 1, 3, 5, 7, 9, 11, 13, 15, 17, 19, 21, 23, 25, 27, 29, 31, 33, 35, 37, 39, 41, 43, 45, 56, 58, 60, 62, 64, 66, 68, 70, 72, 74, or the reverse complement thereof.

Depending on the technique used, a person skilled in the art can introduce entirely random or else more directed mutations into genes or else noncoding nucleic acid regions (which are for example important for regulating expression) and subsequently generate genetic libraries. The methods of molecular biology required for this purpose are known to the skilled worker and for example described in Sambrook and Russell, Molecular Cloning. 3rd Edition, Cold Spring Harbor Laboratory Press 2001.

Methods for modifying genes and thus for modifying the polypeptide encoded by them have been known to the skilled worker for a long time, such as, for example
- site-specific mutagenesis, where individual or several nucleotides of a gene are replaced in a directed fashion (Trower MK (Ed.) 1996; In vitro mutagenesis protocols. Humana Press, New Jersey),
- saturation mutagenesis, in which a codon for any amino acid can be exchanged or added at any point of a gene (Kegler-Ebo DM, Docktor CM, DiMaio D (1994) Nucleic Acids Res 22:1593; Barettino D, Feigenbutz M, Valcárel R, Stunnenberg HG (1994) Nucleic Acids Res 22:541; Barik S (1995) Mol Biotechnol 3:1),
- error-prone polymerase chain reaction, where nucleotide sequences are mutated by error-prone DNA polymerases (Eckert KA, Kunkel TA (1990) Nucleic Acids Res 18:3739);
- the SeSaM method (sequence saturation method), in which preferred exchanges are prevented by the polymerase. Schenk et al., Biospektrum, Vol. 3, 2006, 277-279
- the passaging of genes in mutator strains, in which, for example owing to defective DNA repair mechanisms, there is an increased mutation rate of nucleotide sequences (Greener A, Callahan M, Jerpseth B (1996) An efficient random mutagenesis technique using an E.coli mutator strain. In: Trower MK (Ed.) In vitro mutagenesis protocols. Humana Press, New Jersey), or
- DNA shuffling, in which a pool of closely related genes is formed and digested and the fragments are used as templates for a polymerase chain reaction in which, by repeated strand separation and reassociation, full-length mosaic genes are ultimately generated (Stemmer WPC (1994) Nature 370:389; Stemmer WPC (1994) Proc Natl Acad Sci USA 91:10747).

Using so-called directed evolution (described, inter alia, in Reetz MT and Jaeger K-E (1999), Topics Curr Chem 200:31; Zhao H, Moore JC, Volkov AA, Arnold FH (1999), Methods for optimizing industrial polypeptides by directed evolution, In: Demain AL, Davies JE (Ed.) Manual of industrial microbiology and biotechnology. American Society for Microbiology), a skilled worker can produce functional mutants in a directed manner and on a large scale. To this end, in a first step, gene libraries of the respective polypeptides are first produced, for example using the methods given above. The gene libraries are expressed in a suitable way, for example by bacteria or by phage display systems.

The relevant genes of host organisms which express functional mutants with properties that largely correspond to the desired properties can be submitted to another mutation cycle. The steps of the mutation and selection or screening can be repeated iteratively until the present functional mutants have the desired properties to a sufficient extent. Using this iterative procedure, a limited number of mutations, for example 1, 2, 3, 4 or 5 mutations, can be performed in stages and assessed and selected for their influence on the activity in question. The selected mutant can then be submitted to a further mutation step in the same way. In this way, the number of individual mutants to be investigated can be reduced significantly.

The results according to the invention also provide important information relating to structure and sequence of the relevant polypeptides, which is required for generating, in a targeted fashion, further polypeptides with desired modified properties. In particular, it is possible to define so-called "hot spots", i.e. sequence segments that are potentially suitable for modifying a property by introducing targeted mutations.

Information can also be deduced regarding amino acid sequence positions, in the region of which mutations can be effected that should probably have little effect on the activity, and can be designated as potential "silent mutations".

### Constructs for preparing polypeptides of the invention

The nucleotide sequence as described above may be part of an expression cassette. The terms expression cassette and expression construct are used synonymously. The preferably recombinant expression construct contains a nucleotide sequence which encodes a polypeptide according to the invention and which is under genetic control of regulatory nucleic acid sequences.

In a process applied according to the invention, the expression cassette may be part of an expression vector, in particular of a recombinant expression vector.

An "expression unit" is understood as meaning, in accordance with the invention, a nucleic acid with expression activity which comprises a promoter as defined herein and, after functional linkage with a nucleic acid to be expressed or a gene, regulates the expression, i.e. the transcription and the translation of said nucleic acid or said gene. It is therefore in this connection also referred to as a "regulatory nucleic acid sequence". In addition to the promoter, other regulatory elements, for example enhancers, can also be present.

An "expression cassette" or "expression construct" is understood as meaning, in accordance with the invention, an expression unit which is functionally linked to the nucleic acid to be expressed or the gene to be expressed. In contrast to an expression unit, an expression cassette therefore comprises not only nucleic acid sequences which regulate transcription and translation, but also the nucleic acid sequences that are to be expressed as protein as a result of transcription and translation.

The terms "expression" or "overexpression" describe, in the context of the invention, the production or increase in intracellular activity of one or more polypeptides in a microorganism, which are encoded by the corresponding DNA. To this end, it is possible for example to introduce a gene into an organism, replace an existing gene with another gene, increase the copy number of the gene(s), use a strong promoter or use a gene which encodes for a corresponding polypeptide with a high activity; optionally, these measures can be combined.

Preferably such constructs according to the invention comprise a promoter 5'-upstream of the respective coding sequence and a terminator sequence 3'-downstream and optionally other usual regulatory elements, in each case in operative linkage with the coding sequence.

A "promoter", a "nucleic acid with promoter activity" or a "promoter sequence" is understood as meaning, in accordance with the invention, a nucleic acid which, when functionally linked to a nucleic acid to be transcribed, regulates the transcription of said nucleic acid.

In this context, a "functional" or "operative" linkage is understood as meaning for example the sequential arrangement of one of the nucleic acids with promoter activity and of a nucleic acid sequence to be transcribed and optionally further regulatory elements, for example nucleic acid sequences which ensure the transcription of nucleic acids, and for example a terminator, in such a way that each of the regulatory elements can perform its function upon transcription of the nucleic acid sequence. This does not necessarily require a direct linkage in the chemical sense. Genetic control sequences, for example enhancer sequences, can even exert their function on the target sequence from more remote positions or even from other DNA molecules. Preferred arrangements are those in which the nucleic acid sequence to be transcribed is positioned behind (i.e. at the 3'-end of) the promoter sequence so that the two sequences are joined together covalently. The distance between the promoter sequence and the nucleic acid sequence to be expressed recombinantly can be smaller than 200 base pairs, or smaller than 100 base pairs or smaller than 50 base pairs.

In addition to promoters and terminator, the following may be mentioned as examples of other regulatory elements: targeting sequences, enhancers, polyadenylation signals, selectable markers, amplification signals, replication origins and the like. Suitable regulatory sequences are described, for example, in Goeddel, Gene Expression Technology: Methods in Enzymology 185, Academic Press, San Diego, CA (1990).

Nucleic acid constructs according to the invention comprise in particular a sequence coding for a polypeptide for example derived from SEQ ID No. 1, 3, 5, 7, 9, 11, 13, 15, 17, 19, 21, 23, 25, 27, 29, 31, 33, 35, 37, 39, 41, 43, 45, 56, 58, 60, 62, 64, 66, 68, 70, 72, 74, or the reverse complement thereof, or derivatives and homologs thereof, and the nucleic acid sequences which can be derived therefrom and which have been linked operatively or functionally with one or more regulatory signals, advantageously for controlling, for example increasing, gene expression.

In addition to these regulatory sequences, the natural regulation of these sequences may still be present before the actual structural genes and optionally may have been genetically modified so that the natural regulation has been switched off and expression of the genes has been enhanced. The nucleic acid construct may, however, also be of simpler construction, i.e. no additional regulatory signals have been inserted before the coding sequence and the natural promoter, with its regulation, has not been removed. Instead, the natural regulatory sequence is mutated such that regulation no longer takes place and the gene expression is increased.

A preferred nucleic acid construct advantageously also comprises one or more of the already mentioned "enhancer" sequences in functional linkage with the promoter, which sequences make possible an enhanced expression of the nucleic acid sequence. Additional advantageous sequences may also be inserted at the 3'-end of the DNA sequences, such as further regulatory elements or terminators. One or more copies of the nucleic acids according to the invention may be present in a construct. In the construct, other markers, such as genes which complement auxotrophisms or antibiotic resistances, may also optionally be present so as to select for the construct.

Examples of suitable regulatory sequences are present in promoters such as cos, tac, trp, tet, trp-tet, lpp, lac, lpp-lac, lacI^{q}, T7, T5, T3, gal, trc, ara, rhaP (rhaP_{BAD})SP6, lambda-P_{R} or in the lambda-P_{L} promoter, and these are advantageously employed in Gram-negative bacteria. Further advantageous regulatory sequences are present for example in the Gram-positive promoters amy and SPO2, in the yeast or fungal promoters ADC1, MFalpha, AC, P-60, CYC1, GAPDH, TEF, rp28, ADH. Artificial promoters may also be used for regulation.

For expression in a host organism, the nucleic acid construct is inserted advantageously into a vector such as, for example, a plasmid or a phage, which makes possible optimal expression of the genes in the host. Vectors are also understood as meaning, in addition to plasmids and phages, all the other vectors which are known to the skilled worker, that is to say for example viruses such as SV40, CMV, baculovirus and adenovirus, transposons, IS elements, phasmids, cosmids and linear or circular DNA. These vectors are capable of replicating autonomously in the host organism or else chromosomally. These vectors are a further development of the invention.

Suitable plasmids are, for example, in E. coli pLG338, pACYC184, pBR322, pUC18, pUC19, pKC30, pRep4, pHS1, pKK223-3, pDHE19.2, pHS2, pPLc236, pMBL24, pLG200, pUR290, pIN-III¹¹³-B1, λgt11 or pBdCI, in Streptomyces pIJ101, pIJ364, pIJ702 or pIJ361, in Bacillus pUB110, pC194 or pBD214, in Corynebacterium pSA77 or pAJ667, in fungi pALS1, pIL2 or pBB116, in yeasts 2alphaM, pAG-1, YEp6, YEp13 or pEMBLYe23 or in plants pLGV23, pGHlac⁺, pBIN19, pAK2004 or pDH51. The abovementioned plasmids are a small selection of the plasmids which are possible. Further plasmids are well known to the skilled worker and can be found for example in the book Cloning Vectors (Eds. Pouwels P. H. et al. Elsevier, Amsterdam-New York-Oxford, 1985, ISBN 0 444 904018).

In a further development of the vector, the vector which comprises the nucleic acid construct according to the invention or the nucleic acid according to the invention can advantageously also be introduced into the microorganisms in the form of a linear DNA and integrated into the host organism's genome via heterologous or homologous recombination. This linear DNA can consist of a linearized vector such as a plasmid or only of the nucleic acid construct or the nucleic acid according to the invention.

For optimal expression of heterologous genes in organisms, it is advantageous to modify the nucleic acid sequences to match the specific "codon usage" used in the organism. The "codon usage" can be determined readily by computer evaluations of other, known genes of the organism in question.

An expression cassette according to the invention is generated by fusing a suitable promoter to a suitable coding nucleotide sequence and a terminator or polyadenylation signal. Customary recombination and cloning techniques are used for this purpose, as are described, for example, in T. Maniatis, E.F. Fritsch and J. Sambrook, Molecular Cloning: A Laboratory Manual, Cold Spring Harbor Laboratory, Cold Spring Harbor, NY (1989) and in T.J. Silhavy, M.L. Berman and L.W. Enquist, Experiments with Gene Fusions, Cold Spring Harbor Laboratory, Cold Spring Harbor, NY (1984) and in Ausubel, F.M. et al., Current Protocols in Molecular Biology, Greene Publishing Assoc. and Wiley Interscience (1987).

For expression in a suitable host organism, the recombinant nucleic acid construct or gene construct is advantageously inserted into a host-specific vector which makes possible optimal expression of the genes in the host. Vectors are well known to the skilled worker and can be found for example in "cloning vectors" (Pouwels P. H. et al., Ed., Elsevier, Amsterdam-New York-Oxford, 1985).
The content of the documents cross-referenced is incorporated by reference.

Nucleic acid and amino acid sequences identified and/or used herein are listed below:
OR2W1
   *Homo sapiens*_OR2W1_1-octen-3-ol, transpirol, 3-hydroxy-3-methylhexanoic acid, 3-methyl-2-hexenoic acid and DMTS olfactory receptor (GI: 26692)
DNA: SEQ ID NO: 1 
atggaccaaagcaattatagttctttacatggttttattctgcttggcttctctaaccatccaaaaatggagatgatcctgtcaggagtt
gtcgccatcttctacttaattacattggtgggtaacacagccatcattcttgcatctctcctggattcccagcttcatacaccaatgtact
ttttcctcagaaatttatctttcctagatctatgtttcacaaccagcatcatccctcaggtgctggtcaacttgtggggacctgataagac
catcagctatgtgggttgtatcatccaactctatgtttacatgtggttgggctcagttgagtgccttctcctggctgttatgtcctatgatc
gttttacagctatatgtaagcccttgcattattttgtagtcatgaacccacatctatgtctaaagatgattatcatgatctggagtattag
tttggccaattctgtagtattatgtacactcactctgaatttgcccacatgtggaaacaacattctggatcatttcttgtgtgagttgcca
gctctggtcaagatagcttgtgtagacaccacaacagttgaaatgtctgttttcgctttaggcattataattgtcctcacacctctcatcc
ttattcttatatcctatggctacattgccaaagctgtgctgagaacgaagtcaaaagcaagccagcgaaaagcaatgaatacctgtg
gatctcatcttactgtagtgtctatgttctatggaactattatctacatgtacctgcaaccaggtaacagggcttccaaagaccagggc
aagttcctcaccctcttttacactgtcatcactccaagtctcaacccgctcatttacaccttaagaaataaggacatgaaggatgccct
gaagaaactgatgagatttcaccacaaatctacaaaaataaagaggaattgcaagtcatag
PROT: SEQ ID NO: 2
**OR1A1**
   *Homo sapiens*_OR1A1_1-octen-3-ol, transpirol, DMTS, and geonol olfactory receptor (GI:8383)
DNA: SEQ ID NO: 3 
atgagggaaaataaccagtcctctacactggaattcatcctcctgggagttactggtcagcaggaacaggaagatttcttctacatcc
tcttcctgttcatttaccccatcacattgattggaaacctgctcatcgtcctagccatttgctctgatgttcgccttcacaaccccatgtat
tttctccttgccaacctctccttggttgacatcttcttctcatcggtaaccatccctaagatgctggccaaccatctcttgggcagcaaat
ccatctcttttgggggatgcctaacgcagatgtatttcatgatagccttgggtaacacagacagctatattttggctgcaatggcatatg
atcgagctgtggccatcagccgcccacttcactacacaacaattatgagtccacggtcttgtatctggcttattgctgggtcttgggtga
ttggaaatgccaatgccctcccccacactctgctcacagctagtctgtccttctgtggcaaccaggaagtggccaacttctactgtgac
attacccccttgctgaagttatcctgttctgacatccactttcatgtgaagatgatgtacctaggggttggcattttctctgtgccattact
atgcatcattgtctcctatattcgagtcttctccacagtcttccaggttccttccaccaagggcgtgctcaaggccttctccacctgtggtt
cccacctcacggttgtctctttgtattatggtacggtcatgggcacgtatttccgccctttgaccaattatagcctaaaagacgcagtga
tcactgtaatgtacacggcagtgaccccaatgttaaattctttcatctacagtctgagaaatcgggacatgaaggctgccctgcggaa
actcttcaacaagagaatctcctcgtaa
PROT: SEQ ID NO: 4 
MRENNQSSTLEFILLGVTGQQEQEDFFYILFLFIYPITLIGNLLIVLAICSDVRLHNPMYFLLANLSLVDIFFSSV
TIPKMLANHLLGSKSISFGGCLTQMYFMIALGNTDSYILAAMAYDRAVAISRPLHYTTIMSPRSCIWLIAGS
WVIGNANALPHTLLTASLSFCGNQEVANFYCDITPLLKLSCSDIHFHVKMMYLGVGIFSVPLLCIIVSYIRVFS
TVFQVPSTKGVLKAFSTCGSHLTVVSLYYGTVMGTYFRPLTNYSLKDAVITVMYTAVTPMLNSFIYSLRNR
DMKAALRKLFNKRISS
**OR2J3**
   *Homo sapiens*_OR2J3_1-octen-3-ol, and transpirol olfactory receptor (GI: 442186)
DNA: SEQ ID NO: 5 
atgaatgatgatggaaaagtcaatgctagctctgaggggtactttattttagttggattttctaattggcctcatctggaagtagttatct
ttgtggttgtcttgatcttctacttgatgacactgataggaaacctgttcatcatcatcctgtcatacctggactcccatctgcacacacc
aatgtacttcttcctttcaaacctctcatttctggatctctgctacaccaccagctctatccctcagttgctggtcaatctctggggcccgg
aaaagaccatctcttatgctggttgcatgattcaactttactttgttctcgcactgggaaccgcagagtgtgtcctactggtggtgatgt
cctatgaccgttatgcagctgtgtgtagacctttgcattacactgtcctcatgcaccctcgtttctgccacctgctggctgtggcttcttgg
gtaagtggttttaccaactcagcacttcattcctccttcaccttctgggtacctctgtgtggacaccgccaagtagatcacttttctgtg
aagttccagcacttctgcgattatcgtgtgttgatacccatgtcaatgagctgaccctcatgatcacaagctccatatttgttctcatacc
tctcatcctcattctcacttcttatggtgccatcgtccgagctgtactgaggatgcagtcaaccactgggcttcagaaagtgtttggaac
atgtggagctcatcttatggctgtatctctcttttcattccggccatgtgcatatatctccagccaccatcaggaaattctcaagatcaa
ggcaagttcattgccctcttttatactgttgtcacacctagtcttaaccctctaatctacaccctcagaaacaaagttgtaagaggggca
gtgaagagactaatggggtgggaatga
PROT: SEQ ID NO: 6
**OR4Q3**
   *Homo sapiens*_OR4Q3_1-octen-3-ol olfactory receptor (GI:441669)
DNA: SEQ ID NO: 7 
atgaaaaaagaacaagattctaatgtgacagaatttgttcttctgggcctatcatcttcttgggagctgcagctatttctcttcttactat
ttttgtttttttacattgctattgtcctgggaaacctcttgatagtggtaacagtgcaagcccatgctcacctgctccaatctcctatgtat
tattttttaggtcatctctctttcattgacctatgcctgagctgtgttactgtgccaaagatgttaggggatttcctacagcagggcaaga
gcatctcttttcaggatgcctggcccagatctacttcctccactttctaggagccagtgagatgtttttgctgacagtcatggcctatga
caggtatgttgccatctgtaaccctttgcgctaccttacagtcatgaacccccagctatgcctttggttggttcttgcctgctggtgtggg
ggttttatccactctatcatgcaggtcatactagtcatccagctgcctttctgtgggcccaatgaactggacaacttctactgtgatgtcc
cacaagtcatcaagctggcctgcatggacacctatgtggtagaggtgctggtgatagccaacagtggtctgctgtctcttgtctgcttct
tggtcttactattctcttatgctatcatcctgatcaccctgagaacacacttctgccagggccagaacaaggtcctctctacctgtgcttc
tcacctgacagtggtcagcctgatcttcgtgccatgcgtattcatctatttgaggcctttctgcagcttctctgtggataagatattctcct
tgttttacacagtgattacacctatgttgaacccccctcatctacacactcagaaatactgatatgaagacagctatgaagaagctgag
gataaaaccatgtggcattccattgccttgttaa
PROT: SEQ ID NO: 8 
MKKEQDSNVTEFVLLGLSSSWELQLFLFLLFLFFYIAIVLGNLLIVVTVQAHAHLLQSPMYYFLGHLSFIDLCL
SCVTVPKMLGDFLQQGKSISFSGCLAQIYFLHFLGASEMFLLTVMAYDRYVAICNPLRYLTVMNPQLCWL
VLACWCGGFIHSIMQ
VILVIQLPFCGPNELDNFYCDVPQVIKLACMDTYVVEVLVIANSGLLSLVCFLVLLFSYAIILITLRTHFCQGQ
NKVLSTCASHLTVVSLIFVPCVFIYLRPFCSFSVDKIFSLFYTVITPMLNPLIYTLRNTDMKTAMKKLRIKPCGI
PLPC
**OR5K1**
   *Homo sapiens*_OR5K1_1-octen-3-ol, transpirol, and DMTS olfactory receptor (GI:26339)
DNA: SEQ ID NO: 9 
atggctgaagaaaatcataccatgaaaaatgagtttatcctcacaggatttacagatcaccctgagctgaagactctgctgtttgtggt
gttctttgccatctatctgatcaccgtggtggggaatattagtttggtggcactgatatttacacaccgtcggcttcacacaccaatgta
catctttctgggaaatctggctcttgtggattcttgctgtgcctgtgctattaccccaaaatgttagagaacttcttttctgagaacaaa
aggatttccctctatgaatgtgcagtacagttttatttctttgcactgtggaaactgcagactgctttcttctggcagcaatggcctatg
accgctatgtggccatatgcaacccactgcagtaccacatcatgatgtccaagaaactctgcattcagatgaccacaggggccttcat
agctggaaacctgcattccatgattcatgtagggcttgtatttaggttagttttctgtggatcgaatcacatcaaccacttttactgtgat
attcttcccttgtatagactctcttgtgttgatccttatatcaatgaactggttctattcatcttctcaggttcagttcaagtctttaccata
ggtagtgtcttaatatcttatctctatattcttcttactattttcaaaatgaaatccaaagagggaagggccaaagctttttctacctgtg
catcccactttttgtcagtttcattattctatggatctcttttcttcatgtacgttagaccaaatttgcttgaagaaggggataaagatata
ccagctgcaattttatttacaatagtagttcccttactaaatcctttcatttatagcctgagaaatagggaagtaataagtgtcttaaga
aaaattctgatgaagaaataa
PROT: SEQ ID NO: 10 
MAEENHTMKNEFILTGFTDHPELKTLLFVVFFAIYLITVVGNISLVALIFTHRRLHTPMYIFLGNLALVDSCCA
CAITPKMLENFFSENKRISLYECAVQFYFLCTVETADCFLLAAMAYDRYVAICNPLQYHIMMSKKLCIQMT
TGAFIAGNLHSMIHV
GLVFRLVFCGSNHINHFYCDILPLYRLSCVDPYINELVLFIFSGSVQVFTIGSVLISYLYILLTIFKMKSKEGRAK
AFSTCASHFLSVSLFYGSLFFMYVRPNLLEEGDKDIPAAILFTIVVPLLNPFIYSLRNREVISVLRKILMKK
**OR11A1**
   *Homo sapiens_OR11A1_geonol* olfactory receptor (GI:26531)
DNA: SEQ ID NO: 11 
atggaaattgtctccacaggaaacgaaactattactgaatttgtcctccttggcttctatgacatccctgaactgcatttcttgtttttat
tgtattcactgctgtctatgtcttcatcatcatagggaatatgctgattattgtagcagtggttagctcccagaggctccacaaacccat
gtatattttcttggcgaatctgtccttcctggatattctctacacctccgcagtgatgccaaaaatgctggagggcttcctgcaagaagc
aactatctctgtggctggttgcttgctccagttctttatcttcggctctctagccacagctgaatgcttactgctggctgtcatggcatatg
accgctacctggcaatttgctacccactccactacccactcctgatggggcccagacggtacatggggctggtggtcacaacctggct
ctctggatttgtggtagatggactggttgtggccctggtggcccagctgaggttctgtggccccaaccacattgaccagttttactgtga
ctttatgcttttcgtgggcctggcttgctcggatcccagagtggctcaggtgacaactctcattctgtctgtgttctgcctcactattccttt
tggactgattctgacatcttatgccagaattgtggtggcagtgctgagagttcctgctggggcaagcaggagaagggctttctccaca
tgctcctcccacctagctgtagtgaccacattctatggaacgctcatgatcttttatgttgcaccctctgctgtccattcccagctcctctc
caaggtcttctccctgctctacactgtggtcacccctctcttcaatcctgtgatctataccatgaggaacaaggaggtgcatcaggcac
ttcggaagattctctgtatcaaacaaactgaaacacttgattga
PROT: SEQ ID NO: 12 
MEIVSTGNETITEFVLLGFYDIPELHFLFFIVFTAVYVFIIIGNMLIIVAVVSSQRLHKPMYIFLANLSFLDILYTS
AVMPKMLEGFLQEATISVAGCLLQFFIFGSLATAECLLLAVMAYDRYLAICYPLHYPLLMGPRRYMGLVVT
TWLSGFVVDGLVVALVAQLRFCGPNHIDQFYCDFMLFVGLACSDPRVAQVTTLILSVFCLTIPFGLILTSYA
RIVVAVLRVPAGASRRRAFSTCSSHLAVVTTFYGTLMIFYVAPSAVHSQLLSKVFSLLYTVVTPLFNPVIYTM
RNKEVHQALRKILCIKQTETLD
**OR2M3**
   *Homo sapiens_OR2M3_geonol* olfactory receptor (GI:127062)
DNA: SEQ ID NO: 13 
atggcaagggagaattcgaccttcaactccgacttcatcctcctgggaatcttcaatcacagccccacccacaccttcctcttctttctg
gtcctggccatcttttcagtggccttcatgggaaactctgtcatggttctcctcatctacctggacacccagctccacacccccatgtac
ctcctcctcagccaactgtccctcatggacctcatgctcatctgcaccaccgtacccaagatggccttcaactacctgtctggcagcaa
gtccatttctatggctggttgtgccacacaaattttcttctatacatcactgcttggctctgaatgctttcttttggctgttatggcttatga
ccgctacactgccatttgccaccctctaagatacaccaatctcatgagccctaaaatttgtggacttatgactgccttttcctggatcct
gggctctacggatggaattattgatgttgtagcaacattttccttctcctactgtgggtctcgggaaatagcccacttcttctgtgacttc
ccctccctactaatcctctcatgcagtgacacatcaatatttgaaaagattcttttcatctgctgtatagtaatgattgttttccctgttgc
aatcatcattgcttcctatgctcgagttatcctggctgtcattcacatgggatctggagagggtcgtcgcaaagcttttactacttgttcc
tctcacctcttggtggtgggaatgtactatggagcagctttgttcatgtacatacggcccacatctgatcgctccccaacacaggacaa
gatggtgtctgtattctacaccatcctcactcccatgttgaatcccctcatctacagcctccgcaacaaggaggtgaccagagcattca
tgaagatcttaggaaagggcaagtctggagagtga
PROT: SEQ ID NO: 14 
MARENSTFNSDFILLGIFNHSPTHTFLFFLVLAIFSVAFMGNSVMVLLIYLDTQLHTPMYLLLSQLSLMDLM
LICTTVPKMAFNYLSGSKSISMAGCATQIFFYTSLLGSECFLLAVMAYDRYTAICHPLRYTNLMSPKICGLMT
AFSWILGSTDGIIDVVATFSFSYCGSREIAHFFCDFPSLLILSCSDTSIFEKILFICCIVMIVFPVAIIIASYARVILA
VIHMGSGEGRRKAFTTCSSHLLVVGMYYGAALFMYIRPTSDRSPTQDKMVSVFYTILTPMLNPLIYSLRNK
EVTRAFMKILGKGKSGE
**ORS1E1**
   *Homo sapiens*_OR51E1_butyric olfactory receptor (GI: 14503)
DNA: SEQ ID NO: 15 
atggtggatcccaatggcaatgaatccagtgctacatacttcatcctaataggcctccctggtttagaagaggctcagttctggttggc
cttcccattgtgctccctctaccttattgctgtgctaggtaacttgacaatcatctacattgtgcggactgagcacagcctgcatgagcc
catgtatatatttctttgcatgctttcaggcattgacatcctcatctccacctcatccatgcccaaaatgctggccatcttctggttcaatt
ccactaccatccagtttgatgcttgtctgctacagatgtttgccatccactccttatctggcatggaatccacagtgctgctggccatgg
cttttgaccgctatgtggccatctgtcacccactgcgccatgccacagtacttacgttgcctcgtgtcaccaaaattggtgtggctgctg
tggtgcgggggctgcactgatggcacccttcctgtcttcatcaagcagctgcccttctgccgctccaatatcctttcccattcctactg
cctacaccaagatgtcatgaagctggcctgtgatgatatccgggtcaatgtcgtctatggccttatcgtcatcatctccgccattggcct
ggactcacttctcatctccttctcatatctgcttattcttaagactgtgttgggcttgacacgtgaagcccaggccaaggcatttggcact
tgcgtctctcatgtgtgtgctgtgttcatattctatgtacctttcattggattgtccatggtgcatcgctttagcaagcggcgtgactctcc
actgcccgtcatcttggccaatatctatctgctggttcctcctgtgctcaacccaattgtctatggagtgaagacaagggagattcgac
agcgcatccttcgacttttccatgtggccacacacgcttcagagccctag
PROT: SEQ ID NO: 16
**OR4S2**
   *Homo sapiens_OR4S2_DMTS* olfactory receptor (GI: 219431)
DNA: SEQ ID NO: 17 
atggaaaaaataaacaacgtaactgaattcattttctggggtctttctcagagcccagagattgagaaagtttgttttgtggtgttttct
ttcttctacataatcattcttctgggaaatctcctcatcatgctgacagtttgcctgagcaacctgtttaagtcacccatgtatttctttctc
agcttcttgtcttttgtggacatttgttactcttcagtcacagctcccaagatgattgttgacctgttagcaaaggacaaaaccatctcct
atgtggggtgcatgttgcaactgtttggagtacatttctttggttgcactgagatcttcatccttactgtaatggcctatgatcgttatgtg
gctatctgtaaacccctacattatatgaccatcatgaaccgggagacatgcaataaaatgttattagggacgtgggtaggtgggttct
tacactccattatccaagtggctctggtagtccaactacccttttgtggacccaatgagatagatcactacttttgtgatgttcaccctgt
gttgaaacttgcctgcacagaaacatacattgttggtgttgttgtgacagccaacagtggtaccattgctctggggagttttgttatctt
gctaatctcctacagcatcatcctagtttccctgagaaagcagtcagcagaaggcaggcgcaaagccctctccacctgtggctcccac
attgccatggtcgttatctttttcggcccctgtacttttatgtacatgcgccctgatacgacctttcagaggataagatggtggctgtatt
ttacaccattatcactcccatgttaaatcctctgatttatacactgagaaatgcagaagtaaagaatgcaatgaagaaactgtggggc
agaaatgttttcttggaggctaaagggaaatag
PROT: SEQ ID NO: 18 
MEKINNVTEFIFWGLSQSPEIEKVCFVVFSFFYIIILLGNLLIMLTVCLSNLFKSPMYFFLSFLSFVDICYSSVTA
PKMIVDLLAKDKTISYVGCMLQLFGVHFFGCTEIFILTVMAYDRYVAICKPLHYMTIMNRETCNKMLLGT
WVGGFLHSIIQVALVVQLPFCGPNEIDHYFCDVHPVLKLACTETYIVGVVVTANSGTIALGSFVILLISYSIILV
SLRKQSAEGRRKALSTCGSHIAMVVIFFGPCTFMYMRPDTTFSEDKMVAVFYTIITPMLNPLIYTLRNAEV
KNAMKKLWGRNVFLEAKGK
**OR51I2**
   *Homo sapiens*_OR51I2_3-methyl-2-hexenoic acid olfactory receptor (GI: 507078)
DNA: SEQ ID NO: 19 
atggggttgttcaatgtcactcaccctgcattcttcctcctgactggtatccctggtctggagagctctcactcctggctgtcagggcccc
tctgcgtgatgtatgctgtggcccttgggggaaatacagtgatcctgcaggctgtgcgagtggagcccagcctccatgagcccatgta
ctacttcctgtccatgttgtccttcagtgatgtggccatatccatggccacactgcccactgtactccgaaccttctgcctcaatgcccgc
aacatcacttttgatgcctgtctaattcagatgtttcttattcacttcttctccatgatggaatcaggtattctgctggccatgagttttga
ccgctatgtggccatttgtgaccccttgcgctatgcaactgtgctcaccactgaagtcattgctgcaatgggtttaggtgcagctgctcg
aagcttcatcaccctttttccctcttccctttcttattaagaggctgcctatctgcagatccaatgttctttctcactcctactgcctgcaccc
agacatgatgaggcttgcctgtgctgatatcagtatcaacagcatctatggactctttgttcttgtatccacctttggcatggacctgttt
tttatcttcctctcctatgtgctcattctgcgttctgtcatggccactgcttcccgtgaggaacgcctcaaagctctcaacacatgtgtgtc
acatatcctggctgtacttgcattttatgtgccaatgattggggtctccacagtgcaccgctttgggaagcatgtcccatgctacataca
tgtcctcatgtcaaatgtgtacctatttgtgcctcctgtgctcaaccctctcatttatagcgccaagacaaaggaaatccgccgagccat
tttccgcatgtttcaccacatcaaaatatga
PROT: SEQ ID NO: 20 
MGLFNVTHPAFFLLTGIPGLESSHSWLSGPLCVMYAVALGGNTVILQAVRVEPSLHEPMYYFLSMLSFSD
VAISMATLPTVLRTFCLNARNITFDACLIQMFLIHFFSMMESGILLAMSFDRYVAICDPLRYATVLTTEVIAA
MGLGAAARSFITLFPLPFLIKRLPICRSNVLSHSYCLHPDMMRLACADISINSIYGLFVLVSTFGMDLFFIFLSY
VLILRSVMATASREERLKALNTCVSHILAVLAFYVPMIGVSTVHRFGKHVPCYIHVLMSNVYLFVPPVLNPLI
YSAKTKEIRRAIFRMFHHIKI
**Olfr263**
   *Mus musculus*_Olfr263_1-octen-3-ol, DMTS, 3-hydroxy-3-methylhexanoic acid, transpirol, and 3-methyl-2-hexenoic acid olfactory receptor (GI:18341)
DNA: SEQ ID NO: 21 
atggacccaagcaattacagtactttgcatgtttttatcctgcttggattctctgaccaccctcacctggaaatgatcctctctggagttg
tcaccttcttttacattattacactggtgggtaacactgctataattcttgcatccctcctggatccccatctccatacaccaatgtacttt
ttcctcaggaatctatctttcctggatttgtgtttcacaacaagcattgtccctcagatgctggttaacttgtggggacccgaaaagacc
attagctctgtgggctgtattgttcagctctatgtgtatatggctgggctccattgagtgtctgctcctagctgtcatgtcctatgatcg
tttcacagctatttgtaagcccttgcattactttgtaatcatgaatccacgtctatgcgtcaagatgatagtcatggtctggggtattagt
ttggccaactctgtaatattatgcacactcactgtgaatttgcctcgatgtggacacaacatcctggaccacttcctgtgtgagttgcca
gccatggtcaggatagcttgtgtagacacgacaacagttgaattgtctgtttttgctctaggcattgtcattgtccttacacctctcatcc
ttattcttatttcctatggctacatagccaaaactgtgctcaacatgaagtcaaaggcagggcaacaaaaagcaatgaatacctgtgg
atcccatctcactgtggtctccatattctatggaactatcatctacatgtatctacaacccggtaacagggcctctaaggaccagggca
agttcctcaccctcttttacaccatcatcactccaagtctcaacccccctcatttacaccctaaggaacagagacatgaaagatgcactg
aaaaaactcatgaggttttaccacagatttgccgaagtaaggagaaactag
PROT: SEQ ID NO: 22 
MDPSNYSTLHVFILLGFSDHPHLEMILSGVVTFFYIITLVGNTAIILASLLDPHLHTPMYFFLRNLSFLDLCFTT
SIVPQMLVNLWGPEKTISSVGCIVQLYVYMWLGSIECLLLAVMSYDRFTAICKPLHYFVIMNPRLCVKMIV
MVWGISLANSVILCTLTVNLPRCGHNILDHFLCELPAMVRIACVDTTTVELSVFALGIVIVLTPLILILISYGYIA
KTVLNMKSKAGQQKAMNTCGSHLTVVSIFYGTIIYMYLQPGNRASKDQGKFLTLFYTIITPSLNPLIYTLRN
RDMKDALKKLMRFYHRFAEVRRN
**Olfr403**
   *Mus musculus*_Olfr403_1-octen-3-ol, DMTS, transpirol, and geonol olfactory receptor (GI:404316)
DNA: SEQ ID NO: 23 
atgagagaagaaaatgagtcttctaccatagatttcactctcctgggagttacaaggcagcgggagcaggaatatttcttcttcatcct
cttcctgttcatttaccccatcacagtgtttggaacatgctcatcatcctagccattcactctgacactcgcctccataaccctatgtac
tttttcctggccaacctctcccttgttgacatcttcttctcctctgtaactattcccaagatgctggccaaccatctcctaggtagcaaggc
catctcctttgggggatgtatggcacagatgtacttcatgatatcattgggaaacacagacagttatatactagctgcaatggcatatg
accgagctgtggctatcagtcgcccgcttcattatgcaacaattatgagtccacaactttgtgtcctgctggttgctgggtcctgggtga
ttgcaaatgctaatgcactgccccacaccctactcacagctagattgtccttctgtggcaataaggatgtggccaacttctactgtgac
attacacctttgctccagctgtcctgttctgacatccgcttcaatgtgaagatgatgtaccttggggtgggggtcttctctgtgccactgc
tgtgcatcatcatctcctatgtccgggtcttttccacagtcttgcgggttccatctaccaagggcttcctgaaggccttgtccacctgtgg
ctctcacctgacagtggtgtccttgtattatggacagtcatgggcatgtatttccggcccctgaccagttacagtctgaagcatgcatt
gataactgtgatgtacacggcagtgaccccaatgctgaaccctttcatctacagcctgaggaaccgggacatgaaggctgctctgaa
gaaactcttccactgccactcttcctcttcttccctcatgtga
PROT: SEQ ID NO: 24 
MREENESSTIDFTLLGVTRQREQEYFFFILFLFIYPITVFGNMLIILAIHSDTRLHNPMYFFLANLSLVDIFFSSV
TIPKMLANHLLGSKAISFGGCMAQMYFMISLGNTDSYILAAMAYDRAVAISRPLHYATIMSPQLCVLLVA
GSWVIANANALPHTLLTARLSFCGNKDVANFYCDITPLLQLSCSDIRFNVKMMYLGVGVFSVPLLCIIISYV
RVFSTVLRVPSTKGFLKALSTCGSHLTVVSLYYGTVMGMYFRPLTSYSLKHALITVMYTAVTPMLNPFIYSL
RNRDMKAALKKLFHCHSSSSSLM
**Olfr735**
   *Mus musculus*_Olfr735_1-octen-3-ol olfactory receptor (GI:257909)
DNA: SEQ ID NO: 25 
atggtacacgcagacaagcaagaagtactttctcataatttttcatttttgtttaatttaacctacttagtccagtttaatttttcttccact
aggtcgcttgatactcttggcttggagaaaaacaaaaacagctctgatgtgtcacgatttgtgcttctgggcctgtcgtcttcctggga
gctccagttgtttctcttcttcacatttttgttgatttacctggttattgtcctgggaaacctttttgatagtgatggtcgtccaagctgatgc
tcacctgttccagtcacccatgtactattttctaagccatctgtctttcattgacctttgcctgagctgtgtggctgtacccaaaatgctag
gagatttcctacagaaggagaagacaatatctttttcaggatgcctggcccaggtattctttcttcactttcttggagccagtgagatgt
tcctgttgactgttatggcctatgataggtatgttgccatatgcaacccctttgcactacctgacggtcatgaataaccacctgcgacttc
ggttggtgtttggctgctggtgtggaggtttcattcactctatcacacaggtcatgattgtcattcagctgcctttttgtggtcccaatga
actggacaacttctactgtgatgtcccccaggttgtcaagctggcctgcatggacacttacttggttgaggtgctgatggtctctaaca
gtggcatactgtctcttgtctgcttcttggtcctgctcttctcctatgctctcatcttgattactctgagaactcacctccaccggggccag
agcaaggcactgtccacctgtgcttctcacttgacggtggtcagcctgatctttgtaccctgtgtattcatttacttgaggcctttctgtac
cttctctgtggataaagtagtctctgtgttttacacagtgatcacacctatgctgaacccccctcatctatacactcagaaatgcagacat
gaagcaagccatagaaaagctgagaaagaagcaagtggcatcccactgctttgctaaaggatga
PROT: SEQ ID NO: 26 
MVHADKQEVLSHNFSFLFNLTYLVQFNFSSTRSLDTLGLEKNKNSSDVSRFVLLGLSSSWELQLFLFFTFLLI
YLVIVLGNLLIVMVVQADAHLFQSPMYYFLSHLSFIDLCLSCVAVPKMLGDFLQKEKTISFSGCLAQVFFLHF
LGASEMFLLTVMAY
DRYVAICNPLHYLTVMNNHLRLRLVFGCWCGGFIHSITQVMIVIQLPFCGPNELDNFYCDVPQVVKLACM
DTYLVEVLMVSNSGILSLVCFLVLLFSYALILITLRTHLHRGQSKALSTCASHLTVVSLIFVPCVFIYLRPFCTFS
VDKVVSVFYTVITPMLNPLIYTLRNADMKQAIEKLRKKQVASHCFAKG
**Olfr96**
   *Mus musculus_Olfr96_* geonol olfactory receptor (GI:258507)
DNA: SEQ ID NO: 27 
atgggaatcctttccacaggaaatcaaactgtcactgagtttgtacttcttggtttccatgaagtccctgggctgcacctcctgtttttttc
tgtgttcaccatcctctatgcctccatcatcacagggaacatgctcattgcagtggtggtgagctcccagaggcttcacacaccca
tgtatttctttctggtgaatctgtccttcatagagattgtctatacctccacagtggtgcccaaaatgctggaaggcttcttacaggagg
ccaccatatctgtggctggctgcttgctccagttctttgtttttggctctctggccacagatgagtgttttctgctggctgtgatggcatat
gatcgatatctcgcaatttgtcaccctctacgatacccacacctcatggggcctcaatggtgcctggggttggtgctcacagtctggct
gtctggcttcatggtagatggactagttgttgctctgatggcccagttgagattctgtggccccaacttagttgatcacttttactgtgat
ttttcacctttgatggtcctggcttgctcagatacccaagtggcccaggtgactacatttgttctctctgtggtcttcctgactgtcccttt
gggctggttctgatctcctatgctcagattgtagtgactgtgctgagagttccttctgggaccagaagaaccaaggccttctccacatg
ctcctctcacctggctgtggtgtccacgttctatggaacactcatggtattgtacattgtgccctctgctgttcattctcagctcctctcca
aggtcattgccctgctctacacagtggtcactcccatcttcaaccctgtcatctacaccttgaggaaccaggaggtgcagcaggcact
aagaaggcttctctactgcaaaccaactgaaatgtga
PROT: SEQ ID NO: 28 
MGILSTGNQTVTEFVLLGFHEVPGLHLLFFSVFTILYASIITGNMLIAVVVVSSQRLHTPMYFFLVNLSFIEIV
YTSTVVPKMLEGFLQEATISVAGCLLQFFVFGSLATDECFLLAVMAYDRYLAICHPLRYPHLMGPQWCLGL
VLTVWLSGFMVDGLVVALMAQLRFCGPNLVDHFYCDFSPLMVLACSDTQVAQVTTFVLSVVFLTVPFGL
VLISYAQIVVTVLRVPSGTRRTKAFSTCSSHLAVVSTFYGTLMVLYIVPSAVHSQLLSKVIALLYTVVTPIFNPV
IYTLRNQEVQQALRRLLYCKPTEM
**Olfr558**
   *Mus musculus_Olfr558_* butyric olfactory receptor (GI: 259097)
DNA: SEQ ID NO: 29 
atggtgggcttcaatagcaatgaatccagtgccacatatttcatcttaataggccttccaggattggaagaggttcagttttggctggc
tttccctttgtgttccctctaccttattgctgtgctaggtaacttgacaatcatctacattgtgaggacagagcacagcctgcatgagccc
atgtatatctttctttgcatgctttctggcctagacatcctcatttccacctcatccatgccaaaaatgatggccatcttttggttcaattc
cactaccatccagtttgatgcttgtctggtacagatgtttgccatccattccttgtctggcatggagtccacagtgctgctggccatggcc
tttgaccgctatgtggccatttgccatcctctacggcatgccactgtgctgacattgcctcgtgttgccaagattggcatggctgctgtg
gtcaggggtgcagtactaatggcacccttacctgtcttcatcaaaaggttgcctttctgtcgttccaacatcctttctcattcctactgcct
acaccaagatgtcatgaagctggcctgtgctgacatccgtgtcaatatcatttatggactcattgtcatcatctcagccattggtttgga
ctcacttctcatttccttctcatatttgctcatcctcaagactgtgttgggcttgacacgtgaagcccaggcaaaagcgtttggcacttgt
gtttcacatgtgtgtgctgttttcatcttctatgtgcctttcattggattgtcgatggtgcaccgtttcagcaagaggcgtgactctctcct
gcctgtcattatggctaacatctatctgctagttcctcctgtgctcaaccccattgtctatggagtaaagacaaaggagatccggcagc
gcattcttcgtcttttcctcgtgaccacacacacttcagatcactag
PROT: SEQ ID NO: 30 
MVGFNSNESSATYFILIGLPGLEEVQFWLAFPLCSLYLIAVLGNLTIIYIVRTEHSLHEPMYIFLCMLSGLDILIS
TSSMPKMMAIFWFNSTTIQFDACLVQMFAIHSLSGMESTVLLAMAFDRYVAICHPLRHATVLTLPRVAKI
GMAAVVRGAVLMAPLPVFIKRLPFCRSNILSHSYCLHQDVMKLACADIRVNIIYGLIVIISAIGLDSLLISFSYL
LILKTVLGLTREAQAKAFGTCVSHVCAVFIFYVPFIGLSMVHRFSKRRDSLLPVIMANIYLLVPPVLNPIVYGV
KTKEIRQRILRLFLVTTHTSDH
**Olfr1193**
   *Mus musculus_Olfr1193_DMTS_olfactory* receptor (GI: 329460)
DNA: SEQ ID NO: 31 
atggcctcaaggacttattccatggaagaagtaaataatgtcactgaattcattttcttgggtctttctcagaaccctgaggttgaaaa
agtgtgctttgtggtgttctccttcttttacatggtcattctgctaggaaacctcctcatcatgttgacagtttgcagtggcaatcttttca
agtttcccatgtatttttcctcaactttctgtcttttgtggacatttgctactcctcagtcacagcacccaagatgattattgacctgttag
tgaagaaaaagactatatcctatgtggggtgcatgttacaactctttgtggttcatttctttggttgcactgagatcttcattcttactgtc
atggcctatgatagatatgtggccatttgtaaacctctccactatatgactatgatggaccgggaaagatgcaataagatgttgctcg
gaacatggatcggtggcttcttacattctattatccaagtggctcttgtggtccagctcccctttgtggaccgaatgagattgatcacta
tttctgtgatgtacatcctgtactgaaacttgcctgcactgacacttacattgttggtatttttgtgacagcaaacagtggcaccattgca
ttgggaagttttgtcatcttgctgatctcatacacagtcattctcatgtctctgagaaagcagtcatctgaaggcagacgcaaagctctc
tccacttgtggatcccacattgctgttgtcatcatttttttggcccctgtacttttatgtatatgcggcctgacactaccttctctgaggac
aagatggtagctatattttacaccattatcactcccatgctgaatcctctaatttacactctaagaaatgcagaagtaaagaatgcaat
gagaaaactgtgggctagaaagttttcctgggaaactactgggaaatag
PROT: SEQ ID NO: 32 
MASRTYSMEEVNNVTEFIFLGLSQNPEVEKVCFVVFSFFYMVILLGNLLIMLTVCSGNLFKFPMYFFLNFLS
FVDICYSSVTAPKMIIDLLVKKKTISYVGCMLQLFVVHFFGCTEIFILTVMAYDRYVAICKPLHYMTMMDRE
RCNKMLLGTWIGGFLHSIIQVALVVQLPFCGPNEIDHYFCDVHPVLKLACTDTYIVGIFVTANSGTIALGSF
VILLISYTVILMSLRKQSSEGRRKALSTCGSHIAVVIIFFGPCTFMYMRPDTTFSEDKMVAIFYTIITPMLNPLI
YTLRNAEVKNAMRKLWARKFSWETTGK
**Olfr641**
   *Mus musculus*_Olfr641_3-methyl-2-hexenoic acid olfactory receptor (GI: 25075)
DNA: SEQ ID NO: 33 
atggcgctgttcaatgtcactcaccctgcctcctttctcctgactggtatccctggtctggagagcctccacccctggctggcaggaccc
ctttgtgtgatgtatgccgtggccctcggggcaaatactgtgattctgcaggctgtgagagtggagcccatcctccatgctcccatgta
ctatttcctgtccatgttgtctttcagtgatgtggccatgtcaatggccacactgcccaccgtgctgagaaccttctgctttgatgcccgc
agcattgcttttgacgcctgtctagtccagatgttcctcattcattctttctcgatgatggagtcagggattctgctggctatgagttttga
ccgctatgtggccatatgcaatcccttgcattatgccactgtcctcaccaatgaattcattgctgggatgggcttagctgtgactgctcg
aagcttcatcaccctttttccctctgcccttcctcatcaaaaggctacctatctgcaaatccaatgttctttcccactcctactgcctgcacc
cagacatgatgaagcttgcctgtgctgacatcaccatcaacagtatttatggactctttgttcttgtgtctacctttggcatggacctgct
tttatttttcctctcctatgtgctcattctgcgttctgttatggccattgcttctcatgaggaacgccttaaagctctcaacacatgtgtatc
acatatcttggctgtgcttgcattttatgtgccaatgataggggtctctacagttcaccgttttgggaagcatgccccacgatacataca
tgtcctcatgtccaatgtctacctctttgtgcctcctgttctcaaccctctcatttacagtgccaagacaaaggagatccgccgtgccata
ttccgcatgtttcgccgtatcaaactgtga
PROT: SEQ ID NO: 34 
MALFNVTHPASFLLTGIPGLESLHPWLAGPLCVMYAVALGANTVILQAVRVEPILHAPMYYFLSMLSFSDV
AMSMATLPTVLRTFCFDARSIAFDACLVQMFLIHSFSMMESGILLAMSFDRYVAICNPLHYATVLTNEFIA
GMGLAVTARSFITLFPLPFLIKRLPICKSNVLSHSYCLHPDMMKLACADITINSIYGLFVLVSTFGMDLLFIFLS
YVLILRSVMAIASHEERLKALNTCVSHILAVLAFYVPMIGVSTVHRFGKHAPRYIHVLMSNVYLFVPPVLNP
LIYSAKTKEIRRAIFRMFRRIKL
**Olfr137**
   *Mus musculus*_Olfr137_1-octen-3-ol, and transpirol olfactory receptor (GI: 258481)
DNA: SEQ ID NO: 35 
Atggtggaaaatttcaatgcaagttgggaagggtactttatttttctgggtttttctaaatggcctcatctggaagttgttctctttgtggtgata
ttgatattctacatgatgacactgatgggcaatctcttcatcataattttgtcacacctagattcccacctgcataccccctatgtacttcttcctct
caaatctctctgctttggatctctgttataccactagctctgtcccccagctgctatttaacctctggggcccaaagaaaacaatatcttatgc
tggttgcatgcttcagctctattttgtccttgctctgggtaccacagagtgtgttctgttggtggtgatgtcctatgaccgctatgtagctgtgt
gtaaacccttgcattactctgtacttatgaatcctcgtttttgccaactgctggctgcagcatcctgggtatgtggctttaccacttcagcccctt
cactcttcctttaccttctgggtacccctgtgtggccatcgcaaagtagaccacttcttctgtgaagtgcctgcactgttgcagttgtcctgtg
ttgatatccatgcaaatgagatgactctcatggtcatgagtgctatttttgttgtcataccacttattctcatcctgagctcctatgctgccattg
catggactgtcctggaaatgcaatcaaccactcgacttcagaaagttttcgggacctgtggagcccatcttactgttgtttctctctttttcatt
ccaatcatgtgcatttatcttcagccttcaacaaaaagttctcaagatcatgccaagttcattgctctcttctacaccgttgtcacacctagcct
caaccctctcatttatactctaagaaacaaagatgtgagaggggcaataaggaggctgagtaggtatgagagggagaaatga
PROT: SEQ ID NO: 36 
MVENFNASWEGYFIFLGFSKWPHLEVVLFVVILIFYMMTLMGNLFIIILSHLDSHLHTPMYFFLSNLSALDL
CYTTSSVPQLLFNLWGPKKTISYAGCMLQLYFVLALGTTECVLLVVMSYDRYVAVCKPLHYSVLMNPRFC
QLLAAASWVCGFTTSALHSSFTFWVPLCGHRKVDHFFCEVPALLQLSCVDIHANEMTLMVMSAIFVVIPLI
LILSSYAAIAWTVLEMQSTTRLQKVFGTCGAHLTVVSLFFIPIMCIYLQPSTKSSQDHAKFIALFYTVVTPSLN
PLIYTLRNKDVRGAIRRLSRYEREK
**Olfr173**
   *Mus musculus*_Olfr173_1-octen-3-ol, transpirol, and DMTS olfactory receptor (GI: 259002)
DNA: SEQ ID NO: 37 
atggttgaagaaaaccacaccatgaaacgtgagtttgtcctcacaggatttacagaccaccctgaaatgaagggccttctgtttgcag
tattctttttcatctatctgattaccatgatagggaatatgggtctggtgattctgatttccaaagaacgttctcttcacaccccaatgtac
atctttctgggcaacctggcttttatagattcttgttgcctgtgctattacccctaaaatgttggagaactttttctctgaggacagaat
aatctctctgtatgaatgtatggcacagttttatttctgtgcactgttgaaactgcagactgctttcttctgtcagcaatggcctatgacc
gctatgtggccatatgcaacccactgcagtaccacaccacgatgtccaagaagctctgccttcagatgacgacgggagctttcatagc
tggaaacctgcattctatggttcatgtggggctattatttagactagcattctgtgggtctaatcaaatcaaccacttttattgtgatatt
cttcctttgtacagactctcctgtgttgacccctatatcaacgagcttgtattatttgtcttttcaggctcaatccaagtcttcacaatagg
ttgtgtcctcatatcttacctttttcattgtttatacaattttccaaatgaaatccaaagagggaaggatcaaagccttctccacctgtgca
tcccactttctgtctgtttcactattctatggatctcttttgtttatgtacattagaccaaatttgctagaagaaggtgataaggacatgcc
agctgctattttgtttacaatagtggttcccttactaaacccatttatttatagcttgagaaataaagaagtaaagaatgtcttgcagaa
aattctgcagaaaaaaataatttcaaaaaattttaaacaagcatctattattgtgtaa
PROT: SEQ ID NO: 38 
MVEENHTMKREFVLTGFTDHPEMKGLLFAVFFFIYLITMIGNMGLVILISKERSLHTPMYIFLGNLAFIDSC
CACAITPKMLENFFSEDRIISLYECMAQFYFLCTVETADCFLLSAMAYDRYVAICNPLQYHTTMSKKLCLQ
MTTGAFIAGNLHSMVHVGLLFRLAFCGSNQINHFYCDILPLYRLSCVDPYINELVLFVFSGSIQVFTIGCVLIS
YLFIVYTIFQMKSKEGRIKAFSTCASHFLSVSLFYGSLFFMYIRPNLLEEGDKDMPAAILFTIVVPLLNPFIYSLR
NKEVKNVLQKILQKKIISKNFKQASIIV
**Olfr164**
   *Mus musculus_Olfr164_geonol* olfactory receptor (GI: 258443)
DNA: SEQ ID NO: 39 
atgatacaatggaacaactggaccaggaactctgatttcatcctactaggattatttgatcacagtcctcttcatactttttcttttccct
aattctgggtatcttcttcatggccttcataggaaattctatcatggtgattctcatctacttggatgctcatctccacacccccatgtac
atccttctcagccaactttctctcatggatctcatgctcatctgtactacagtaccccaaatggccttcaacttcctttctggaaacaagt
ccatctccatggttggctgtggaatccagatattcttctatgtatctctacttggagctgagtgtttttgttggccgcaatggcttatgac
cgttatgtagctatttgctacccattaaggtaccctattctcatgagtgacaaaatatgtggcctcatggctgcctcctcttgggttcttg
gctctcttgatggtataatagaagttgcagctgcattgtctttttcatattgtggtgccagagaaatacctcatttttctgtgatgttcct
gccttactcacactttcatgtagtaataccttgatatttgaaagaattatatttttctgctgtgtaattatgcttactttacctgtagcaatc
attattgcttcctatactcgtgtgattctgaccgttcttcatatgagctcagctgagagtcgccacaaagcttttgccacctgttcctctca
cctcatggtggtgggaatgtactatggggcagccatgttcatatacatgcggccttcttctggtcgttctcccacccaggacaaaatag
tgtcagctttctacactatccttacgccaatgttgaacccccctcatttatagtctccggaacaaagaagtagccagagcattcatgaaa
gtgttagggattgataaggcagcagcatga
PROT: SEQ ID NO: 40 
MIQWNNWTRNSDFILLGLFDHSPLHTFFFSLILGIFFMAFIGNSIMVILIYLDAHLHTPMYILLSQLSLMDL
MLICTTVPQMAFNFLSGNKSISMVGCGIQIFFYVSLLGAECFLLAAMAYDRYVAICYPLRYPILMSDKICGL
MAASSWVLGSLDGIIEVAAALSFSYCGAREIPHFFCDVPALLTLSCSNTLIFERIIFFCCVIMLTLPVAIIIASYT
RVILTVLHMSSAESRHKAFATCSSHLMVVGMYYGAAMFIYMRPSSGRSPTQDKIVSAFYTILTPMLNPLIY
SLRNKEVARAFMKVLGIDKAAA
**Olfr1487**
   *Mus Musculus_Olfr1487_geonol* olfactory receptor (GI: 258629)
DNA: SEQ ID NO: 41 
Atgattcagaatatttcagaattgtctgaatttattcttgttgggttaacagatgcaccattcctgcaaattcctttatttatcatctttact
ctcatttatttgacaacattgtttgggaatcttgggatgattttgctgattctgctggactccagactccacactcccatgtactttttcct
cagtaacctctctggtggactgtgtttatgcctcagctgtcactcccaaggtgatggaagggtttctcacaggaaataagatcatat
cctacaatgcatgtgctgcccagatgttcttctttgcagcctttgctactgttgaaagtttcatgctggcctcaatggcctatgaccgtca
tgcagcagtgtgcaaacccttgcattactccactactatgacaactacgatatgtgtcctgcttcttgctggatcttatgtcagtggactc
ttacaatcttccattcatgtttccttcacatttcaactctccttctgccattctaatgtagtcaatcactttttctgtgatatcccaccactat
tagccctttcttgctccagtattcacacaaatgaaattatactctttatgttggcagcattcaatgttgcttttactctattagttatattgt
cctcttacttactaattttgttgcaattctgagaatgcgctctgctgagagcaggaagaaggccatttccacctgtgcatcccacctta
ccactgtttccatcttctatggcacaattatcttcatgtacttacagcccagctccaatcactccatggatactgacaagatggcatctg
ttttctacaccatggtcattcccatgctgaaccctcttgtatatagcctgagaaataaagaagtcaagaatgcattcaagaaggttgct
ggaaaagcagtgctttcactgggattagtcaattaa
PROT: SEQ ID NO: 42 
MIQNISELSEFILVGLTDAPFLQIPLFIIFTLIYLTTLFGNLGMILLILLDSRLHTPMYFFLSNLSLVDCVYASAVT
PKVMEGFLTGNKIISYNACAAQMFFFAAFATVESFMLASMAYDRHAAVCKPLHYSTTMTTTICVLLLAGS
YVSGLLQSSIHVSFTFQLSFCHSNVVNHFFCDIPPLLALSCSSIHTNEIILFMLAAFNVAFTLLVILSSYLLIFVAI
LRMRSAESRKKAISTCASHLTTVSIFYGTIIFMYLQPSSNHSMDTDKMASVFYTMVIPMLNPLVYSLRNKE
VKNAFKKVAGKAVLSLGLVN
**Olfr339**
   *Mus musculus_Olfr339_geonol* olfactory receptor (GI:258951)
DNA: SEQ ID NO: 43 
atgaggatggataatgacagcgctctgtctgagttcatcctcctggggctccccattcgagcagaagaccaagctttgtactctgtcct
gatcctggccatgtacctgacaactgtgctggggaacctgctcatcatcctactcatcaggctggactctcacctccacacccccatgt
acttcttcctcagccacttggccttcacagacatctctttttcatcagtcacagctcctaagatgctcgtgaatatgctgacacacagta
agtccatcccatatacagggtgtgtttcccaggtgtatttttcactgtttttgcaagcattgatagctttcttctaacatccatggcatat
gacaggtatgtggccatctgccatcctctgcattataacatcatcatgaatctgaggctctgtgttcttctagtagtaatatcatgggcc
ttatctttaaccaatgctcttgcgcacacccttctcttggctcgcctatctcactttagaaacaataccatcccccactatttctgtgacct
ctctaccttgctgaagctgtctagctcagacaccaccatcaatgagctggtcatctttgttttaggtaatgtggtcattaccctgccattc
atatgcattctggtttcttatggctacattggtgtcaccattctgaaaactccctccatcaagggaatccacaaagccttgtccacgtgt
ggctctcacctctgtgtggtatccttgtactatggggccatcattggactatattttgtcccctcatctaataacactaatgacaaagatg
tcattgtggctgtgatgtacactgtggtcatacccatgctgaatccctttatttatagtctgaggaatcgggatatgaaaagaacactg
agaaatatcctcagcagaacaaaatga
PROT: SEQ ID NO: 44 
MRMDNDSALSEFILLGLPIRAEDQALYSVLILAMYLTTVLGNLLIILLIRLDSHLHTPMYFFLSHLAFTDISFSS
VTAPKMLVNMLTHSKSIPYTGCVSQVYFFTVFASIDSFLLTSMAYDRYVAICHPLHYNIIMNLRLCVLLVVIS
WALSLTNALAHTLLLARLSHFRNNTIPHYFCDLSTLLKLSSSDTTINELVIFVLGNVVITLPFICILVSYGYIGVTI
LKTPSIKGIHKALSTCGSHLCVVSLYYGAIIGLYFVPSSNNTNDKDVIVAVMYTVVIPMLNPFIYSLRNRDMK
RTLRNILSRTK
**Olfr1126**
   *Mus musculus_Olfr1126_* 3-hydroxy-3-methylhexanoic acid olfactory receptor (GI:258834)
DNA: SEQ ID NO: 45 
atgaaattcacagaaatcagggcagaggacaatgcttccactgtgacagagtttctcctcctgggattttctgaccttcctaacttaca
agggattttgtttgggttgttctccataatttacttgatcatattgattggaaatagtttcataattgtgataactagaattgatcctgcac
tacagaagcccatgtatttttcctggcaaatttttcttctctggaaatctgttatgtatcagtcactctccctaggattctgtttaacattg
ctactcaagacagaagcatttctgtggtgagctgtgccacacaaatgtgtttcttccttatgctgggagctactgagtgtttcctgctgg
ctgtgatgtcctatgatcgctatgtggccatctgcaatcctctgcactatcctctggtcatgaacccaacaaagtgcactcagctggca
gcagcctcctggcttggaggcatccctgtccagataggacaaacctgtcagatatttctctacatttctgcaattctaaccaaatagat
cacttcttttgtgacttacctcccattctcaagctggcctgtggagatacttcaatacacgagttgtctgtctacttagtagctatgctgtt
tgttgcattccctttcttgttgatacttgcctcttataccaaaatcattgccaccattctgaagttgccaacagccacaggacgggcaaa
agccttctcaacatgttcttcccatttgcttgtggtgtttttgtttttggatcagctactattacctacttgaggccaaagtccacccattc
tccaggaactgataaactactctctttgttttacaccattgtgactcccatgttcaatcctctgatatacagtcttagaaataaggaggt
gattgctgcactgagaaaactgttacatatcaagtag
PROT: SEQ ID NO: 46 
MKFTEIRAEDNASTVTEFLLLGFSDLPNLQGILFGLFSIIYLIILIGNSFIIVITRIDPALQKPMYFFLANFSSLEIC
YVSVTLPRILFNIATQDRSISVVSCATQMCFFLMLGATECFLLAVMSYDRYVAICNPLHYPLVMNPTKCTQL
AAASWLGGIPVQIGQTCQIFSLHFCNSNQIDHFFCDLPPILKLACGDTSIHELSVYLVAMLFVAFPFLLILASY
TKIIATILKLPTATGRAKAFSTCSSHLLVVFLFFGSATITYLRPKSTHSPGTDKLLSLFYTIVTPMFNPLIYSLRNK
EVIAALRKLLHIK
**FLAG^{®} tag**
   DNA - SEQ ID NO: 47
   gattacaaggacgacgacgataag
Protein - SEQ ID NO: 48
   DYKDDDDK
Rho tag
   DNA - SEQ ID NO: 49
   atgaacgggaccgagggcccaaacttctacgtgcctttctccaacaagacgggcgtggtg
Protein - SEQ ID NO: 50
   MNGTEGPNFYVPFSNKTGVV
**Lucy tag**
   DNA - SEQ ID NO: 51
   atgagaccccagatcctgctgctcctggccctgctgaccctaggcctggct
Protein - SEQ ID NO: 52
   MRPQILLLLALLTLGLA
SEQ ID NO: 53
   Motif
   MAYDRYVAIC
SEQ ID NO: 54
   Motif
   FSTCSSH
SEQ ID NO: 55
   Motif
   PMLNPFIY
**OR2H1**
   *Homo sapiens*_OR2H1_1-octen-3-ol olfactory receptor (GI: 937628050)
DNA: SEQ ID NO: 56 
ATGGTTAACCAAAGCTCCCCATGGGCTTCCTCCTTCTGGGCTTCTCTGAACACCCAGCACTGGAAAG
GACTCTCTTTGTGGTTGTCTTCACTTCCTACCTCTTGACCCTGGTGGGCAACACACTCATCATCCTGCT
GTCTGTACTGTACCCCAGGCTCCACTCTCCAATGTACTTTTCCTCTCTGACCTCTCCTTCTTGGACCTC
TGCTTTACCACAAGTTGTGTCCCCCAGATGCTGGTCAACCTCTGGGGCCCAAAGAAGACCATCAGCTT
CCTGGGATGCTCTGTCCAGCTCTTCATCTTCCTGTCCCTGGGGACCACTGAGTGCATCCTCCTGACAGT
GATGGCCTTTGACCGATACGTGGCTGTCTGCCAGCCCCTCCACTATGCCACCATCATCCACCCCCGCCT
GTGCTGGCAGCTGGCATCTGTGGCCTGGGTTATGAGTCTGGTTCAATCGATAGTCCAGACACCATCC
ACCCTCCACTTGCCCTTCTGTCCCCACCAGCAGATAGATGACTTTTTATGTGAGGTCCCATCTCTGATT
CGACTCTCCTGTGGAGATACCTCCTACAATGAAATCCAGTTGGCTGTGTCCAGTGTCATCTTCGTGGT
TGTGCCTCTCAGCCTCATCCTTGCCTCTTATGGAGCCACTGCCCAGGCAGTGCTGAGGATTAACTCTG
CCACAGCATGGAGAAAGGCCTTTGGGACCTGCTCCTCCCATCTCACTGTGGTCACCCTCTTCTACAGC
TCAGTCATTGCTGTCTACCTCCAGCCCAAAAATCCGTATGCCCAAGGAGGGGCAAGTTCTTTGGTCT
CTTCTATGCAGTGGGCACTCCTTCACTTAACCCTCTCGTATACACCCTGAGGAACAAGGAGATAAAGC
GAGCACTCAGGAGGTTACTAGGGAAGGAAAGAGACTCCAGGGAAAGCTGGAGAGCTGCTTAA
PROT: SEQ ID NO: 57 
MVNQSSPMGFLLLGFSEHPALERTLFVVVFTSYLLTLVGNTLIILLSVLYPRLHSPMYFFLSDLSFLDLCFTTSC
VPQMLVNLWGPKKTISFLGCSVQLFIFLSLGTTECILLTVMAFDRYVAVCQPLHYATIIHPRLCWQLASVA
WVMSLVQSIVQTPSTLHLPFCPHQQIDDFLCEVPSLIRLSCGDTSYNEIQLAVSSVIFVVVPLSLILASYGATA
QAVLRINSATAWRKAFGTCSSHLTVVTLFYSSVIAVYLQPKNPYAQGRGKFFGLFYAVGTPSLNPLVYTLRN
KEIKRALRRLLGKERDSRESWRAA
**OR2W3**
   *Homo sapiens*_OR2W3_1-octen-3-ol olfactory receptor (GI: 937628231)
DNA: SEQ ID NO: 58 
ATGGATGGAACCAATGGCAGCACCCAAACCCATTTCATCCTACTGGGATTCTCTGACCGACCCCATCT
GGAGAGGATCCTCTTTGTGGTCATCCTGATCGCGTACCTCCTGACCCTCGTAGGCAACACCACCATCA
TCCTGGTGTCCCGGCTGGACCCCCACCTCCACACCCCCATGTACTTCTTCCTCGCCCACCTTTCCTTCCT
GGACCTCAGTTTCACCACCAGCTCCATCCCCCAGCTGCTCTACAACCTTAATGGATGTGACAAGACCA
TCAGCTACATGGGCTGTGCCATCCAGCTCTTCCTGTTCCTGGGTCTGGGTGGTGTGGAGTGCCTGCTT
CTGGCTGTCATGGCCTATGACCGGTGTGTGGCTATCTGCAAGCCCCTGCACTACATGGTGATCATGAA
CCCCAGGCTCTGCCGGGGCTTGGTGTCAGTGACCTGGGGCTGTGGGGTGGCCAACTCCTTGGCCATG
TCTCCTGTGACCCTGCGCTTACCCCGCTGTGGGCACCACGAGGTGGACCACTTCCTGCGTGAGATGCC
CGCCCTGATCCGGATGGCCTGCGTCAGCACTGTGGCCATCGACGGCACCGTCTTTGTCCTGGCGGTG
GGTGTTGTGCTGTCCCCCTTGGTGTTTATCCTGCTCTCTTACAGCTACATTGTGAGGGCTGTGTTACAA
ATTCGGTCAGCATCAGGAAGGCAGAAGGCCTTCGGCACCTGCGGCTCCCATCTCACTGTGGTCTCCCT
TTTCTATGGAAACATCATCTACATGTACATGCAGCCAGGAGCCAGTTCTTCCCAGGACCAGGGCATGT
TCCTCATGCTCTTCTACAACATTGTCACCCCCCTCCTCAATCCTCTCATCTACACCCTCAGAAACAGAGA
GGTGAAAGGGGGCACTGGAAGGTTGCTTCTGGGGAAGAGAGAGCTAGGAAAGGAGTA
PROT: SEQ ID NO: 59 
MDGTNGSTQTHFILLGFSDRPHLERILFVVILIAYLLTLVGNTTIILVSRLDPHLHTPMYFFLAHLSFLDLSFTT
SSIPQLLYNLNGCDKTISYMGCAIQLFLFLGLGGVECLLLAVMAYDRCVAICKPLHYMVIMNPRLCRGLVSV
TWGCGVANSLAMSPVTLRLPRCGHHEVDHFLREMPALIRMACVSTVAIEGTVFVLAVGVVLSPLVFILLSY
SYIVRAVLQIRSASGRQKAFGTCGSHLTVVSLFYGNIIYMYMQPGASSSQDQGMFLMLFYNIVTPLLNPLIY
TLRNREVKGALGRLLLGKRELGKE
**OR8G1**
   *Homo sapiens*_OR8G1_1-octen-3-ol olfactory receptor (GI:937628054)
DNA: SEQ ID NO: 60 
ATGTCAGGAGAAAATAATTCCTCAGTGACTGAGTTCATTCTGGCTGGGCTCTCAGAACAGCCAGAGC
TCCAGCTGCCCCTCTTCCTCCTGTTCTTAGGAATCTATGTGGTCACAGTGGTGGGCAACCTGGGCATG
ACCACACTGATTTGGCTCAGTTCTCACCTGCACACCCCTATGTACTATTTCCTCAGCAGTCTGTCCTTCA
TTGACTTCTGCCATTCCACTGTCATTACCCCTAAGATGCTGGTGAACTTTGTGACAGAGAAGAACATC
ATCTCCTACCCTGAATGCATGACTCAGCTCTACTTCTTCCTCGTTTTTGCTATTGCAGAGTGTCACATGT
TGGCTGCAATGGCGTATGACCGTTACATGGCCATCTGTAGCCCCTTGCTGTACAGTGTCATCATATCC
AATAAGGCTTGCTTTTCTCTGATTTTAGGGGTGTATATAATAGGCCTGGTTTGTGCATCAGTTCATACA
GGCTGTATGTTTAGGGTTCAATTCTGCAAATTTGATTTGATTAACCATTATTTCTGTGATCTTCTTCCCC
TCCTAAAGCTCTCTTGCTCTAGTATCTATGTCAACAAACTACTTATTCTATGTGTTGGTGCATTTAACAT
CCTTGTCCCCAGCCTGACCATCCTTTGCTCTTACATCTTTATTATTGCCAGCATCCTCCACATTCGCTCC
ACTGAGGGCAGGTCCAAAGCCTTCAGCACTTGTAGCTCCCACATGTTGGCGGTTGTAATCTTTTTTGG
ATCTGCAGCATTCATGTACTTGCAGCCATCTTCAATCAGCTCCATGGACCAGGGGAAAGTATCCTCTG
TGTTTTATACTATTATTGTGCCCATGTTGAACCCTCTGATTTATAGCCTGAGGAATAAAGATGTCCATG
TTTCCCTGAAGAAAATGCTACAGAGAAGAACATTATTGTAA
PROT: SEQ ID NO: 61 
MSGENNSSVTEFILAGLSEQPELQLPLFLLFLGIYVVTVVGNLGMTTLIWLSSHLHTPMYYFLSSLSFIDFCH
STVITPKMLVNFVTEKNIISYPECMTQLYFFLVFAIAECHMLAAMAYDRYMAICSPLLYSVIISNKACFSLILG
VYIIGLVCASVHTGCMFRVQFCKFDLINHYFCDLLPLLKLSCSSIYVNKLLILCVGAFNILVPSLTILCSYIFIIASI
LHIRSTEGRSKAFSTCSSHMLAVVIFFGSAAFMYLQPSSISSMDQGKVSSVFYTIIVPMLNPLIYSLRNKDVH
VSLKKMLQRRTLL
**Olfr93**
   *Mus musculus*_Olfr93_1-octen-3-ol olfactory receptor (GI: 32060027)
DNA: SEQ ID NO: 62 
ATGGTCAACCAGAGCACCCCCGTGGGCTTCCTCCTGGGCTTCTCTGAACACCCACAACTGGAAAA
GGTTCTCTTCGTCGTTGTCTTGTGCTCCTACCTCCTCACCCTCCTAGGAAACACACTCATCCTCCTGCTG
TCCACCCTGGACCCCAGGCTCCACTCTCCAATGTACTTCTTCCTCTCTAACCTCTCCTTCTTGGACCTCT
GCTTCACCACAACCTGTGTTCCCCAGATGTTGTTCAACCTCTGGGGCCCACAAAGACCATCAGCTTCC
TGGGATGCTCTGTCCAGCTCTTCATCTTCATGCTCCTGGGGACAACAGAGTGCATCCTGTTGACAGTA
ATGGCCTTTGACCGCTATGTGGCTGTCTGTCAGCCCCTGCACTATGCCACCATAATCCACCCCCGCCTA
TGTCGCCAGTTGGCAGGTGTGGCCTGGGCTATAGGTTTGGTCCAATCCATAGTTCAAATACCACCCAC
CCTCACACTGCCCTTCTGTTCCCATAGGCAAATAGATGACTTTCTGTGTGAAGTCCCATCTCTAATCCG
ACTCTCTTGTGGGGACACCACTTTTAATGAGATTCAGTTGTCAGTTGCAGGTGTCATCTTCCTGCTTGT
ACCTCTGAGCCTCATCATTGTCTCTTATGGTGTCATTGCCAGGGCTGTACTAAAAACTAACTCTTCAAA
AGGGCGCAGAAAAGCTTTTGGGACCTGTTCATCCCACCTCATTGTGGTTACCCTCTTTTACAGCTCAG
TCATTGCTGTCTATCTGCAGCCCAAAAATCCCTATGCCCAAGAGAGGAGCAAGTTCTTTGGTCTCTTTT
ATGCAGTGGGCACCCCCACACTCAACCCTCTTGTATATACCCTGAGGAACAAGGAGGTAAAGAGGGC
ATTCTGGAGGCTGCTGGGGAAGGATGCGGCTTCTGGAGGAAACTAA
PROT: SEQ ID NO: 63 
MVNQSTPVGFLLLGFSEHPQLEKVLFVVVLCSYLLTLLGNTLILLLSTLDPRLHSPMYFFLSNLSFLDLCFTTT
CVPQMLFNLWGPTKTISFLGCSVQLFIFMLLGTTECILLTVMAFDRYVAVCQPLHYATIIHPRLCRQLAGVA
WAIGLVQSIVQIPPTLTLPFCSHRQIDDFLCEVPSLIRLSCGDTTFNEIQLSVAGVIFLLVPLSLIIVSYGVIARA
VLKTNSSKGRRKAFGTCSSHLIVVTLFYSSVIAVYLQPKNPYAQERSKFFGLFYAVGTPTLNPLVYTLRNKEV
KRAFWRLLGKDAASGGN
**Olfr398**
   *Mus musculus*_Olfr398_1-octen-3-ol olfactory receptor (GI: 32049056)
DNA: SEQ ID NO: 64 
ATGGCTGTGACCAACCTCACATACAAACCACAGTTCCAGCTCCTCGGCTTGATGGATGGCACAGATCC
CCACCCGCTGCTGTTTCTTCTTTTCCTTAGCATTTACCTACTCAATGCCCTGGGCAATCTGAGCATGGT
GGTGCTGGTGAGGTCTGATGGGGCTCTGTGCTCCCCCATGTATTATTTCCTGGGTCACCTGAGCCTCG
TGGATGTCTGCTTTACCACAGTCACTGTCCCCAGACTGCTGGCCACCTTGCTCCATCCTGGCCAGGCC
ATATCCTTCCAGGCTTGTTTTGCCCAGATGTACTTCTTTGTAGCTCTGGGTATCACCGAGAGCTACCTT
CTGGCAGCCATGTCTTATGACCGTGCGGTGGCTGTGTGTAGACCGCTGCACTATGGTGCAGTCATGA
CACCCTGGCGCTGCTTTTTGTTGGTGGCTGCATCCTGGGCTGTGGCCCATCTGCACTCTCTTCTACATA
CACTGCTCATCTCTGCTCTCACCTACCCTCCCTCTGCCCCAGTGCGCCACTTCTTTTGTGACATGACTGT
AATGCTGAGCTTGGCAACCTCTGACACATCAGCTGCAGAGACTGCCATCTTCTCAGAGGGGCTAACA
GTGGTGTTGACCCCTCTGCTCCTCGTGTCCCTTTCCTATGCACGCATCCTTGTGGCAGTGCTTGGAATT
CGGACCACAGGAGGCCGGCACCGTGTCTTCTCCACCTGTGGGGCCCACCTGGTGGTAGTGTCACTTT
TCTTTGGCTCAGTCCTCTCCGTCTACTTCCGTCCATCATCTGCCTACTCAGCTCGTTATGACCGCATGGC
CAGTGTGGTCTATGCGGTAGTCACACCGACCTTGAACCCTTTTATCTACAGTCTTCGTAACAAAGAGG
TCAAGAGTGCCCTAAAAAGGGGTTTCAGATGGAGAGCAGCACCCCAAGATGAGTAA
PROT: SEQ ID NO: 65 
MAVTNLTYKPQFQLLGLMDGTDPHPLLFLLFLSIYLLNALGNLSMVVLVRSDGALCSPMYYFLGHLSLVDV
CFTTVTVPRLLATLLHPGQAISFQACFAQMYFFVALGITESYLLAAMSYDRAVAVCRPLHYGAVMTPWRC
FLLVAASWAVAHLHSLLHTLLISALTYPPSAPVRHFFCDMTVMLSLATSDTSAAETAIFSEGLTVVLTPLLLV
SLSYARILVAVLGIRTTGGRHRVFSTCGAHLVVVSLFFGSVLSVYFRPSSAYSARYDRMASVVYAVVTPTLN
PFIYSLRNKEVKSALKRGFRWRAAPQDE
**Olfr120**
   *Mus musculus*_Olfr120_1-octen-3-ol olfactory receptor (GI: 32060790)
DNA: SEQ ID NO: 66 
ATGAGTGTCAACTGCTCTCTGTGGCAGGAAAATAAGTTGTCTGTCAAACACTTTGCATTTGCCAAGTT
CTCTGAGGTTCCTGAAGAATGCTTCCTCCTGTTTACCCTGATTCTCCTCATGTTCTTAGTATCACTGACA
GGAAATGCACTTATAACCCTTGCCATATGCACCAGTCCAGCCCTACACACCCCCATGTACTTCTTTCTG
GCCAACTTGTCTCTCCTGGAAATTGGCTACACTTGTTCTGTCATACCGAAGATGTTGAAGAACCTTGTA
ACTGAGGCCCGAGGGATCTCTCGGGAAGGGTGTGCCACACAGATGTTTTTCTTTATATTCTTTGGTAT
AACTGAGTGTTGCCTACTGGCAGCTATGGCCTTTGACCGCTACATGGCCATATGCTCCCCACTCCACT
ATGCAACCCGAATGAGTCGTGAGGTATGTGCCCATTTGGCAATAGTTTCATGGGGAATGGGATGCAT
AGTAGGGTTGGGACAGACCAATTTTATTTTCTCCTTAAACTTCTGTGGACCCTGTGAGATAGACCACT
TCTTCTGTGACCTTCCACCTGTCCTGGCACTTGCCTGTGGAGATACATCCCAAAATGAGGCTGCAATCT
TCGTGACAGTAGTTCTCTGCATATCTAGCCCATTTTTGTTGATCATTTATTCCTATGTCAGAATTTTGTT
TGCAGTGCTGGTGATGCCTTCACCTGAGGGGCGCCATAAAGCTCTCTCCACCTGTTCCTCCCATCTACT
TGTAGTCACATTGTTCTATGGCTCAGCATCTATTACCTACTTGAGGCCCAAGTCTAGCCACTCACCAGG
AATAGATAAACTCTTGGCCCTTTTCTACACCGCGGTGACTTCCATGCTGAACCCCATCATCTATAGCTT
AAGGAACAAGGAAGTGAAGGCAGCACTGAGAAGAACTCTGAGTCTGAAGAAACCTCTGGCAATAAA
TAGGTAA
PROT: SEQ ID NO: 67 
MSVNCSLWQENKLSVKHFAFAKFSEVPEECFLLFTLILLMFLVSLTGNALITLAICTSPALHTPMYFFLANLSL
LEIGYTCSVIPKMLKNLVTEARGISREGCATQMFFFIFFGITECCLLAAMAFDRYMAICSPLHYATRMSREV
CAHLAIVSWGMGCIVGLGQTNFIFSLNFCGPCEIDHFFCDLPPVLALACGDTSQNEAAIFVTVVLCISSPFLL
IIYSYVRILFAVLVMPSPEGRHKALSTCSSHLLVVTLFYGSASITYLRPKSSHSPGIDKLLALFYTAVTSMLNPII
YSLRNKEVKAALRRTLSLKKPLAINR
**Olfr1364**
   *Mus musculus*_Olfr1364_1-octen-3-ol olfactory receptor (GI: 32078800)
DNA: SEQ ID NO: 68 
ATGATCAACCAGAGCTGCCAGGAACAGTTCATCCTGCTGGGCTTCTCAGACAGACCGAGGCTAGAAT
CCATCCTCTTTGTGTTTGTCCTCATCTTCTACCTGGTGACTTTAGTGGGCAACATCATCATTATCTTAGT
TTCCTATCTGGACCCCTGTCTTCACACCCCCATGTACTTCTTCCTCACCAACTTGTCCTTTCTTGACCTCT
GTTTCACTACCAGTTCCATCCCTCAATTGCTCTTCAACTTAGGAGGCCAGGACAAGAGCATCAGTTAC
ATTGGCTGTGCAGTCCAGCTGTTCATGTTTCTTGGCCTAGGAGGTACTGAGTGTGTTTTGCTGGCTGT
CATGGCTTACGACCGTTTCACTGCAATCTGCAAGCCCCTTCACTACTCGGTCATTATGCATTCTCAGCT
CTGCTGGACATTGGTGTCTGTAGCCTGGAGTGTGGGACTCCTCAACTCCCTGGTCATGTCTCCTGTGA
CCATGAAGCTGCCTCGATGTGGCAGATGTCAGGTGAGGCACTTCCTGTGTGAGATGCCAGCTCTGAT
AAAAATTGCCTGTGTGGACACTGTGGCTGTGGAAAGCACTGTTTTCATTTTATCGGTGATAATCGTGC
TGGTGCCTTTGACTCTCATCCTCATTTCTTACAGCTATATTGCTCTAGCAGTGATGAGGATCAAGTCGG
CCTCAGGAAGGAGGAAGGCTTTCAACACGTGTGGGTCTCACCTCACCGTAGTCTCCTTGTTTTACGGG
AATATCATCTATATGTATATGCAGCCTGGACACAAGGCTTCCCAGGACCAAGGGAAATTTCTTACCCT
CTTCTACAACTTGGTAACCCCCATGTTAAACCCTGTCATCTATACACTGAGAAACAAGGATGTAAAGG
GCGCACTGAAGAGGCTTGTGACTACGAAATAA
PROT: SEQ ID NO: 69 
MINQSCQEQFILLGFSDRPRLESILFVFVLIFYLVTLVGNIIIILVSYLDPCLHTPMYFFLTNLSFLDLCFTTSSIP
QLLFNLGGQDKSISYIGCAVQLFMFLGLGGTECVLLAVMAYDRFTAICKPLHYSVIMHSQLCWTLVSVAW
SVGLLNSLVMSPVTMKLPRCGRCQVRHFLCEMPALIKIACVDTVAVESTVFILSVIIVLVPLTLILISYSYIALA
VMRIKSASGRRKAFNTCGSHLTVVSLFYGNIIYMYMQPGHKASQDQGKFLTLFYNLVTPMLNPVIYTLRN
KDVKGALKRLVTTK
**Olfr937**
   *Mus musculus*_Olfr937_1-octen-3-ol olfactory receptor (GI: 32064616)
DNA: SEQ ID NO: 70 
ATGGCAACAGGAAACTATTGCATGTTACCTGAGTTCATTCTGACTGGGCTCTCAAAGAAGCCACAACT
CCAGATGCCTCTCTTCCTCCTCTTCCTAGGAATCTATGTGGTCACTGTAGTGGGGAATTTGGGCATGA
TTACACTGATTAAGCTCAGTTCTCACCTGCATACCCCCATGTACTATTTCCTCAGTAGTTTGTCCTTTAT
TGATCTCTGCCATTCCACTGTCATTACCCCCAAAATGTTGGTGAACTTTGTGATAGAGAAAAACATCAT
TTCCTACACTGGATGCATGGCTCAGCTCTATTTCTTTCTAATTTTTGCTATTGCAGAGTGTCATATGTTA
GCTGCAATGGCATATGATCGCTATGTTGCCATCTGTAACCCCTTGCTTTACAATGTAACCATGTCCTAT
CAGATTTACACTTCCCTGATCTTTGGAGTCTATATTATTGGTGTGGTTTGTGCATCAGCTCACACAGGC
TTCATGATCAGAATACAGTTTTGTAACTTAGAGGTGATCAACCACTATTTCTGTGATCTTCTTCCCCTG
CTGGAGCTTGCTCACTCTAGTACTTATGTTAATGAATTGTTAGTTTTATGTTTTGGTACATTTAACATTG
TTGTTCCAACCATGACTATTCTTACTTCTTACATCTTCATCATTGCCAACATCTTACGCATTCGTTCTACT
GGAGGCAGGTCCAAAGCCTTCAGTACCTGCAGCTCCCACATCTTAGCTGTTGCTGTGTTCTTTGGGTC
TGCTGCATTCATGTACCTTCAACCATCATCAGTCAGCTCCATGGACCAAGGGAAAGTGTCCTCTGTGT
TTTATACCATTGTTGTTCCCATGCTGAACCCCTTGATCTATAGTCTGAGGAATAAGGATGTCAGTGTTG
CCCTGAAGAAAATACTAGAAAGAAAATTATTCATGTAA
PROT: SEQ ID NO: 71 
MATGNYCMLPEFILTGLSKKPQLQMPLFLLFLGIYVVTVVGNLGMITLIKLSSHLHTPMYYFLSSLSFIDLCH
STVITPKMLVNFVIEKNIISYTGCMAQLYFFLIFAIAECHMLAAMAYDRYVAICNPLLYNVTMSYQIYTSLIF
GVYIIGVVCASAHTGFMIRIQFCNLEVINHYFCDLLPLLELAHSSTYVNELLVLCFGTFNIVVPTMTILTSYIFII
ANILRIRSTGGRSKAFSTCSSHILAVAVFFGSAAFMYLQPSSVSSMDQGKVSSVFYTIVVPMLNPLIYSLRNK
DVSVALKKILERKLFM
**Olfr1322**
   *Mus musculus_Olfr1322_geonol* olfactory receptor (GI: 46404727)
DNA: SEQ ID NO: 72 
ATGACTCAAATTTCAGAAATTATTCTTTTGGGTTTTGGAGATCTCCATGGCCTTCAGTTTCTTCTTTTTG
GGCTATTTCTAGCCATTTATGTTATGACTCTCCTGGGTAACATTGTAATTCTCACCGTAGTATCCACTG
ATTGCTCCCTTCACACACCCATGTATTTCTTTCTTGGCCACTTCTCCTTCCTAGAGATCAGCTACACCAC
AACAATTGAGCCTGTAATGCTGTGGACATTGTTATCGGCTCATGTGCCTATCTCCTTGCCAGCCTGTG
CTTGTCAGTTTTACTTTTTTGCATCTTTGGTGGCAACAGAATGCTTCCTCCTTGCTGTAATGTCTTATGA
TAGATACATAGCCATTTGTAACCCACTACACTATTCCAGTATCATGGACTCCTGGGGTTGTTTCCAGCT
AGCATTGGCCTCTTGGTTGGCTGGATTTCTGGCACCTATCTTACTCATGATCTTGATTTTTCGCTTAAC
ATTTTGTTCTGCCAATGAGATTGACCACTTCTTCTGTGATTTGAAGCCCATCATGAAACTGGCCTGCAC
TAATACTCAAGTAGCTGAAATGACTTCTTTTATATGTACCTCATTATTCGCTCTTGGTCCCTTCATACTG
ACCTTGGCTTCCTATATTCACATCATTTGCACCATTCTGAGGATCCCTTCGACCACAGGGAAGCAGAG
GGCTTTCTCCACCTGTTCATCTCACCTTATAGTGGTCAGTCTTTATTATGGGACTCTGGGCATTGTCTAT
GGCTTCCCATCAATGCCTCAATATGAAAGTATATTGAAGTTGCTTTCTCTTCTGTATACAGTGTTTACC
CCTGCTGCCAATCCTATCATTTACACCCTAAGGAACAAGGATGTGAAGGTAGCTCTGAGAAAATTGAC
ACAATGGCATACATACCTGGTCAAAGAAGGCTAA
PROT: SEQ ID NO: 73 
MTQISEIILLGFGDLHGLQFLLFGLFLAIYVMTLLGNIVILTVVSTDCSLHTPMYFFLGHFSFLEISYTTTIEPV
MLWTLLSAHVPISLPACACQFYFFASLVATECFLLAVMSYDRYIAICNPLHYSSIMDSWGCFQLALASWLA
GFLAPILLMILIFRLTFCSANEIDHFFCDLKPIMKLACTNTQVAEMTSFICTSLFALGPFILTLASYIHIICTILRIP
STTGKQRAFSTCSSHLIVVSLYYGTLGIVYGFPSMPQYESILKLLSLLYTVFTPAANPIIYTLRNKDVKVALRKL
TQWHTYLVKEG
**Olfr46**
   *Mus musculus_Olfr46_transpirol* olfactory receptor (GI: 32054916)
DNA: SEQ ID NO: 74 
ATGGTCAGTCCCAACCAGACAGTAGTGACAGAGTTTGTGCTGCAAGGGTTCTCAGAGCACCCAAGTC
TAAGACTGTTCCTGATGGGCTGTTTCCTGTCCCTGTACACAGTGGCTTTAATGGGCAACATGGTGATC
ATTGCTTTGATCACCTCAAGCACTGGGCTCCACAGTCCCATGTACTTTTTCCTGTGCAACTTAGCGACC
ATGGATATTATCTGCACCTCCTCTGTACTGCCCAAGGCGCTGGTTGGTCTACTGTCTGAGGAAAACAC
CATCTCCTTCAAAGGGTGCATGACTCAGCTCTTCTTTCTTGTGTGGTCTGGATCCTCTGAGCTGCTGCT
GCTCACAGTCATGGCCTATGACCGCTATGTGGCCATCTGTTTGCCCCTGCATTACAGCTCTAGGATGA
GTCCACAGCTCTGTGGGACCTTTGCCGTGGGTGTATGGTCCATCTGCGCACTAAATGCATCTATCAAC
ACTGGTCTGATGACACGGCTGTCATTCTGTGGCCCCAAGGTCATCACCCACTTCTTCTGTGAGATTCCC
CCACTCCTCCTGCTCTCCTGTAGTCCTACATATATAAATAGCGTTATGACTCTTGTGGCAGATGCCTTTT
ATGGAGGCATCAATTTTTTACTTACCTTGCTATCCTATGGCTGCATCATTGCCAGCATCCTGCGCATGC
GTTCTGCTGAGGGCAAGAGGAAGGCCTTTTCTACCTGCTCATCCCACCTCATCGTGGTCTCTGTGTAC
TACTCATCTGTGTTCTGTGCCTATATCAGTCCTGGTTCCAGCTACAGCCCAGAAAGAAGCAAATTTACC
TCGGTTTTGTACTCAGTCCTCAGCCCAACCCTCAACCCCCTCATCTATACACTGAGGAACAAGGATGTC
AAGCTTGCCCTGAGAAGACTCTTCCCCTCTTTCTCAAATTAA
PROT: 75

MVSPNQTVVTEFVLQGFSEHPSLRLFLMGCFLSLYTVALMGNMVIIALITSSTGLHSPMYFFLCNLATMDII
CTSSVLPKALVGLLSEENTISFKGCMTQLFFLVWSGSSELLLLTVMAYDRYVAICLPLHYSSRMSPQLCGTF
AVGVWSICALNASINTGLMTRLSFCGPKVITHFFCEIPPLLLLSCSPTYINSVMTLVADAFYGGINFLLTLLSY
GCIIASILRMRSAEGKRKAFSTCSSHLIVVSVYYSSVFCAYISPGSSYSPERSKFTSVLYSVLSPTLNPLIYTLRNK
DVKLALRRLFPSFSN

The following examples are illustrative only and are not meant to limit the scope of invention as set forth in the Summary, Description or in the Claims.

The numerous possible variations that will become immediately evident to a person skilled in the art after heaving considered the disclosure provided herein also fall within the scope of the invention.

All materials and microorganisms or cells employed are commercially available products.

Unless otherwise specified, recombinant proteins are cloned and expressed by standard methods, such as, for example, as described by Sambrook, J., Fritsch, E.F. and Maniatis, T., Molecular cloning: A Laboratory Manual, 2nd Edition, Cold Spring Harbor Laboratory, Cold Spring Harbor Laboratory Press, Cold Spring Harbor, NY, 1989.

### EXAMPLES

### Example 1: Identification and Functional Characterization of Mouse and Human Odorant Receptors Activated by Geonol, DMTS, 1-octen-3-ol, Butyric Acid, 3-methyl-2-hexenoic acid, 3-hydroxy-3-methyl-hexanoic acid and Transpirol

The identification of new odorant receptors was performed according to the method disclosed in WO2014/210585. Briefly, murine olfactory sensory neurons were exposed to the following MCS's: geonol, DMTS, 1-octen-3-ol, butyric acid, 3-methyl-2-hexenoic acid, 3-hydroxy-3-methyl-hexanoic acid, or transpirol and screened using a Ca²⁺ imaging technique. Neurons that were activated by DMTS were further isolated for full transcriptome analysis to identify the responsive odorant receptor. The cDNA corresponding to the isolated cell mRNA was generated and amplified by PCR based method (Clontech/Takara, SMARTer^{®} Ultra^{®} Low Input RNA Kit for Sequencing - v3, cat. 634848). Amplified cDNA was then used to generate an Illumina cDNA library for Next-Generation-Sequencing and the generation of 100 base pair single read sequences. Sequences were aligned to a mouse reference genome (such as UCSC version mm10) in order to generate the full transcriptome. Because only a single odorant receptor (OR) is strongly transcribed per olfactory sensory neuron, the subsequent identification of the MCS responsive OR can be achieved. Phylogenetic relationship evaluation using sequence similarity searches were then used to identify the corresponding human OR. The following ORs were identified Olfr263, Olfr96, Olfr1487, Olfr339, Olfr641, Olfr1126, Olfr46, Olfr1322, Olfr93, Olfr398, Olfr120, Olfr1364 and Olfr937.

In the second method, a panel of odorant receptors comprising the 400 putatively functional human receptor repertoire were transfected into a cultured human HEK293T cell line described in WO2014/210585 expressing Human olfactory G-protein alpha subunit (Golf - SEQ. ID No. 49) and coupled to a cyclicAMP reporter. Said panel of *in vitro* odorant receptors were exposed to MCSs and the level of cAMP signal was measured for each receptor. Receptors showing a signal significantly different from baseline were subsequently tested in functional dose-response experiments. Referring to Figure 1, the following human odorant receptors were identified: OR11A1, OR2M3, OR4S2, OR2J3, OR4Q3, OR51E1, OR2W1, OR1A1, OR5K1, and OR51I2.

Functional dose-response experiments were performed to evaluate the level of MCS-induced activity of the modified cell line expressing individual putative odorant receptors, according to the assays and methods disclosed in WO2014/210585, using either geonol, DMTS, 1-octen-3-ol, butyric acid, 3-methyl-2-hexenoic acid, 3-hydroxy-3-methyl-hexanoic acid, or transpirol.

### Example 2: Identification of Butyric Acid Olfactory Receptor Inhibitors

A cell line expressing the OR51E1 olfactory receptor was used as an antagonist screening platform to identify compounds that have the property to decrease the butyric acid induced receptor activity. The cell line was screened with a volatile compound library for their inhibitory properties and potential butyric acid smell inhibition. First, individual binary mixtures of butyric acid with each one of the test compounds were presented to the cells.

Single concentration monitoring of the butyric acid induced cell activity in the presence or absence of a test compound allowed for the identification of compounds with a putative suppression or inhibitory effect. These hits were further confirmed in an inhibitory dose-response assay to evaluate the % inhibition normalized to the baseline agonist activity.

A dose-dependent decrease of receptor activity was recorded with increasing concentrations of test compounds in the presence of a single activating concentration of butyric acid (EC₈₇) and corresponding dose-response inhibition curves were obtained. A summary of the maximum % inhibition for the compounds tested is shown in Figure 2.

The assay system was designed to select for highly selective antagonists, in that the data were obtained using a high butyric acid concentrations (EC₈₇). These data demonstrate that the compounds of Formula (I) are butyric acid olfactory receptor antagonists. However, some compounds performed better than others, as suggested by the relative magnitudes of the inhibition of the butyric acid olfactory receptor.

### Example 3: Identification of Geonol Olfactory Receptor Inhibitors

Cell-Based Screening: Cell lines expressing either the OR11A1, or the OR2M3 olfactory receptor were used as antagonist screening platforms to identify compounds that have the property to decrease the geonol induced receptor activity. Each cell line was screened with a volatile compound library for their inhibitory properties and potential geonol smell inhibition. First, individual binary mixtures of geonol with each one of the test compounds were presented to the cells.

Single point monitoring of the geonol induced cell activity in the presence or absence of a test compound allowed for the identification of compounds with a putative suppression or inhibitory effect. These hits were further confirmed in an inhibitory dose-response curve assay to evaluate the potency of activity inhibition as a measure of the IC₅₀ (the inhibitor concentration at which the receptor activity is inhibited by the half-maximal inhibition efficacy level of a given test compound).

A dose-dependent decrease of receptor activity was recorded with increasing concentrations of test compounds in the presence of a single activating concentration of geonol (EC₈₀) and corresponding dose-response inhibition curves were obtained. The compounds listed in Table 2 are examples of compounds that decreased the geonol induced activity of at least one olfactory receptor.

Sensory Evaluation: Five 16oz glass jars containing either REF (geonol only), geonol only control, antagonist only, mixture of geonol and isointense antagonist, or mixture of geonol and low intensity antagonist were presented to the panelists (last four samples were randomized across panelists). The panelists were asked to familiarize themselves with the earthy/moss odor quality from the REF jar first, then rate the pleasantness, earthy/moss odor intensity and overall odor intensity of the next four samples on a 0-10 linear scale. The inter-stimulus interval between the samples was two minutes to avoid adaptation. The results are superimposed in Figure 4, wherein the figure shows the percentage of geonol malodor (earthy/moss odor) remaining in binary iso-intense mixtures of geonol and 25 different antagonists to REF sample.

A one-way Analysis of Variance (ANOVA) with Duncan mean comparison at 95% confidence level was done to compare the average earthy/moss odor intensity among geonol only sample, antagonist only sample, and mixtures of Geonol reference/antagonist at two different ratios. The results from the cell-based screening and the sensory evaluation are shown in Table 2 and Figure 4.
* means that no significant difference of earthy/moss odor intensity was found between the iso-intense mixture and antagonist, and it was significantly lower than geonol only sample at 95% confidence level.
* means that the earthy/moss odor intensity was significantly higher in iso-intense mixture than in antagonist only sample, and it was significantly lower than geonol only sample at 95% confidence level.
NS means that no significant difference of earthy/moss odor intensity was found between the iso-intense mixture and antagonist, and no significant difference was found between the iso-intense mixture and geonol only sample at 95% confidence level.
NS means that the earthy/moss odor intensity was significantly higher in iso-intense mixture than in antagonist only sample, and no significant difference was found between the iso-intense mixture and in geonol only sample at 95% confidence level.

**Table 2: Geonol Olfactory Receptor Antagonists**

| Compound | IC₅₀ | %Inhibition | % Remaining in human sensory tests |
|---|---|---|---|
| 2-tertbutyl phenol | 88 | 100 | 46* |
| thymol | 107 | 100 | 53 |
| nona-2,6-dienal | 156 | 80 | 67 |
| 1,1-diethoxy-3,7-dimethylocta-2,6-diene | 371 | 55 | 51* |
| [1IR-(1R*,4R*,6R*,10S*)]-4,12,12-trimethyl-9-methylene-5-oxatricyclo[8.2.0.0^{4,6}]dodecane | 1233 | 50 | NT |
| 3-(4-hydroxy-4-methylpentyl)cyclohex-3-ene-1-carbaldehyde;4-(4-hydroxy-4-methylpentyl)cyclohex-3-ene-1-carbaldehyde | 217 | 60 | NT |
| 1,4,8-trimethyl-13-oxabicyclo[10.1.0]trideca-4,8-diene;1,5,8-trimethyl-13-oxabicyclo[10.1.0]trideca-4,8-diene | 81 | 50 | 85 |
| 3-(4-tert-butylphenyl)propanal | 316 | 50 | NT |
| (4R,4aS,6R)-4,4a-dimethyl-6-prop-1-en-2-yl-3,4,5,6,7,8-hexahydronaphthalen-2-one | 486 | 50 | NT |
| 6,10-dimethylundeca-5,9-dien-2-one | 133 | 50 | 45* |
| 1-(3,3-dimethylcyclohexen-1-yl)pent-4-en-1-one;1-(5,5-dimethylcyclohexen-1-yl)pent-4-en-1-one | 111 | 45 | 58* |
| 1-(3,3-dimethylcyclohexen-1-yl)pent-4-en-1-one;1-(5,5-dimethylcyclohexen-1-yl)pent-4-en-1-one | 148 | 45 | NT |
| 2-ethoxy-5-prop-1-enylphenol | 111 | 33 | 34* |
| 1-(2,6,6-trimethyl-1-cyclohex-3-enyl)but-2-en-1-one | 301 | 10 | 66* |
| 2-ethoxy-5-prop-1-enylphenol, 8,9-epoxy-2,6,6,8-tetramethyl-tricyclo[5.3.1.0(1,5)]undecane | 301 | 30 | NT |
| 1-(4,8-dimethyl-12-methylidenecyclododeca-4,8-dien-1-yl)ethanone;1-(2,6,10-trimethylcyclododeca-1,5,9-trien-1-yl)ethanone;1-(2,6,10-trimethylcyclododeca-2,5,9-trien-1-yl)ethanone | 301 | 20 | NT |
| nonylenic aldehyde | 151 | 30 | NT |
| isobutylquinoline | 109 | 45 | 52* |
| cyclopentadec-4-en-1-one | 61 | 45 | 47 |
| (-)-(R)-3,7-dimethyl-6-octenenitrile | 300 | 10 | NT |
| 2-methyl-5-propan-2-ylphenol | 215 | | 27** |
| isopropyl quinoline | 400 | | 110 |
| 5-hexyl-2-methylpyridine 10% Citr | 555 | | 37 |
| (2,5-dimethyl-1,3-dihydroinden-2-yl)methanol | | | 37* |
| 2-pentylcyclopentan-1-one | | | 78 |
| 4-(2,6,6-trimethylcyclohexen-1-yl)but-3-en-2-one;4-(2,6,6-trimethylcyclohex-2-en-1-yl)but-3-en-2-one | | | 59* |
| 3-methyl-4-(2,6,6-trimethylcyclohex-2-en-1-yl)but-3-en-2-one;1-(2,6,6-trimethylcyclohex-2-en-1-yl)pent-1-en-3-one | | | 38* |
| 2,4-dimethylcyclohex-3-enecarbaldehyde | | | 45* |
| phenylethylol | | | 58 |
| 3,7-dimethylocta-2,6-dienal | | | 42* |
| (2,2,2-trichloro-1-phenylethyl) acetate crist | | | 61 |
| 4-(2,6,6-trimethylcyclohexen-1-yl)but-3-en-2-one | | | 43* |
| phenyl ethyl acetate | | | 47* |
| 7-hydroxy-3,7-dimethyloctanal | | | 39* |

### Example 4: Identification of 1-octen-3-ol Olfactory Receptor Inhibitors

Cell lines expressing either the OR2W1 or the OR1A1 olfactory receptor were used as antagonist screening platforms to identify compounds that have the property to decrease the 1-octen-3-ol induced receptor activity. Each cell line was screened with a volatile compound library for their inhibitory properties and potential 1-octen-3-ol smell inhibition. First, individual binary mixtures of 1-octen-3-ol with each one of the test compounds were presented to the cells.

Single concentration monitoring of the 1-octen-3-ol induced cell activity in the presence or absence of a test compound allowed for the identification of compounds with a putative suppression or inhibitory effect. These hits were further confirmed in an inhibitory dose-response assay to evaluate the potency of activity inhibition and efficacy as a measure of the IC₅₀ (the inhibitor concentration at which the receptor activity is inhibited by the half-maximal inhibition efficacy level of a given test compound) and % inhibition (normalized to baseline agonist activity) accordingly.

A dose-dependent decrease of receptor activity was recorded with increasing concentrations of test compounds in the presence of a single activating concentration of 1-octen-3-ol (EC₈₀) and corresponding dose-response inhibition curves were obtained. The compounds listed in Table 3 are examples of compounds that decreased the 1-octen-3-ol induced activity of at least one receptor.

**Table 3: 1-octen-3-ol Olfactory Receptor Antagonists**

| Compound | IC₅₀ (µM) | % Inhibition |
|---|---|---|
| Cyclododecanone | 8.79 | 118.47 |
| 2,4-dimethylcyclohex-3-enecarbaldehyde | 18.76 | 107.99 |
| 1,4-dioxacyclohexadecane-5,16-dione | 24.25 | 116.54 |
| [1R-(1R*,4R*,6R*,10S*)]-4,12,12-trimethyl-9-methylene-5-oxatricyclo[8.2.0.0^{4,6}]dodecane | 25.11 | 123.63 |
| Pentanol, 1- | 28.32 | 94.31 |
| OXACYCLOHEXADECAN-2-ONE | 32.15 | 111.31 |
| tert-butylphenol, 2- | 36.95 | 100.34 |
| (+-)-3-methylcyclopentadecanone | 43.96342 | 113.50 |
| 1,1,2,3,3-pentamethyl-2,5,6,7-tetrahydroinden-4-one | 51.185156 | 118.44 |
| (4-methyl-4-phenylpentan-2-yl) acetate | 61.70 | 98.90 |
| (+-)-perhydro-5,5,8A-trimethyl-2-trans-naphthalenone | 63.22 | 102.97 |
| 2,3,3-trimethyl-2H-inden-1-one | 75.60 | 128.39 |
| 1-(4,8-dimethyl-12-methylidenecyclododeca-4,8-dien-1-yl)ethanone; 1-(2,6,10-trimethylcyclododeca-1,5,9-trien-1-yl)ethanone; 1-(2,6,10-trimethylcyclododeca-2,5,9-trien-1-yl)ethanone | 82.36 | 113.23 |
| 4-(4,8-dimethylnona-3,7-dienyl)pyridine | 134.31 | 111.87 |
| Cyclopentadec-4-en-1-one | 155.72 | 90.98 |
| 4-[(1~{R},3~{R},6~{R})-2,2,3,6-tetramethylcyclohexyl]but-3-en-2-one;4-[(1~{S},3~{S},6~{R})-2,2,3,6-tetramethylcyclohexyl]but-3-en-2-one | 186.12 | 90.82 |
| (+)-(1R,7R)-10,10-dimethyl-tricyclo[7.1.1.0(2,7)]undec-2-en-4-one 90 DIPG | 193.99 | 128.51 |
| (9<I>E</I>)-cycloheptadec-9-en-1-one | 210.10 | 97.22 |
| 1,4,8-trimethyl-13-oxabicyclo[10.1.0]trideca-4,8-diene;1,5,8-trimethyl-13-oxabicyclo[10.1.0]trideca-4,8-diene | 266.47 | 70.92 |

| Compound | IC₅₀(µM) | % Inhibition |
|---|---|---|
| 8,8-dimethyl-2,3,4,6,7,8~{a}-hexahydro-1~{H}-naphthalene-2-carbaldehyde;8,8-dimethyl-2,3,5,6,7,8~ { a }-hexahydro-1~{H}-naphthalene-2-carbaldehyde;8,8-dimethyl-2,3,4,5,6,7-hexahydro-1~{H}-naphthalene-2-carbaldehyde;2,2-dimethyl-octahydro-1~{H}-2,4~{a}-methanonapthalen-8-one | 1641.48 | 83.15 |
| 2,4~{a},8,8-tetramethyl-1~{a},2,4,5,6,7-hexahydro-1~{H}-cyclopropa[j]naphthalen-3-one;2,6,6,8-tetramethyltricyclo[5.3.1.0^{1,5}]undecan-9-one | 2362.92 | 109.43 |
| Isobutylquinoline | > 600 | 30.14 |

### Example 5: Identification of Butyric Acid Olfactory Receptor Inhibitors

A cell line expressing the OR51E1 olfactory receptor was used as an antagonist screening platform to identify compounds that have the property to decrease the butyric acid induced receptor activity. The stable cell line was screened with a volatile compound library for their inhibitory properties and potential butyric acid smell inhibition. First, individual binary mixtures of butyric acid with each one of the test compounds were presented to the cells.

Single concentration monitoring of the butyric acid induced cell activity in the presence or absence of a test compound allowed for the identification of compounds with a putative suppression or inhibitory effect. These hits were further confirmed in an inhibitory dose-response assay to evaluate the potency of activity inhibition as a measure of the IC₅₀ (the inhibitor concentration at which the receptor activity is inhibited by the half-maximal inhibition efficacy level of a given test compound).

A dose-dependent decrease of receptor activity was recorded with increasing concentrations of test compounds in the presence of a single activating concentration of butyric acid (EC₉₄) and corresponding dose-response inhibition curves were obtained. The compounds listed in Table 4 are examples of compounds that decreased the butyric acid induced activity of at least one receptor.

**Table 4: Butyric Acid Olfactory Receptor Antagonists**

| IUPAC Name | IC50 | Inhibition (%) | Sensory Performance (%) |
|---|---|---|---|
| (2,3,9,9-tetramethylspiro[4.5]dec-2-en-10-yl) acetate | >600 | 116 | 40% |
| (1,5,10,10-tetramethylspiro[5.5]undec-3-en-11-yl) acetate | 72.33 | 72 | 43% |
| (2,3,9,9-tetramethylspiro [4.5]decan-10-yl) acetate | 51.40 | 62 | 50% |
| (5,10,10-trimethylspiro[5.5]undec-2-en-11-yl)formate | 44.37 | 129 | |
| (3,5,10,10-tetramethylspiro[5.5]undec-2-en-11-yl) acetate | 71.39 | 104 | |
| (9,9-dimethylspiro [4.5] dec-2-en-10-yl) acetate | 90.30 | 32 | |
| 10-methoxy-9,9-dimethylspiro[4.5]dec-2-ene | 119.54 | 33 | |
| 4,10,10,11-tetramethylspiro[5.5]undec-2-en-11-ol, 5,10,10,11-tetramethylspiro[5.5]undec-3-en-11-ol, or mixtures thereof | 47.23 | 94 | |
| 2,4,8-trimethylspiro[5.5]undec-3-en-11-ol, 2,9,11-trimethylspiro[5.5]undec-9-en-5-ol, or mixtures thereof | 117.03 | 70 | |
| 3-methyl-5-propan-2-ylspiro[5.5]undec-2-en-11-ol | 366.37 | 144 | |
| 11-methylspiro[5.5]undecan-5-ol | 68.82 | 52 | |
| 3,10,10-trimethylspiro[5.5]undec-3-en-11-ol, 4,10,10-trimethylspiro[5.5]undec-3-en-11-ol, or mixtures thereof | >600 | 121 | |
| [2,2-dimethyl-1-(2,4,6-trimethylcyclohex-3-en-1-yl)propyl] acetate | 52.56 | 89 | |
| [2,2-dimethyl-1-(2,4,6-trimethylcyclohexyl)propyl] acetate | 241.99 | 101 | |

| IUPAC Name | IC50 | Inhibition | Sensory Performance (%) |
|---|---|---|---|
| [(1~{R})-2,2-dimethyl-1-[(1~{S},2~{S})-2-methylcyclohexyl]propyl] acetate | 169.82 | 86 | |
| 2,3,9,9-tetramethylspiro[4.5]dec-2-en-10-ol | 78.69 | 16 | 60% |
| 2,3,9,9-tetramethylspiro[4.5]dec-2-en-10-one | | | 56% |
| 2,2-dimethyl-6,6-bis(2-methylprop-2-enyl)cyclohexan-1-one | 46.59 | 103 | 50% |
| (2,3,9,9-tetramethylspiro[4.5]dec-2-en-10-yl) formate | 25.90 | 69 | |
| (2,4,9,9-tetramethylspiro[4.5]dec-2-en-10-yl) acetate | 34.79 | 131 | |
| 2,2-dimethyl-5,5-bis(2-methylprop-2-enyl)cyclopentan-1-ol | 35.11 | 119 | |
| 9,9-dimethylspiro[4.5]dec-2-en-10-ol | 44.06 | 91 | |
| (4,9,9-trimethylspiro[4.5]dec-2-en-10-yl) acetate | 48.52 | 106 | |
| 2,2-dimethyl-5,5-bis(2-methylprop-2-enyl)cyclopentan-1-one | 51.71 | 82 | |
| 1,1',1',5-tetramethyl-2'-methylidenespiro[6-oxabicyclo[3.1.0]hexane-3,3'-cyclohexane] | 75.67 | 37 | |
| 2-but-3-en-2-yl-6,6-dimethyl-2-prop-2-enylcyclohexan-1-one | 76.42 | 115 | |
| (4~{S},5~{R},6~{S},11~{S})-4,11-dimethylspiro[5.5]undecan-5-ol | 76.93 | 54 | |
| 2,4,9,9-tetramethylspiro[4.5]dec-2-en-10-ol | 81.58 | 22 | |
| 2,2-dimethyl-1-(2-methylcyclohexyl)propan-1-ol, 2,2-dimethyl-1-(3-methylcyclohexyl)propan-1-ol, or mixtures thereof | 83.27 | 17 | |
| 1-methoxy-2,6-dimethyl-1-prop-2-enylcyclohexane | 100.21 | 61 | |
| 2,4,9,9-tetramethylspiro[4.5]dec-2-en-10-one | 109.82 | 63 | |
| 2,3,9,9-tetramethylspiro[4.5]decan-10-ol | 119.78 | 12 | |
| 2,2-dimethyl-6,6-bis(2-methylprop-2-enyl)cyclohexan-1-ol | 124.71 | 110 | |
| 9,9-dimethylspiro[4.5]dec-2-en-10-one | 135.02 | 23 | |
| 2,3,8,8-tetramethylspiro[4.4]non-2-en-9-one | 198.38 | 10 | |
| 9-methoxy-8,8-dimethylspiro[4.4]non-2-ene | 237.96 | 100 | |
| 4,9,9-trimethylspiro[4.5]dec-2-en-10-one | 267.67 | 102 | |
| 1-(2,6-dimethylcyclohex-3-en-1-yl)-2,2-dimethylpropan-1-ol | 321.14 | 31 | |
| 4-methoxy-3,3-dimethylspiro[4.4]nonane | 337.13 | 105 | |
| (2,2,3,3,9,9-hexamethylspiro[4.5]decan-10-yl) acetate | 424.91 | 39 | |
| (8,8-dimethylspiro[4.4]non-2-en-9-yl) acetate | 488.31 | 107 | |
| methyl 1-(3-methylbut-2-enyl)cyclohex-2-ene-1-carboxylate, methyl 1-(3-methylbut-2-enyl)cyclohex-3-ene-1-carboxylate, or mixtures thereof | 576.63 | 38 | |

**Table 5: Butyric Acid Olfactory Receptor Antagonists**

| IUPAC Name | IC50 | Inhibition (%) |
|---|---|---|
| 1-(2-methyl-4-tricyclo[5.2.1.0^{1,5}]decanyl)ethyl acetate | 97.01 | 23 |
| 1-(2,4-dimethyl-4-tricyclo[5.2.1.0^{1,5}]decanyl)ethyl acetate | 54.79 | 34 |
| 1-[(1-{S},2~{S},4~{S},7-{R})-2,3,4-trimethyl-4-tricyclo[5.2.1.0^{1,5}]decanyl]ethanone | 32.58 | 27 |
| 1-(2,4-dimethyl-4-tricyclo[5.2.1.0^{1,5}]decanyl)ethanone | 60.53 | 26 |
| 2,3,4-trimethyltricyclo[5.2.1.0^{1,5}]decane-4-carbaldehyde | 269.71 | 28 |
| (2,3,4-trimethyl-4-tricyclo[5.2.1.0^{1,5}]decanyl)methyl acetate | 60.80 | 27 |
| ~{N}-[[(1~{S},7~{R})-2,4-dimethyl-4-tricyclo[5.2.1.0^{1,5}]decanyl]methylidene]hydroxylamine | 88.55 | 38 |
| -{N}-[1-[(1-{R},7-{R})-2,3,4-trimethyl-4-tricyclo[5.2.1.0^{1,5}]decanyl]ethylidene]hydroxylamine | 79.71 | 81 |
| -{N}-[1-[(1-{S},7-{R})-2,3-dimethyl-4-tricyclo[5.2.1.0^{1,5}]decanyl]ethylidene]hydroxylamine | 76.58 | 19 |

### Example 6: Identification of DMTS Olfactory Receptor Inhibitors

Cell lines expressing either the OR52N5, OR2L13, OR2AJ1, OR4C15, OR5AC2, OR8H3, OR11G2, OR52N2, or OR5T1 odorant receptors were used as an antagonist screening platform to identify compounds that have the property to decrease the DMTS induced receptor activity, according to the assays and methods disclosed in WO2014/210585. Each cell line expressing a human odorant receptor was screened with a volatile compound library for their inhibitory properties and potential DMTS smell inhibition. First, individual binary mixtures of DMTS with each one of the test compounds were presented to the cells. Single point monitoring of the DMTS induced cell activity in the presence or absence of a test compound allowed for the identification of compounds with a putative suppression or inhibitory effect. These hits were further confirmed in an inhibitory dose-response curve assay to evaluate the potency of activity inhibition as a measure of the IC₅₀ (the inhibitor concentration at which the receptor activity is inhibited by the half-maximal inhibition efficacy level of a given test compound). A dose-dependent decrease of receptor activity was recorded with increasing concentrations of test compounds in the presence of a single activating concentration of DMTS (EC₈₀) and corresponding dose-response inhibition curves were obtained. The compounds recited in Table 5 are examples of compounds that decreased the DMTS induced activity of at least one receptor.

**Table 6: DMTS Olfactory Receptor Antagonists**

| |
|---|
| 1,2,3,4,5,6,7,8-octahydro-8,8-dimethyl-2-naphthalenecarbaldehyde (A) + (B,C,D) + octahydro-5,5-dimethyl-2-naphthalenecarbaldehyde |
| (+-)-perhydro-4alpha,8abeta-dimethyl-4a-naphthalenol |
| 3-(3,3-dimethyl-2,3-dihydro-1H-inden-5-yl)propanal (A) + 3-(1,1-dimethyl-2,3-dihydro-1H-inden-4-yl)propanal (B) + 3-(1,1-dimethyl-2,3-dihydro-1H-inden-5-yl)propanal (C) |
| (+-)-2,5-dimethyl-2-indanmethanol |
| (-)-(3R,6S,8S)-2,2,6,8-tetramethyltricyclo[5.3.1.0~3,8~]undecan-3-ol |
| patchouli oil |
| (+-)-2,2,2-trichloro-1-phenylethyl acetate |
| 5RS,6RS)-2,6,10,10-tetramethyl-1-oxaspiro[4.5]decan-6-ol |
| 2,2,7,7-tetramethyltricyclo[6.2.1.0-1,6~]undecan-6-ol |
| (+)-(1S,2S,3S,5R)-2,6,6-trimethylspiro[bicyclo[3.1.1]heptane-3,1'-cyclohexane]-2'-en-4'-one |

## Claims

1. A method for identifying a compound that binds, suppresses, blocks, inhibits, and/or modulates the activity of an olfactory receptor that is activated by a laundry malodor causing substance, a moldy malodour causing substance and/or a sweat malodor-causing substance comprising:
a. contacting a test substance and said laundry malodor and/or sweat malodor-causing substance to at least one olfactory receptor polypeptide selected from the group consisting of OR2W1, OR1A1, OR2J3, OR4Q3, OR5K1, OR11A1, OR2M3, OR51E1, OR4S2, OR51I2, OR2H1, OR2W3, OR8G1, Olfr263, Olfr403, Olfr735, Olfr96, Olfr1193, Olfr641 Olfr137, Olfr173, Olfr164, Olfr1487, Olfr339, Olfr1126 and Olfr558, Olfr1322, Olfr93, Olfr398, Olfr120, Olfr1364, Olfr937, Olfr46, and chimera or functional fragments thereof; or to at least one olfactory receptor polypeptide having at least 70% amino acid sequence identity to at least one of the aforementioned olfactory receptor polypeptides; and
b. measuring the response of said at least one olfactory receptor to the malodor-causing substance by measuring the response of said olfactory receptor in the presence and absence of the test substance.

2. The method of claim 1, further comprising
c. identifying a test substance that modulates the response of said at least one olfactory receptor on the basis of the response that was measured in the presence and absence of the test substance; and
d. selecting the identified test substance as a compound that modulates the response of said at least one olfactory receptor to the malodor-causing substance.

3. The method of claim 1 or 2, wherein said malodor-causing substance is selected from the group consisting of geonol, dimethyl trisulfide (DMTS), 1-octen-3-ol, butyric acid, 3-methyl-2-hexenoic acid, 3-hydroxy-3-methyl-hexanoic acid, 3-methyl-3-sulphanyl hexanol (transpirol), and mixtures of at least two of said substances.

4. The method of anyone of the preceding claims, wherein said at least one olfactory receptor polypeptide
a. comprises an amino acid sequence having at least 70% sequence identity to SEQ ID NO: 2, 4, 6, 8, 10, 12, 14, 16, 18, 20, 22, 24, 26, 28, 30, 32, 34, 36, 38, 40, 42, 44, 46, 57, 59, 61, 63, 65, 67, 69, 71, 73, or 75; and/or
b. is encoded by a nucleic acid molecule comprising a nucleotide sequence having at least 70% sequence identity to SEQ ID NO: 1, 3, 5, 7, 9, 11, 13, 15, 17, 19, 21, 23, 25, 27, 29, 31, 33, 35, 37, 39, 41, 43, 45, 56, 58, 60, 62, 64, 66, 68, 70, 72, 74, or the reverse complement thereof.

5. The method of claim 4, wherein the polypeptide
a. comprises an amino acid sequence having at least 90%, 95%, 98%, 99% or 100% sequence identity to SEQ ID NO: 2, 4, 6, 8, 10, 12, 14, 16, 18, 20, 22, 24, 26, 28, 30, 32, 34, 36, 38, 40, 42, 44, 46, 57, 59, 61, 63, 65, 67, 69, 71, 73, or 75; and/or
b. is encoded by a nucleic acid molecule comprising a nucleotide sequence having at least 90%, 95%, 98%, 99% or 100% sequence identity to SEQ ID NO: 1, 3, 5, 7, 9, 11, 13, 15, 17, 19, 21, 23, 25, 27, 29, 31, 33, 35, 37, 39, 41, 43, 45, 56, 58, 60, 62, 64, 66, 68, 70, 72, 74, or the reverse complement thereof.

6. The method of any one of the claims 1 to 5, wherein said malodor-causing substance is a laundry malodor causing substance selected from the group consisting of geonol, DMTS, 1-octen-3-ol, and mixtures of at least two of said substances.

7. The method of any one of the embodiments 1 to 5, wherein said malodor-causing substance is a moldy malodor causing substance selected from the group consisting of geonol, DMTS, 1-octen-3-ol, and mixtures of at least two of said substances.

8. The method of anyone of the claims 1 to 5, wherein said malodor-causing substance is a sweat malodor causing substance selected from the group consisting of butyric acid, 3-methyl-2-hexenoic acid, 3-hydroxy-3-methyl-hexanoic acid, transpirol, and mixtures of at least two of said substances.

9. The method of claim 6 or 7, wherein the olfactory receptor polypeptide is selected from the group consisting of OR2W1, OR1A1, OR2J3, OR4Q3, OR5K1, OR11A1, OR2M3, OR4S2, Olfr263, Olfr403, Olfr735, Olfr1193, Olfr137, Olfr173, Olfr164, Olfr1487, Olfr339, Olfr96, Olfr1322, Olfr93, Olfr398, Olfr120, Olfr1364 and Olfr937; chimera and functional fragments thereof; and olfactory receptor polypeptides having at least 70% amino acid sequence identity to at least one of the aforementioned olfactory receptor polypeptides.

10. The method of claim 9, wherein the malodour causing substance comprises geonol and the olfactory receptor is selected from the group consisting of OR1A1, OR11A1, OR2M3, Olfr403, Olfr164, Olfr1487, Olfr339, Olfr96 and Olfr1322; chimera and functional fragments thereof; and olfactory receptor polypeptides having at least 70% amino acid sequence identity to at least one of the aforementioned olfactory receptor polypeptides.

11. The method of claim 9, wherein the malodour causing substance comprises 1-octen-3-ol and the olfactory receptor is selected from the group consisting of OR2W1, OR1A1, OR2J3, OR4Q3, OR5K1, OR2H1, OR2W3, OR8G1, Olfr263, Olfr137, Olfr735, Olfr173, Olfr93, Olfr398, Olfr120, Olfr1364, Olfr937; chimera and functional fragments thereof; and olfactory receptor polypeptides having at least 70% amino acid sequence identity to at least one of the aforementioned olfactory receptor polypeptides.

12. The method of claim 9, wherein the malodour causing substance comprises DMTS and the olfactory receptor is selected from the group consisting of OR2W1, OR1A1, OR5K1, OR4S2, Olfr263, Olfr1193, Olfr173 and Olfr403; chimera and functional fragments thereof; and olfactory receptor polypeptides having at least 70% amino acid sequence identity to at least one of the aforementioned olfactory receptor polypeptides.

13. The method of claim 8, wherein the olfactory receptor polypeptide is selected from the group consisting of OR2W1, OR1A1, OR2J3, OR5K1, OR51E1, OR51I2, Olfr263, Olfr403, Olfr641, Olfr137, Olfr173, Olfr1126, Olfr558 and Olfr46; chimera and functional fragments thereof; and olfactory receptor polypeptides having at least 70% amino acid sequence identity to at least one of the aforementioned olfactory receptor polypeptides.

14. The method of claim 13, wherein the malodour causing substance comprises butyric acid and the olfactory receptor is selected from the group consisting of OR51E1, and Olfr558; chimera and functional fragments thereof; and olfactory receptor polypeptides having at least 70% amino acid sequence identity to at least one of the aforementioned olfactory receptor polypeptides.

15. The method of claim 13, wherein the malodour causing substance comprises 3-methyl-2-hexenoic acid, and the olfactory receptor is selected from the group consisting of ORS1I2, Olfr641, OR2W1 and Olfr263; chimera and functional fragments thereof; and olfactory receptor polypeptides having at least 70% amino acid sequence identity to at least one of the aforementioned olfactory receptor polypeptides.

16. The method of claim 13, wherein the malodour causing substance comprises 3-hydroxy-3-methyl-hexanoic acid, and the olfactory receptor is selected from the group consisting of OR2W1, Olfr1126 and Olfr263; chimera and functional fragments thereof; and olfactory receptor polypeptides having at least 70% amino acid sequence identity to at least one of the aforementioned olfactory receptor polypeptides.

17. The method of claim 13, wherein the malodour causing substance comprises transpirol and the olfactory receptor is selected from the group consisting of OR2W1, OR1A1, OR2J3, OR5K1, Olfr263, Olfr137, Olfr173, Olfr403 and Olfr46; chimera and functional fragments thereof; and olfactory receptor polypeptides having at least 70% amino acid sequence identity to at least one of the aforementioned olfactory receptor polypeptides.
